# EUROPEAN PATENT APPLICATION

(11) **EP 4 803 000 A1**
(43) Date of publication of application: **09.09.2026**
(21) Application number: 24953959.4
(22) Date of filing: 26.12.2024
(51) Int. Cl.: A61B 5/1473

(54) **ANALYTE MONITORING SYSTEM**

(71) Applicant: SHENZHEN SISENSING CO., LTD., Shenzhen Guangdong 518110 (CN)
(72) Inventor: LIU, Wei, Shenzhen, Guangdong 518110 (CN); XIA, Bin, Shenzhen, Guangdong 518110 (CN); QI, Jiaming, Shenzhen, Guangdong 518110 (CN); CHEN, Sai, Shenzhen, Guangdong 518110 (CN); CHEN, Zhi, Shenzhen, Guangdong 518110 (CN)
(74) Representative: Isern Patentes y Marcas S.L.
(86) International application number: PCT/CN2024/142940
(87) International publication number: WO 2026/137362

(57) **Abstract**

The present disclosure provides an analyte monitoring system(1), characterized in that the analyte monitoring system(1) includes a medical device(800), a piercing member(260), an application device(1000), and a cap(700). The medical device(800) includes an application portion(860) and a sensor(820) supported by the application portion(860) and at least partially positionable subcutaneously. The piercing member(260) is configured to carry at least a portion of the sensor(820) into the subcutaneous tissue. The application device(1000) is configured to move the piercing member(260). The cap(700) is coupled to the application device(1000) and has a sealed space for accommodating at least a portion of the sensor(820). Thereby, the analyte monitoring system(1) may be provided to the user in an integrated architecture for ease of use.

## Description

### TECHNICAL FIELD

The present disclosure relates to the field of biomedical engineering industry, and specifically to an analyte monitoring system.

### BACKGROUND

Diabetes is a chronic metabolic disease characterized by high glucose levels, which seriously affects human health. Patients generally control their condition by monitoring glucose levels. Currently, Continuous Glucose Monitoring (CGM) is an important glucose monitoring tool, which obtains glucose concentration information by implanting a sensor under the patient's skin, thereby realizing real-time monitoring of glucose in the body.

Since CGM needs to be implanted under the patient's skin and undergo chemical reactions with glucose, CGM usually needs to be sterilized before leaving the factory to minimize the impact of adverse factors such as bacteria or foreign bodies on the patient's health. Generally speaking, CGM mainly includes a sensor and an electronic unit. The biological enzymes on the sensor are very sensitive to gaseous sterilants such as ethylene oxide and are prone to inactivation during sterilization, while the circuit of the electronic unit is easily affected by radiation sterilization, resulting in performance degradation. Therefore, in the prior art, the sensor and/or electronic unit usually need to be sterilized separately.

In order to keep the sensor sterile after sterilization and before use by the user, the separately sterilized sensor usually needs to be individually packaged. That is, the sensor and the electronic unit usually need to be packaged separately. This packaging method not only increases the usage of components or packaging, but also requires the user to manually assemble the sensor and the electronic unit when using CGM. This manual assembly process may lead to CGM failure due to assembly errors, making it unable to work normally and affecting the user experience.

### SUMMARY OF THE INVENTION

The present disclosure is made in view of the above-mentioned state of the prior art, and its object is to provide an analyte monitoring system that is easy for users to use.

To this end, the present disclosure provides an analyte monitoring system, which includes a medical device, a piercing member, an application device, and a cap. The medical device includes an application portion and a sensor supported by the application portion and at least partially positionable under the skin. The piercing member is configured to carry at least a portion of the sensor into the subcutaneous tissue. The application device is configured to move the piercing member. The cap is coupled with the application device and has a sealed space for accommodating at least a portion of the sensor.

In addition, in the analyte monitoring system according to the present disclosure, optionally, the sensor includes a tail portion positionable under the skin, a contact portion for electrical connection, and a connection portion connecting the tail portion and the contact portion. The application portion includes a base with an opening. The sensor is supported by the base, and the central axis of the tail portion extends through the opening.

In addition, in the analyte monitoring system according to the present disclosure, optionally, the application portion further includes an upper cover configured to cooperate with the base to form a first space and having a hole aligned with the opening.

In addition, in the analyte monitoring system according to the present disclosure, optionally, a sealed interface is formed around the opening between the upper cover and the base, and the sensor extends through the sealed interface.

In addition, in the analyte monitoring system according to the present disclosure, optionally, the piercing member includes a sharp object having a needle portion and a cap portion. The needle portion is configured to carry the tail portion to pierce into the subcutaneous tissue of the host. The cap portion is removably and sealingly engaged with the application portion above the opening. The cap is removably and sealingly engaged with the application portion below the opening.

In addition, in the analyte monitoring system according to the present disclosure, optionally, the cap includes a mating portion coupled with at least one of the medical device and the cap portion, and the mating portion has a chamber configured to receive at least a portion of the sensor.

In addition, in the analyte monitoring system according to the present disclosure, optionally, the end of the cap portion close to the base has an engaging portion coupled with the mating portion, and the engaging portion and the mating portion are coupled by relatively screwing to form the sealed space.

In addition, in the analyte monitoring system according to the present disclosure, optionally, the base has a guide rail, and the cap has a guided portion that moves along the guide rail when rotated.

In addition, in the analyte monitoring system according to the present disclosure, optionally, the guide rail is a guide groove formed by the concave bottom surface of the base.

In addition, in the analyte monitoring system according to the present disclosure, optionally, the guide rail is arc-shaped with a predetermined radian and a predetermined radius.

In addition, in the analyte monitoring system according to the present disclosure, optionally, the base has a limiting portion disposed on the guide rail or an extension line of the guide rail and cooperating with the guided portion.

In addition, in the analyte monitoring system according to the present disclosure, optionally, the cap further includes a bottom cover engaged with the mating portion and coupled with the housing of the application device.

In addition, in the analyte monitoring system according to the present disclosure, optionally, the housing of the application device has a linear assembly slot, and the bottom cover is assembled to the housing of the application device along the assembly slot.

In addition, in the analyte monitoring system according to the present disclosure, optionally, the housing of the application device further has an engagement feature; the bottom cover moves along the engagement feature to separate from the application device; the extending direction of the engagement feature intersects with the extending direction of the assembly slot.

In addition, in the analyte monitoring system according to the present disclosure, optionally, the mating portion and the bottom cover are integrally formed.

In addition, in the analyte monitoring system according to the present disclosure, optionally, the bottom cover has a chamber configured to accommodate a desiccant, and the chamber of the bottom cover is in communication with the chamber of the mating portion.

In addition, in the analyte monitoring system according to the present disclosure, optionally, a sealing cover for forming the sealed interface is disposed between the upper cover and the base; the upper cover is sealingly engaged with the sealing cover, and the upper surface of the upper cover is substantially coplanar with the upper surface of the sealing cover.

In addition, in the analyte monitoring system according to the present disclosure, optionally, during the coupling of the engaging portion and the mating portion, the starting position of the guided portion is the starting position of the guide rail, and the ending position of the guided portion is the ending position of the guide rail; when the guided portion is located at the ending position, the sealed space is formed.

In addition, in the analyte monitoring system according to the present disclosure, optionally, at the ending position, the application portion is aligned with the accommodating portion, and the engagement feature of the cap is aligned with the assembly slot.

In addition, in the analyte monitoring system according to the present disclosure, optionally, the application device includes a housing having a first retaining mechanism, an auxiliary mechanism releasably retained by the first retaining mechanism, and a first triggering mechanism configured to trigger the first retaining mechanism to release the auxiliary mechanism; the piercing member and the medical device are assembled to the auxiliary mechanism; when the auxiliary mechanism is released, the auxiliary mechanism is driven toward the host and at least partially places the medical device under the skin of the host through the piercing member; wherein the first triggering mechanism and the first retaining mechanism are integrally formed on the housing of the application device.

According to the present disclosure, an analyte monitoring system that is easy for users to use can be provided.

### BRIEF DESCRIPTION OF THE DRAWINGS

The present disclosure will now be explained in further detail by way of example only with reference to the accompanying drawings.
Fig. 1 is a diagram showing the application overview of the analyte monitoring system according to an example of the present embodiment.
Fig. 2 is a schematic diagram showing a first perspective view of the medical device according to an example of the present disclosure.
Fig. 3 is a schematic diagram showing a second perspective view of the medical device according to an example of the present disclosure.
Fig. 4 is a block diagram showing the electronic device of the medical device according to an example of the present disclosure.
Fig. 5A is a schematic diagram showing the overall appearance of the analyte monitoring system according to an example of the present disclosure from a first perspective.
Fig. 5B is a schematic cross-sectional view showing the analyte monitoring system according to an example of the present disclosure from a first perspective.
Fig. 6 is a schematic diagram showing the overall appearance of the analyte monitoring system according to an example of the present disclosure from a second perspective.
Fig. 7A is a schematic diagram showing a first state in which the cap is decoupled from the housing according to an example of the present disclosure.
Fig. 7B is a schematic diagram showing a second state in which the cap is decoupled from the housing according to an example of the present disclosure.
Fig. 8A is a schematic diagram showing a first perspective view of the housing according to an example of the present disclosure.
Fig. 8B is a schematic diagram showing a second perspective view of the housing according to an example of the present disclosure.
Fig. 9A is a schematic diagram showing another example of the housing according to an example of the present disclosure.
Fig. 9B is a schematic diagram showing another example of the housing according to an example of the present disclosure.
Fig. 10A is a schematic diagram showing a cap mated with the housing of Fig. 9A according to an example of the present disclosure.
Fig. 10B is a schematic diagram showing a cap mated with the housing of Fig. 9B according to an example of the present disclosure.
Fig. 11 is an exploded schematic diagram showing the analyte monitoring system according to an example of the present disclosure.
Fig. 12A is a perspective schematic diagram showing a first perspective view of the first housing according to an example of the present disclosure.
Fig. 12B is a perspective schematic diagram showing a second perspective view of the first housing according to an example of the present disclosure.
Fig. 12C is a cross-sectional schematic diagram showing the first housing according to an example of the present disclosure.
Fig. 13 is an exploded schematic diagram showing the first housing, the auxiliary mechanism, and the first driving mechanism according to an example of the present disclosure.
Fig. 14A is a cross-sectional schematic diagram showing the moving body when retained according to an example of the present disclosure.
Fig. 14B is a cross-sectional schematic diagram showing the moving body when released according to an example of the present disclosure.
Fig. 15A is an exploded schematic diagram showing the second housing and the auxiliary mechanism from a first perspective according to an example of the present disclosure.
Fig. 15B is an exploded schematic diagram showing the second housing and the auxiliary mechanism from a second perspective according to an example of the present disclosure.
Fig. 15C is an exploded schematic diagram showing the second housing and the auxiliary mechanism from a third perspective according to an example of the present disclosure.
Fig. 16A is an exploded schematic diagram showing the moving body, the second driving mechanism, and the piercing member from a first perspective according to an example of the present disclosure.
Fig. 16B is an exploded schematic diagram showing the moving body, the second driving mechanism, and the piercing member from a second perspective according to an example of the present disclosure.
Fig. 17A is a cross-sectional schematic diagram showing the piercing member when retained according to an example of the present disclosure.
Fig. 17B is a cross-sectional schematic diagram showing the piercing member when released according to an example of the present disclosure.
Fig. 18A is a cross-sectional view showing a first perspective of the instrument kit before applying the medical device according to an example of the present disclosure.
Fig. 18B is a cross-sectional view showing a second perspective of the instrument kit before applying the medical device according to an example of the present disclosure.
Fig. 18C is a cross-sectional view showing a first perspective of the instrument kit during application of the medical device according to an example of the present disclosure.
Fig. 18D is a cross-sectional view showing a second perspective of the instrument kit during application of the medical device according to an example of the present disclosure.
Fig. 18E is a cross-sectional view showing the instrument kit when applying the medical device to the host according to an example of the present disclosure.
Fig. 18F is a cross-sectional view showing the piercing member in a second state after the instrument kit applies the medical device according to an example of the present disclosure.
Fig. 18G is a cross-sectional view showing the piercing member in a third state after the instrument kit applies the medical device according to an example of the present disclosure.
Fig. 19 is a cross-sectional view showing the moving body locked again to the first retaining portion after the instrument kit applies the medical device according to an example of the present disclosure.
Fig. 20A is an exploded schematic diagram showing the piercing member according to an example of the present disclosure.
Fig. 20B is a schematic diagram showing a first perspective view of the assembled piercing member according to an example of the present disclosure.
Fig. 20C is a cross-sectional schematic diagram showing the piercing member according to an example of the present disclosure.
Fig. 20D is a schematic diagram showing a second perspective view of the assembled piercing member according to an example of the present disclosure.
Fig. 20E is a cross-sectional schematic diagram showing another structure of the piercing member according to an example of the present disclosure.
Fig. 20F is an external view showing another structure of the piercing member according to an example of the present disclosure.
Fig. 20G is a cross-sectional schematic diagram showing another structure of the piercing member according to an example of the present disclosure.
Fig. 20H is a cross-sectional schematic diagram showing another structure of the piercing member according to an example of the present disclosure.
Fig. 20I is a cross-sectional schematic diagram showing another structure of the piercing member according to an example of the present disclosure.
Fig. 20J is a cross-sectional schematic diagram showing another structure of the piercing member according to an example of the present disclosure.
Fig. 21A is an exploded view showing a first perspective of the sterilization assembly according to an example of the present disclosure.
Fig. 21B is an exploded view showing a second perspective of the sterilization assembly according to an example of the present disclosure.
Fig. 22A is a schematic structural diagram showing a first perspective view of the base according to an example of the present disclosure.
Fig. 22B is a schematic structural diagram showing a second perspective view of the base according to an example of the present disclosure.
Fig. 23 is a schematic structural diagram showing the sensor according to an example of the present disclosure.
Fig. 24 is a schematic diagram showing the sensor disposed on the base according to an example of the present disclosure.
Fig. 25A is a schematic diagram showing cover of the sealing cover in the supporting portion according to an example of the present disclosure.
Fig. 25B is a cross-sectional view taken along the line X-X in Fig. 25A.
Fig. 25C is a schematic structural diagram showing a first perspective view of the sealing cover according to an example of the present disclosure.
Fig. 25D is a schematic structural diagram showing a second perspective view of the sealing cover according to an example of the present disclosure.
Fig. 26 is a schematic structural diagram showing the sharp object according to an example of the present disclosure.
Fig. 27 is a schematic structural diagram showing the first sealing element according to an example of the present disclosure.
Fig. 28A is a schematic diagram showing a first perspective view of the sharp object assembled to the base according to an example of the present disclosure.
Fig. 28B is a schematic diagram showing a second perspective view of the sharp object assembled to the base according to an example of the present disclosure.
Fig. 28C is a schematic diagram showing the depth-limiting portion abutting against the sealing cover according to an example of the present disclosure.
Fig. 29A is a schematic structural diagram showing the cap according to an example of the present disclosure.
Fig. 29B is a cross-sectional schematic diagram showing the cap according to an example of the present disclosure.
Fig. 30A is a schematic diagram showing a first perspective view of the cap assembled to the base according to an example of the present disclosure.
Fig. 30B is a schematic diagram showing a second perspective view of the cap assembled to the base according to an example of the present disclosure.
Fig. 30C is a cross-sectional view showing the sterilization assembly according to an example of the present disclosure.
Fig. 31A is a schematic diagram showing before the cap is coupled with the sharp object according to an example of the present disclosure.
Fig. 31B is a schematic diagram showing after the cap is coupled with the sharp object according to an example of the present disclosure.
Fig. 32A is a schematic diagram showing before the cap is coupled with the base according to an example of the present disclosure.
Fig. 32B is a schematic diagram showing after the cap is coupled with the base according to an example of the present disclosure.
Fig. 33A is a schematic diagram showing before the electronic device is assembled with the base according to an example of the present disclosure.
Fig. 33B is a schematic diagram showing after the electronic device is assembled with the base according to an example of the present disclosure.
Fig. 33C is a schematic diagram showing before the elastic piece is assembled with the connector according to an example of the present disclosure.
Fig. 33D is a schematic diagram showing after the elastic piece is assembled with the connector according to an example of the present disclosure.
Fig. 33E is a schematic structural diagram showing the connector according to an example of the present disclosure.
Fig. 34A is a schematic diagram showing the upper cover assembled with the base according to an example of the present disclosure.
Fig. 34B is a schematic diagram showing the upper cover assembled with the base according to an example of the present disclosure.
Fig. 34C is an enlarged schematic diagram showing area A in Fig. 34B.
Fig. 35 is a schematic diagram showing the housing assembled with the sterilization assembly assembled with the electronic device and the upper cover according to an example of the present disclosure.

### DETAILED DESCRIPTION

Hereinafter, preferred embodiments of the present disclosure will be described in detail with reference to the accompanying drawings. In the following description, the same components are denoted by the same reference numerals, and repeated descriptions are omitted. In addition, the drawings are only schematic diagrams, and the proportions of the dimensions between components, the shapes of the components, and the like may be different from the actual ones.

It should be noted that the terms "including" and "having" and any variations thereof in the present disclosure are intended to cover non-exclusive inclusion. For example, a process, method, system, product, or device including a series of steps or units is not limited to the clearly listed steps or units, but may include or have other steps or units not clearly listed or inherent to the process, method, product, or device.

It should be noted that in this document, terms such as "upper", "upward", "lower", "downward", "up-and-down direction", "left", "leftward", "left side", "toward left side", "right", "rightward", "right side", "toward right side", "horizontal direction", "front", "forward", "rear", "backward", "front-to-back direction" and other relative positional and directional terms refer to the usual operating posture and should not be considered as restrictive.

First, related terms involved in the present disclosure will be introduced.

"Decoupling" may refer to releasing the coupling.

"Compactness" may refer to the tightness of assembly between components.

In some examples, the analyte monitoring system involved in the present disclosure may also be referred to as an analyte monitoring device, an analyte monitor, an analyte concentration information collection device, or a biological monitoring device. In addition, the sensor involved in the examples of the present disclosure may also be referred to as a monitoring probe, a sensing probe, or an electrode probe.

In some examples, when the analyte is glucose, the sensor may be a glucose sensor, and the analyte level may be glucose concentration. However, it is not limited to glucose concentration. For example, by changing the sensing layer of the sensor, other body fluid components besides glucose concentration can also be obtained. The body fluid components here may be one or more of glucose, acetylcholine, amylase, bilirubin, cholesterol, chorionic gonadotropin, creatine kinase, creatine, creatinine, DNA, fructosamine, glutamine, growth hormone, hormone, blood ketone, lactate, oxygen, peroxide, prostate-specific antigen, prothrombin, RNA, thyroid-stimulating hormone, or troponin.

For the convenience of description, some examples are described with the analyte being glucose, and accordingly, the analyte level is glucose concentration. It should be noted that this does not represent a limitation of the present disclosure, and unless there is a contradiction, the relevant descriptions are also applicable to other analyte levels.

Hereinafter, the analyte monitoring system according to examples of the present disclosure will be described in detail with reference to the accompanying drawings.

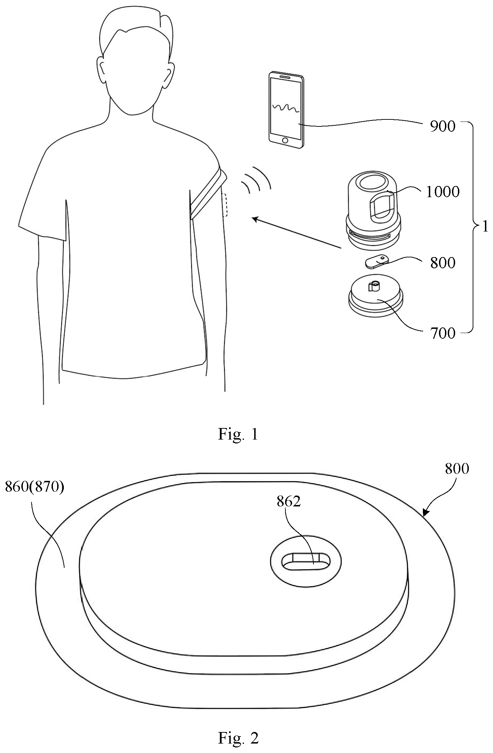

Fig. 1 is a diagram showing the application overview of the analyte monitoring system 1 according to an example of the present embodiment. In some examples, the analyte monitoring system 1 may include a medical device 800 and an application device 1000. The medical device 800 may be applied to the host via the application device 1000, and the host may obtain physiological information through the medical device 800 applied to himself/herself. In some examples, the medical device 800 may be applied to a desired position.

In some examples, the analyte monitoring system 1 may include a reading device 900 communicatively connected to the medical device 800 (see Fig. 1). The medical device 800 applied to the host may transmit the acquired physiological information to the reading device 900 via wireless means, for example, thereby facilitating the host to read and monitor his/her own physiological information.

In addition, the present disclosure also provides an instrument kit 90, which may include the application device 1000 according to the present disclosure for applying the medical device 800 to the host.

In some examples, the medical device 800 may be configured to obtain the analyte level of the host. In some examples, the medical device 800 may be a sensing device. In some examples, the medical device 800 may be an analyte sensor device, which may generate information about specific analytes in body fluids based on body fluids, for example, react with analytes in body fluids and generate analyte information. In this case, the sensor 820 reacts with the analyte in the body fluid, thereby facilitating the acquisition of analyte information in the body fluid. For example, the medical device 800 may be applied to the host's body surface and at least partially located under the host's skin to obtain glucose concentration under the skin of the host.

In the present disclosure, the analyte targeted by the analyte sensor device may be one or more of glucose, acetylcholine, amylase, bilirubin, cholesterol, chorionic gonadotropin, creatine kinase, creatine, creatinine, DNA, fructosamine, glutamine, growth hormone, hormone, ketone body, lactate, oxygen, peroxide, prostate-specific antigen, prothrombin, RNA, thyroid-stimulating hormone, or troponin.

Hereinafter, the medical device 800 according to examples of the present disclosure will be described by taking glucose as the analyte. It should be noted that for other analytes, those skilled in the art can slightly modify the medical device 800 used for glucose to analyze other analytes.

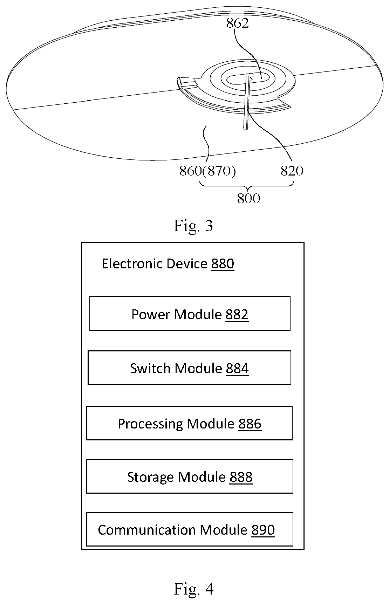

Fig. 2 is a schematic diagram showing a first perspective view of the medical device 800 according to an example of the present disclosure. Fig. 3 is a schematic diagram showing a second perspective view of the medical device 800 according to an example of the present disclosure.

In some examples, the medical device 800 may include a sensor 820 and an application portion 860 (see Fig. 2 or Fig. 3). In some examples, the sensor 820 may be supported by the application portion 860.

In some examples, the sensor 820 may be partially or fully placed under the host's skin. In other words, the sensor 820 may be at least partially placed under the skin. Thus, physiological information of subcutaneous tissue fluid can be monitored.

In some examples, after the sensor 820 is placed under the skin, it can react with glucose in the subcutaneous tissue fluid to generate glucose information of the host. The application portion 860 can be applied to the host's body surface. In some examples, the application portion 860 can also receive the glucose information generated by the sensor 820. In some examples, the application portion 860 can also provide the electric energy required by the sensor 820 to obtain physiological information. Thus, it is beneficial for the sensor 820 to perform continuous monitoring under the host's skin.

In some examples, the implanted part of the sensor 820 may be elongated. In some examples, the implanted part may be rigid. Thus, it can be helpful to be placed under the host's skin. In other examples, the implanted part may also be flexible, in which case the foreign body sensation of the host can be reduced. In some examples, after the implanted part is implanted into the host, its depth can reach the dermis layer and be located between the tissues of the skin, so that the sensor 820 can collect glucose information of the interstitial fluid.

In some examples, the application portion 860 may further include a hole 862 penetrating from the upper surface to the lower surface (see Fig. 2 or Fig. 3), and when the sensor 820 is assembled to the application portion 860, the axis of the sensor 820 may pass through the hole 862 (see Fig. 2 or Fig. 3). In this case, the sharp object 270 (to be described later) is pierced into the subcutaneous tissue through the hole, thereby facilitating the placement of the sensor 820 under the skin.

In some examples, the implanted part may include a working electrode and a counter electrode. In the present disclosure, a sensing layer including glucose enzyme may be provided on the working electrode. The sensor 820 placed under the skin may undergo redox reaction with glucose in tissue fluid or blood through the glucose enzyme on the working electrode, and form a loop with the counter electrode to generate a current signal. The current signal is analyzed and processed to obtain glucose concentration information. In some examples, the current signal generated by the sensor 820 may be transmitted to the application portion 860.

In some examples, the implanted part may further include a reference electrode. In the present disclosure, the reference electrode may form a known and fixed potential difference with tissue fluid or blood. In this case, the potential difference between the working electrode and the tissue fluid or blood can be measured through the potential difference formed between the reference electrode and the working electrode. Thus, the voltage generated by the working electrode can be obtained more accurately. Thus, the electronic device 880 (to be described later) of the application portion 860 can automatically adjust and maintain the stability of the voltage at the working electrode according to a preset voltage value, so that the measured current signal can more accurately reflect the glucose concentration information in the tissue fluid or blood. In some examples, the number of reference electrodes may be one or more, for example two.

In some examples, the application portion 860 is configured to be adhered to the host's body surface. In some examples, the application portion 860 may further include an adhesive sheet 870 with adhesive properties (see Fig. 2 or Fig. 3). The application portion 860 can be applied and fixed to the host's body surface through the adhesive sheet 870.

Fig. 4 is a block diagram showing the electronic device 880 of the medical device 800 according to an example of the present disclosure.

In some examples, referring to Fig. 4, the application portion 860 may further include an electronic device 880. The electronic device 880 may receive the glucose information generated by the sensor 820. In some examples, the electronic device 880 may further process the glucose information. In other examples, the electronic device 880 may also transmit the received glucose information to an external device, such as the reading device 900 to be described below. In addition, the electronic device 880 may also supply power to the sensor 820.

In some examples, the electronic device 880 may include a power module 882, a switch module 884, and a processing module 886 (see Fig. 4). The power module 882 may supply power to the sensor 820 and/or the processing module 886. The switch module 884 may control the connection and disconnection between the power module 882 and the sensor 820 and/or the processing module 886. The processing module 886 may process signal (e.g., glucose information) generated by the sensor 820.

In some examples, the application portion 860 has an initial mode configured to be open circuit and an operating mode configured to be closed circuit. In this case, when the application portion 860 is in the initial mode, it is configured to be open circuit, thereby saving power consumption before the application portion 860 enters the working state. In some examples, when the application portion 860 is configured in the initial mode, the switch module 884 may be in an off state, and when the application portion 860 is configured in the operating mode, the switch module 884 may be in an on state. That is, the application portion 860 can be switched from the initial mode to the operating mode through the switch module 884. In this case, the switch module 884 is used to control the switching of the application portion 860 from the initial mode to the operating mode, thereby effectively reducing power consumption before operation.

In addition, in some examples, the electronic device 880 may further include a storage module 888 (see Fig. 4). The storage module 888 may store the signal generated by the sensor 820. In addition, in some examples, the electronic device 880 may further include a communication module 890 (see Fig. 4). The communication module 890 may communicate with an external device (for example, an intelligent terminal device). In some examples, the communication module 890 may be a Bluetooth module, an NFC module, a Wifi module, or the like. In some examples, the intelligent terminal device may be a smartphone, a smart watch, a tablet computer, a computer, or the like.

In some examples, the application portion 860 is configured to be excited from the initial mode to the operating mode by magnetic action or optical action. In this case, the application portion 860 is excited from the initial mode to the operating mode through non-contact components such as magnetic action or optical action, thereby facilitating the miniaturization of the application portion 860.

In other examples, the switch module 884 may also be configured as a magnetically controlled switch. For example, in the initial mode, the switch module 884 may be in an off state, and when the magnetic action on the switch module 884 changes, the switch module 884 may switch from the off state to the on state, thereby exciting the application portion 860 from the initial mode to the operating mode.

In some examples, the application device 1000 may include a magnet. When the medical device 800 has not been applied to the host, since the electronic device 880 of the medical device 800 is close to the magnet at this time, the switch module 884 of the electronic device 880 may be in an off state. When the medical device 800 is applied to the host, since the electronic device 880 of the medical device 800 is far away from the magnet at this time, the switch module 884 of the electronic device 880 may be excited to the on state.

In addition, in some examples, the power module 882 of the electronic device 880 may also be configured to supply power to the sensor 820. In some examples, when the electronic device 880 is in the initial mode, the power module 882 and the sensor 820 may be disconnected, and when the electronic device 880 is excited to the operating mode (that is, when the switch module 884 is in the on state), the power module 882 may be connected to the sensor 820, thereby supplying power to the sensor 820.

In some examples, as described above, the application portion 860 may be configured to communicate with an external device via a wireless communication method or a wired communication method. In this case, by configuring the application portion 860 to be able to communicate with an external device, analyte information acquired by the sensor 820 can be read in real time or periodically. In some examples, the external device may be a reading device 900.

In some examples, as described above, the medical device 800 may be communicatively connected to the reading device 900. In some examples, the reading device 900 may be a display with a communication function. In some examples, the communication function of the reading device 900 may be implemented via a wireless communication method or a wired communication method. The wireless communication method may be Bluetooth, NFC, Wifi, or the like. The wired communication method may be USB, optical fiber, or the like.

In other examples, the reading device 900 may also be an intelligent terminal installed with an application program matching the medical device 800. The intelligent terminal may be a notebook computer, a tablet computer, a smart phone, or the like.

As described above, the medical device 800 according to the present disclosure may be applied to the host through the application device 1000. Hereinafter, the analyte monitoring system 1 according to the present disclosure will be described in detail with reference to the accompanying drawings.

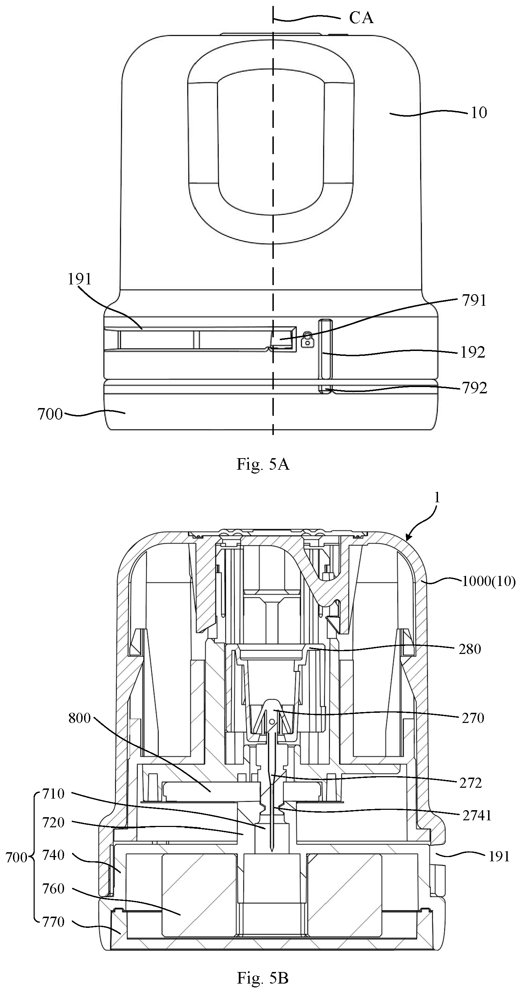

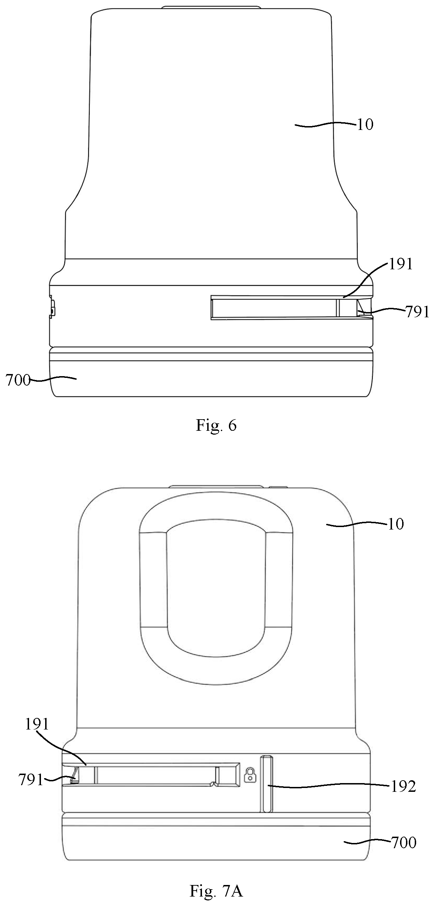

Fig. 5A is a schematic diagram showing the overall appearance of the analyte monitoring system 1 according to an example of the present disclosure from a first perspective; Fig. 5B is a schematic cross-sectional view showing the analyte monitoring system 1 according to an example of the present disclosure from a first perspective. Fig. 6 is a schematic diagram showing the overall appearance of the analyte monitoring system 1 according to an example of the present disclosure from a second perspective. Wherein, Fig. 5A shows a schematic diagram when the cap 700 is coupled with the housing 10, and Fig. 5B does not show the sensor 820.

In some examples, referring to Figs. 5A and 5B, the analyte monitoring system 1 according to the present disclosure is an integrated structure, and the medical device 800 is pre-assembled to the application device 1000 at the factory stage. The analyte monitoring system 1 can be provided to the user in an integrated package, and the user can directly apply the medical device 800 to the host through the application device 1000 without any assembly steps during use.

In some examples, referring to Fig. 5B, the analyte monitoring system 1 may include an application device 1000 and a medical device 800. In some examples, the medical device 800 may be releasably retained by the application device 1000.

In some examples, the analyte monitoring system 1 may include a cap 700 coupled with the application device 1000. In some examples, the cap 700 may have a sealed space for accommodating at least a portion of the sensor 820. Thus, at least a portion of the sensor 820 can be isolated from external contamination.

In some examples, referring to Fig. 5B, the application device 1000 may include a housing 10. In some examples, the cap 700 may be coupled with the housing 700. Thus, the medical device 800 can be covered in the application device 1000.

In some examples, the cap 700 may be removably coupled to the housing 10. In some examples, removing the cap 700 may expose the medical device 800 retained in the application device 1000. In some examples, after the cap 700 is removed, the user can use the application device 1000 to apply the medical device 800 to the host.

As described above, the medical device 800 may include a sensor 820 and an application portion 860. Wherein, the sensor 820 may extend from the bottom of the application portion 860. In some examples, the cap 700 is configured to provide a sealed space for the tail portion 821 of the sensor 820. Thus, the sensor 820 can be isolated from external contamination before being implanted into the host.

In some examples, referring to Fig. 5B, the instrument kit 90 may include a sharp object 270. In some examples, the sharp object 270 may be configured to carry the tail portion 821 to pierce into the subcutaneous tissue of the host.

In some examples, the cap 700 may be coupled with at least one of the medical device 800 or the sharp object 270 to form a sealed space for accommodating at least a portion of the sensor 820. For example, the cap 700 may be coupled with the medical device 800, or with the sharp object 270, or with both the medical device 800 and the sharp object 270 to form the above-mentioned sealed space.

In some examples, the cap 700 may also be configured to provide a sealed space for the tail portion of the sharp object 270. In some examples, the sharp object 270 may pass through the application portion 860 and extend from the bottom of the application portion 860, and the portion of the sharp object 270 extending out of the application portion 860 may be accommodated in the sealed space.

In some examples, the sharp object 270 may have a space for carrying and accommodating the sensor 820, and the sensor 820 may be at least partially accommodated in the sharp object 270. In some examples, the sharp object 270 and the sensor 820 may be at least partially located in the above-mentioned sealed space.

In some examples, the sharp object 270 may not have a space for carrying and accommodating the sensor 820. In this case, the tail portion 821 of the sensor 820 can be attached to the sharp object 270 by appropriately setting the angle between the sensor 820 and the sharp object 270.

In some examples, referring to Fig. 5B, the cap 700 may include a mating portion 720. In some examples, the mating portion 720 may be coupled with at least one of the medical device 800 and the sharp object 270. Thus, a sealed space for accommodating at least a portion of the sensor 820 can be formed.

In some examples, the mating portion 720 has a chamber 710 configured to receive at least a portion of the sensor 820. In some examples, the tail portion of the sharp object 270 and the tail portion 821 of the sensor 820 may be jointly received in the chamber 710. Thus, a sealed space can be provided for the tail portion of the sharp object 270 that can pierce into the subcutaneous tissue and the tail portion 821 of the sensor 820 that is implanted into the subcutaneous tissue.

In some examples, the sharp object 720 may have a needle portion 272 and a cap portion 276. The mating portion 720 may be coupled with the end of the cap portion 276 close to the application portion 860 to form the above-mentioned sealed space. In some examples, the mating portion 720 may be relatively screwed with the end (i.e., the engaging portion 2741) of the cap portion 276 close to the application portion 860 to form the above-mentioned sealed space. In some examples, the above-mentioned sealed space may also be formed by means such as snap fasteners or male-female fitting.

In some examples, the cap 700 may include a bottom cover 770 engaged with the mating portion 720. In some examples, the bottom cover 770 may be coupled with the housing 10 of the application device 1000.

In some examples, the bottom cover 770 may be removably coupled to the housing 10.

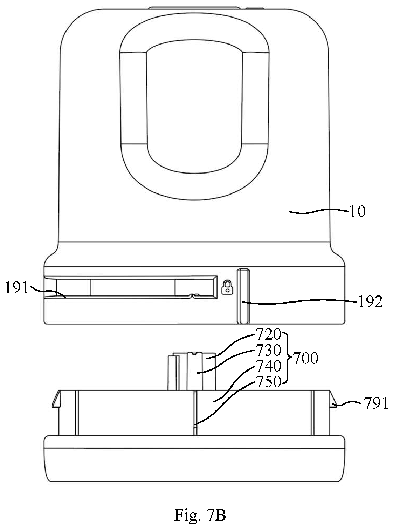

Fig. 7A is a schematic diagram showing a first state in which the cap 700 is decoupled from the housing 10 according to an example of the present disclosure. Fig. 7B is a schematic diagram showing a second state in which the cap 700 is decoupled from the housing 10 according to an example of the present disclosure.

In some examples, the housing 10 may have an engagement feature 191, and the bottom cover 770 may have an engagement feature 791. For convenience of description, the engagement feature 191 of the housing 10 is hereinafter referred to as the first engagement feature 191, and the engagement feature of the bottom cover 770 is referred to as the second engagement feature 791.

In some examples, the bottom cover 770 may be coupled to the housing 10 through the cooperation of the first engagement feature 191 and the second engagement feature 791.

In some examples, the first engagement feature 191 may have a first engaging surface facing the distal end. In some examples, the second engagement feature 791 may have a second engaging surface facing the proximal end. When the first engaging surface abuts against the second engaging surface, the cap 700 may be coupled to the housing 10.

In some examples, referring to Figs. 7A and 7B, the housing 10 may include a proximal portion having the first engagement feature 191. In some examples, the first engagement feature 191 may extend along the circumferential direction of the housing 10. In some examples, the first engagement feature 191 may be an annular opening formed in the proximal portion. In some examples, the first engagement feature 191 may be formed as a guide groove in the proximal portion, for example, an annular groove formed in the inner wall of the proximal portion.

As described above, the bottom cover 770 may have a second engagement feature 791. In some examples, the second engagement feature 791 may be a protrusion formed on the peripheral edge of the bottom cover 770.

In some examples, the bottom cover 770 may move along the first engagement feature 191 to separate from the application device 1000. In some examples, the movement of the bottom cover 770 along the first engagement feature 191 may release the cooperation between the first engagement feature 191 and the second engagement feature 791 to release the coupling between the bottom cover 770 and the application device 1000.

In some examples, referring to Figs. 6 and 7A, during the decoupling of the cap 700 from the housing 10, the second engagement feature 791 and the first engagement feature 191 may move relative to each other. In some examples, the second engagement feature 791 may move along the extending path of the first engagement feature 191. In some examples, the movement path of the second engagement feature 791 relative to the first engagement feature 191 may be an arc.

In some examples, the housing 10 and the cap 700 may be relatively screwed to release the cooperation between the first engagement feature 191 and the second engagement feature 791. In some examples, the housing 10 and the cap 700 may be relatively screwed to expose the medical device 800 retained in the application device 1000. In some examples, the cap 700 may be rotated relative to the housing 10 by a preset angle to release the coupling with the housing 10. In some examples, the cap 700 may release the coupling with the sharp object 270 in response to decoupling from the housing 10.

Let the angle corresponding to the arc of the movement path of the second engagement feature 791 relative to the first engagement feature 191 be a preset angle. In some examples, the preset angle may be the same as the angle applied when the cap 700 is coupled to the sharp object 270. In some examples, the relative screwing direction when decoupling the cap 700 and the housing 10 may be opposite to the relative screwing direction when coupling the cap 700 and the sharp object 270. Thus, the cap 700 can be decoupled from the sharp object 270 while being decoupled from the housing 10.

In some examples, the angle corresponding to the path defined by the first engagement feature 191 may be equal to the preset angle. Thus, when the second engagement feature 791 is moved from the starting position to the ending position of the first engagement feature 191, the cap 700 can be decoupled from the housing 10.

In some examples, the angle corresponding to the path defined by the first engagement feature 191 may be greater than the preset angle, and the user may rotate the second engagement feature 791 along the extending path of the first engagement feature 191 by the preset angle by observing or the like to decouple the cap 700 from the housing 10.

In some examples, referring to Fig. 5A, the housing 10 may have a first indicator 192, and the cap 700 may have a second indicator 792. In some examples, when the first indicator 192 and the second indicator 792 are aligned, the cap 700 is coupled to the housing 10. When the analyte monitoring system 1 is taken out of the packaging box, it can be determined whether the analyte monitoring system 1 has undergone an undesired morphological change before being used based on whether the first indicator 192 and the second indicator 792 are aligned.

In some examples, during the decoupling of the cap 700 from the housing 10, the first indicator 192 and the second indicator 792 may move relative to each other. In some examples, the movement path of the second indicator 792 relative to the first indicator 192 may be an arc, and the angle corresponding to the arc of the arc may be equal to the preset angle.

In some examples, after relatively screwing the cap 700 and the housing 10 by the preset angle, the cap 700 may move in a direction away from the housing 10 to decouple from the housing 10 (see Figs. 5A, 7A and 7B).

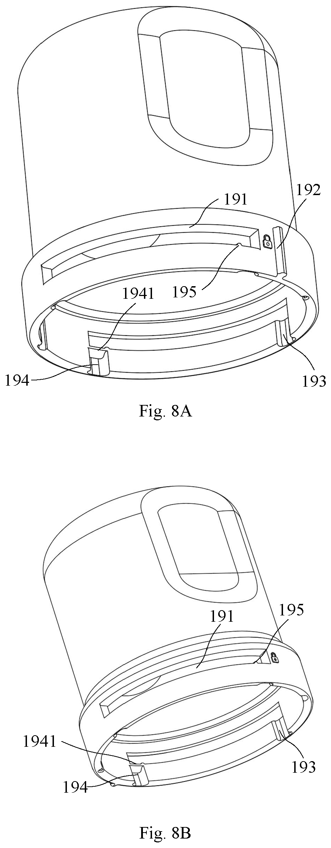

Fig. 8A is a schematic diagram showing a first perspective view of the housing 10 according to an example of the present disclosure; Fig. 8B is a schematic diagram showing a second perspective view of the housing 10 according to an example of the present disclosure.

In some examples, referring to Figs. 8A and 8B, the housing 10 may have a decoupling groove 193. In some examples, the decoupling groove 193 may communicate with the first engagement feature 191. In some examples, the decoupling groove 193 may be configured to provide a path for decoupling the cap 700 from the housing 10. In some examples, the decoupling groove 193 may be provided at one end of the first engagement feature 191, for example, at the ending position of the first engagement feature 191.

In some examples, the second engagement feature 791 may move from the end of the first engagement feature 191 away from the decoupling groove 193 to the end close to the decoupling groove 193. In some examples, when the second engagement feature 791 moves to the end of the first engagement feature 191 close to the decoupling groove 193, the second engagement feature 791 may enter the decoupling groove 193. In some examples, the second engagement feature 791 may move away from the housing 10 along the extending direction of the decoupling groove 193.

In some examples, the housing 10 may have at least one first engagement feature 191, for example, 1, 2, 3, or 4 engagement feature 191. In some examples, when the housing 10 has a plurality of first engagement features 191, the plurality of first engagement features 191 may be symmetrically arranged on the proximal portion of the housing 10. Thus, the cap 700 can be stably coupled to the application device 1000. In some examples, the number of second engagement features 791 may be the same as the number of first engagement features 191.

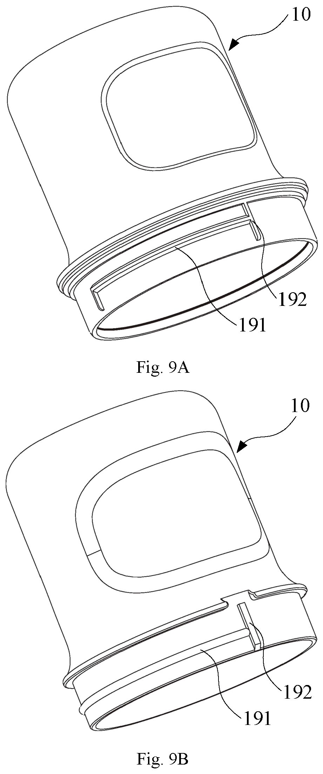

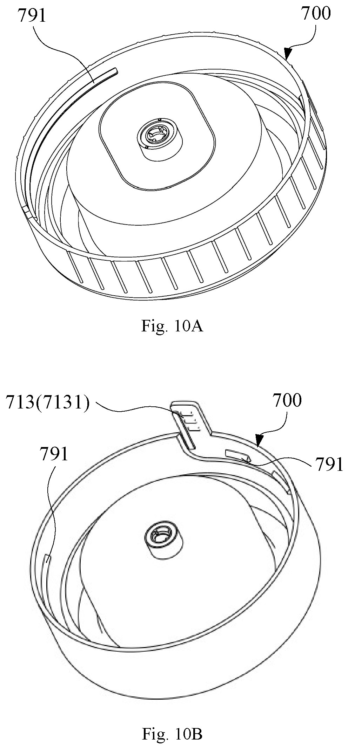

Fig. 9A is a schematic diagram showing another embodiment of the housing 10 according to an example of the present disclosure. Fig. 9B is a schematic diagram showing another embodiment of the housing 10 according to an example of the present disclosure. Fig. 10A is a schematic diagram showing a cap 700 mated with the housing 10 of Fig. 9A. Fig. 10B is a schematic diagram showing a cap 700 mated with the housing 10 of Fig. 9B.

In some examples, the housing 10 may be coupled to the cap 700 in such a way that it is at least partially received in the cap 700.

In some examples, as described above, the first engagement feature 191 may have a first engaging surface facing the distal end. In some examples, as described above, the second engagement feature 791 may have a second engaging surface facing the proximal end. When the first engaging surface 12 abuts against the second engaging surface 712, the cap 700 may be coupled to the housing 10.

In some examples, referring to Figs. 9A and 9B, the first engagement feature 191 may be a circumferential rib formed on the proximal portion. In some examples, referring to Figs. 10A and 10B, the second engagement feature 791 may be a circumferential rib formed on the cap 700.

In some examples, a decoupling structure may be provided between two adjacent first engagement features 191. In some examples, the second engagement feature 791 may be released from the cooperation with the first engagement feature by relatively rotating the cap 700 and the housing 10. Thus, the cap 700 can be decoupled from the housing 10. In some examples, the decoupling structure may refer to a portion of the proximal portion where no other structure is formed, for example, a portion where the first engagement feature 191 is not formed. In this case, there is no need to additionally provide the decoupling groove 193 mentioned above. Thus, when the second engagement feature 791 is operated to correspond to the decoupling structure, it can be released in a direction away from the housing 10 to decouple the cap 700 and the housing 10.

In some examples, at least part of the structure of the second engagement feature 791 may be a tearable feature.

In some examples, referring to Fig. 10B, the tearable feature may have a breakable portion 713. In some examples, the breakable portion may be removed in a direction away from the central axis CA. Thus, the cooperation between the first engagement feature 191 and the second engagement feature 791 can be at least partially released.

In some examples, referring to Fig. 10B, the breakable portion may be formed with an intermittent circumferential rib, and when the intermittent circumferential rib abuts against the first engagement feature 191, the cap 700 may be connected to the housing 10. In some examples, the breakable portion may be formed with a continuous circumferential rib.

In some examples, referring to Fig. 10B, the tearable feature may have a protruding portion 7131. In some examples, the protruding portion may be connected to the breakable portion. Thus, it is convenient for the user to operate to remove the breakable portion. In some examples, the protruding portion 7131 may serve as the second indicator 792 of the cap, cooperating with the first indicator 192 of the housing 10.

In some examples, the second engagement feature 791 includes a circumferential rib disposed opposite to the tearable feature. After removing the tearable portion, the second engagement feature 791 can be released from the cooperation with the circumferential rib disposed opposite to the tearable feature by relatively rotating the cap 700 and the housing 10. Thus, the cooperation between the cap 700 and the housing 10 can be released.

Embodiments of the present disclosure include:
A. A method of using an analyte monitoring system, the analyte monitoring system including an application device having a housing, a cap removably coupled to the housing, a medical device retained in the application device, and a sharp object assembled to the medical device, the method including: relatively screwing the housing and the cap to expose the medical device retained in the application device, wherein the cap releases the coupling with the sharp object in response to decoupling from the housing.

In some examples, Embodiment A may be combined with any one or more of the following additional elements. Element 1: The cap is rotated relative to the housing by a preset angle to release the coupling with the housing. Element 2: The housing includes a proximal portion having a first engagement feature. Element 3: The first engagement feature extends along the circumferential direction of the housing. Element 4: The first engagement feature is a guide groove, an annular opening, or a circumferential rib formed in the proximal portion. Element 5: The cap has a second engagement feature. Element 6: The second engagement feature is a protrusion or a circumferential rib formed on the cap. Element 7: The second engagement feature moves along the extending path of the first engagement feature. Element 8: The movement path of the second engagement feature relative to the first engagement feature is an arc, and the angle corresponding to the arc of the arc is equal to the preset angle. Element 9: The housing has a first indicator, the cap has a second indicator, and during the decoupling of the cap from the housing, the first indicator and the second indicator move relative to each other. Element 10: The movement path of the second indicator relative to the first indicator is an arc, and the angle corresponding to the arc of the arc is equal to the preset angle. Element 11: After relatively screwing the cap and the housing by the preset angle, the second engagement feature moves from one end of the first engagement feature to the other end, and the cap moves in a direction away from the housing to decouple from the housing. Element 12: In response to an action applied to the cap along the central axis of the application device, the second engagement feature is released along the decoupling groove. Element 13: The housing of the application device further has an engagement feature, and the bottom cover moves along the engagement feature to separate from the application device.

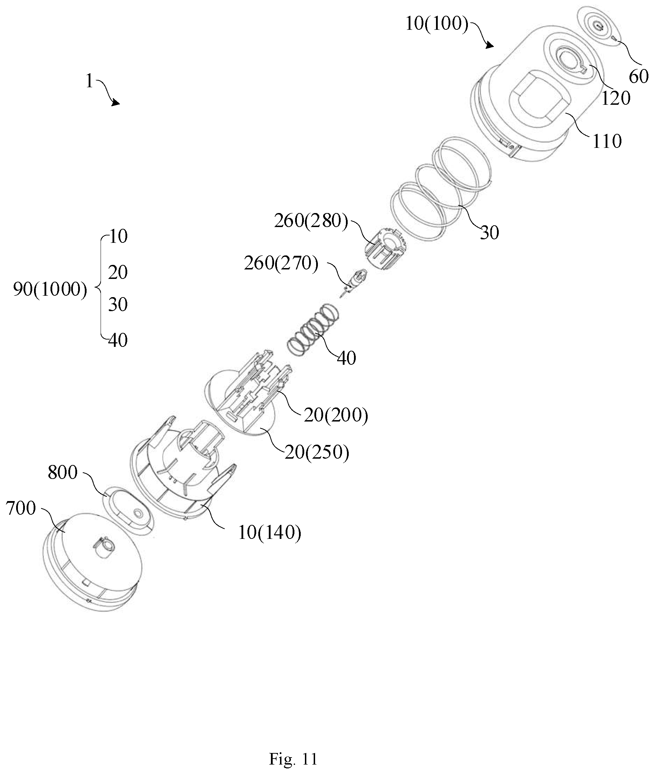

Fig. 11 is an exploded schematic diagram showing the analyte monitoring system 1 according to an example of the present disclosure.

In the present disclosure, the instrument kit 90 may include an application device 1000 for applying the medical device 800 to the host. In some examples, referring to Fig. 11, the application device 1000 may include a housing 10 that can provide a moving space, and an auxiliary mechanism 20 (to be described later) configured to be movable within the moving space of the housing 10. The housing 10 may have a proximal end close to the host and a distal end away from the host during operation. The auxiliary mechanism 20 may be releasably retained by the housing 10 and may receive the medical device 800. The auxiliary mechanism 20 may be configured to be movable relative to the housing 10 when released and driven toward the host to apply the medical device 800 to the host. It can be understood that in this document, "proximal end" may be understood as the end close to the host during operation, and "distal end" may be understood as the end away from the host during operation.

In some examples, the housing 10 may have a moving space, and the auxiliary mechanism 20 may be releasably retained by the housing 10. In some examples, when the auxiliary mechanism 20 is released, it can move within the moving space of the housing 10. In addition, in some examples, the housing 10 may also define the application position of the medical device 800 on the host's body surface.

In some examples, referring to Fig. 11, the housing 10 may include a first housing 100 and a second housing 140. In some examples, the second housing 140 may be assembled to the first housing 100. The first housing 100 and the second housing 140 may form a moving space after being assembled and combined. The first housing 100 may releasably retain the auxiliary mechanism 20. In some examples, the first housing 100 may define the application position of the medical device 800 on the host's body surface. In some examples, the second housing 140 may define the application position of the medical device 800 on the host's body surface.

In some examples, the second housing 140 may be fixedly assembled to the first housing 100. In some examples, the first housing 100 and the second housing 140 may be integrally formed.

In some examples, the second housing 140 may define the moving path of the auxiliary mechanism 20. After being released by the first housing 100, the auxiliary mechanism 20 may move along the moving path defined by the second housing 140 relative to the second housing 140 and apply the medical device 800 to the application position. In the present disclosure, the axis of the first housing 100 and the axis of the second housing 140 may be substantially parallel to the central axis CA of the application device 1000.

In some examples, referring to Fig. 11, the auxiliary mechanism 20 may include a moving body 200 and an accommodating portion 250. In some examples, the moving body 200 may be releasably retained by the first housing 100. In some examples, the moving body 200 may be configured to be movable along the moving path defined by the second housing 140 when released. In some examples, the accommodating portion 250 may be provided on the moving body 200 and configured to releasably retain the medical device 800. In some examples, after being released, the moving body 200 may move toward the proximal end of the housing 10, and the accommodating portion 250 also moves toward the proximal end of the housing 10 to apply the medical device 800 accommodated in the accommodating portion 250 to the host.

In some examples, referring to Fig. 11, the instrument kit 90 may include a sharp object 270 and a support base 280 for supporting the sharp object 270 and assembled to the moving body 200. Hereinafter, for convenience of description, the sharp object 270 and the support base 280 may be collectively referred to as a piercing member 260.

In some examples, the piercing member 260 may be provided on the moving body 200. When the moving body 200 is released, the moving body 200 may be driven toward the proximal end and at least partially place the medical device under the host's skin through the piercing member 260. Specifically, the piercing member 260 may be provided on the moving body 200 in such a way as to pass through the accommodating portion 250. After being released, the moving body 200 may move toward the proximal end of the housing 10, and the accommodating portion 250 and the piercing member 260 also move toward the proximal end of the housing 10 to apply the medical device 800 accommodated in the accommodating portion 250 to the host. In some examples, the piercing member 260 may pierce into the host's subcutaneous tissue, thereby at least partially placing the medical device 800 under the host's skin.

In some examples, the component (for example, a sensor) of the medical device 800 that can be implanted under the host's skin may have a certain hardness. Thus, the sensor can be implanted under the host's skin without the help of the piercing member 260. In other words, the instrument kit 90 may not include the piercing member 260, and the sensor can be implanted under the host's skin by virtue of its own hardness.

In some examples, referring to Fig. 11, the application device 1000 may further include a first driving mechanism 30. In some examples, the first driving mechanism 30 may be configured to apply an action to the auxiliary mechanism 20 in a direction toward the proximal end. In some examples, when the auxiliary mechanism 20 is released, the first driving mechanism 30 may drive the auxiliary mechanism 20 to move toward the host. Thus, the medical device 800 releasably retained by the auxiliary mechanism 20 can be automatically applied to the host.

In some examples, the first driving mechanism 30 may be configured to apply an action to the auxiliary mechanism 20 in a direction toward the proximal end. When the auxiliary mechanism 20 is released, the first driving mechanism 30 may drive the auxiliary mechanism 20 toward the proximal end to push the medical device 800 accommodated in the accommodating portion 250 toward the host, for example, to the application position defined by the housing 10. In addition, the medical device 800 can be at least partially placed under the host's skin through the piercing member 260.

In other examples, the application device 1000 may not include the first driving mechanism 30. In this case, after the moving body 200 is released, the moving body 200 may also be manually driven to move toward the proximal end.

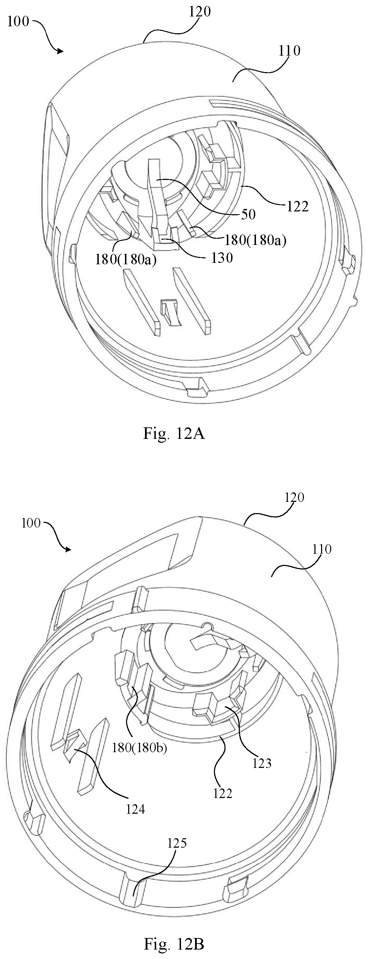

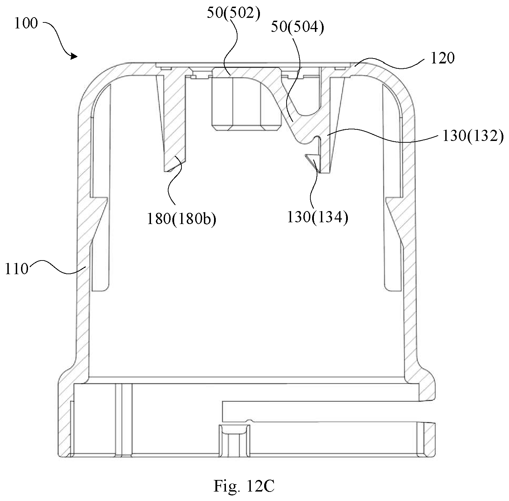

Fig. 12A is a perspective schematic diagram showing a first perspective view of the first housing 100 according to an example of the present disclosure; Fig. 12B is a perspective schematic diagram showing a second perspective view of the first housing 100 according to an example of the present disclosure. Fig. 12C is a cross-sectional schematic diagram showing the first housing 100 according to an example of the present disclosure.

In some examples, as described above, the housing 10 may include a first housing 100. In some examples, the first housing 100 may be formed as a cylindrical shell that is hollow and penetrates up and down along the central axis CA of the application device 1000. In some examples, the first housing 100 may be formed as a cylindrical shell with an opening only at the proximal end.

In some examples, referring to Fig. 12A, the first housing 100 may include a peripheral portion 110 and a distal portion 120. In some examples, the distal portion 120 may be formed on the peripheral portion 110 in a manner close to the distal end of the housing 10. In some examples, the central axis CA may pass through the geometric center of the distal portion 120.

In some examples, referring to Fig. 12A, the first housing 100 may include a first retaining portion 130. In some examples, the first retaining portion 130 may be configured to releasably retain the moving body 200. In some examples, the first retaining portion 130 may be provided on the distal portion 120. In some examples, the first retaining portion 130 may extend from the distal portion 120 in a direction toward the proximal end of the application device 1000 along the central axis CA. In this case, when the moving body 200 is retained by the distal portion 120, the moving body 200 may be located in the hollow portion of the peripheral portion 110, and when the moving body 200 is released, the moving body 200 may move substantially along the axial direction of the peripheral portion 110. That is, when the moving body 200 is released, the moving body 200 may move substantially along the central axis CA.

In some examples, the first retaining portion 130 may be releasably interlocked with the retained member (for example, the first locking portion 236 of the moving body 200 to be described below) through at least one structure of a buckle, a hook, a latch, or a pin. In this case, the first retaining portion 130 and the first locking portion 236 are releasably interlocked through at least one structure of a buckle, a hook, a latch, or a pin, thereby providing an interlocking manner that is easy to release.

In some examples, the number of first retaining portions 130 may be one or more, for example, 1, 2, 3, or 4. In some examples, if there are multiple first retaining portions 130, the multiple first retaining portions 130 may be symmetrically provided on the end portion 120. In the embodiment shown in Fig. 12A, the number of first retaining portions 130 may be 1.

In some examples, the first retaining portion 130 may have a surface facing the distal end, and the first locking portion 236 may have a surface facing the proximal end. When the moving body 200 is retained, the surface of the first retaining portion 130 facing the distal end and the surface of the first locking portion 236 facing the proximal end may engage with each other. In some examples, when the moving body 200 is released, the surface of the first retaining portion 130 facing the distal end and the surface of the first locking portion 236 facing the proximal end may separate from each other.

In some examples, at least a portion of the first retaining portion 130 may be L-shaped or hook-shaped. Thus, it is convenient to form a buckle structure to interlock with the first locking portion 236.

In some examples, referring to Fig. 12C, the first retaining portion 130 may include an arm 132 extending substantially along the direction of the central axis CA, and a protrusion 134 linked with the arm 132 and protruding toward the central axis CA along a direction substantially orthogonal to the central axis CA. In some examples, the arm 132 of the first retaining portion 130 may extend from the distal portion 120. In this case, when the protrusion 134 of the first retaining portion 130 is lapped with the first locking portion 236, the arm 132 is actuated to actuate the protrusion 134, thereby facilitating the release of the first locking portion 236 by the first retaining portion 130.

In some examples, the arm 132 of the first retaining portion 130 may have elasticity. In some examples, the arm 132 of the first retaining portion 130 may have elasticity in a direction substantially orthogonal to the central axis CA.

In some examples, the arm 132 of the first retaining portion 130 may tend to contract toward the central axis CA in a direction from the distal end to the proximal end. That is, in a natural state (i.e., a state not subjected to an external force), the end of the arm 132 close to the proximal end is closer to the central axis CA than the end of the arm 132 close to the distal end. In other examples, the arm 132 may also be substantially parallel to the central axis CA.

In some examples, when the arm 132 is subjected to an action in a direction substantially orthogonal to the central axis CA, the arm 132 may pivot. For example, when an action is applied to the arm 132 in a direction away from the central axis CA and substantially orthogonal to the central axis CA, the arm 132 may pivot, so that the end of the arm 132 close to the proximal end moves in a direction away from the central axis CA. Thus, the retained member can be easily released.

In some examples, when the arm 132 is subjected to an action in a direction substantially orthogonal to the central axis CA, the first retaining portion 130 may swing. For example, when an action is applied to the arm 132 in a direction away from the central axis CA and substantially orthogonal to the central axis CA, the arm 132 may swing, so that the end of the arm 132 close to the proximal end moves in a direction away from the central axis CA.

In some examples, the first retaining portion 130 may pivot or swing with the coupling position of the first retaining portion 130 and the distal end portion 120 as a fulcrum.

In some examples, the arm 132 of the first retaining portion 130 may approach the central axis CA along the direction from the distal end to the proximal end, and the protrusion 134 of the first retaining portion 130 may protrude toward the central axis CA. In this case, the first locking portion 236 can be retained on the inner side (close to the central axis CA) by folding the first retaining portion 130 inward as a whole, and the first locking portion 236 can be released by actuating the first retaining portion 130 towards the outer side (away from the central axis CA), thereby facilitating the retaining and release of the first locking portion 236.

In other examples, the arm 132 of the first retaining portion 130 may move away from the central axis CA in a direction from the distal end to the proximal end, and the protrusion 134 of the first retaining portion 130 may protrude away from the central axis CA. In this case, the first locking portion 236 can be retained on the outer side (away from the central axis CA) by integrally dispersing the first retaining portion 130 outward, and the first locking portion 236 can be released by actuating the first retaining portion 130 inward (close to the central axis CA), thereby facilitating the retaining and release of the first locking portion 236.

However, the examples of the present disclosure are not limited thereto, and the concept of the present invention is that the first retaining portion 130 forms a structure such as a buckle, a hook, a latch, or a pin to retain the retained member, and releases the retained member by moving the buckle, hook, latch, or pin away from the original position. Based on this concept, the arm 132 of the first retaining portion 130 and the protrusion 134 of the first retaining portion 130 may also approach or protrude in other directions as long as a buckle can be formed.

In some examples, the protrusion 134 of the first retaining portion 130 may have a surface facing the distal end. In some examples, the surface of the protrusion 134 facing the distal end may be substantially orthogonal to the central axis CA. The retained component (for example, the first locking portion 236 of the moving body 200 to be described below) may be retained by lapping/abutting against the surface of the protrusion 134 facing the distal end, so that it is retained and does not move toward the proximal end. When the arm 132 of the first retaining portion 130 is actuated to move the surface of the protrusion 134 facing the distal end away from the original position, that is, no longer abutting against the retained member, the retained member will be released.

That is, the first retaining portion 130 may be a structure such as a buckle, a hook, a latch, or a pin formed on the first housing 100 (for example, the distal portion 120 of the first housing 100). For example, the first retaining portion 130 may be formed as a finger-shaped, in-line, L-shaped, J-shaped, Z-shaped structure that can be decomposed into at least two parts, at least a part of which (for example, an arm connected to the first housing 100) can pivot, and another part (for example, a protrusion protruding outward from the arm and abutting against the retained member) can move away from the initial position along with the pivot, thereby releasing the hook, hang, latch, top, support, etc. of the retained member.

In some examples, referring to Fig. 12B, the first housing 100 may include a first limiting portion 180. In some examples, the first limiting portion 180 may be configured to maintain the balance state of the moving body 200 in a plane orthogonal to the central axis CA of the application device 1000. Thus, the probability of the moving body 200 deflecting and thus causing the application device 1000 to be discarded is reduced.

In some examples, the first limiting portion 180 may extend substantially along the direction of the central axis CA of the application device 1000. In some examples, the first limiting portion 180 may extend along the central axis CA in a direction from the distal end to the proximal end. In some examples, the first limiting portion 180 may be provided on the distal portion 120. In some examples, the first retaining portion 130, the first limiting portion 180, and the first housing 100 may be integrally formed.

In some examples, the number of first limiting portions may be one or more. For example, the number of first limiting portions may be 1, 2, 3, or 4.

In some examples, the number of first limiting portions 180 may be2. For example, referring to Figs. 12A and 12B, the first limiting portions may include a first limiting portion 180a and a first limiting portion 180b. The first limiting portion 180a and the first limiting portion 180b may be provided on opposite sides of the central axis CA. Preferably, the first limiting portion 180a and the first limiting portion 180b may be provided on opposite sides of the central axis CA substantially along the direction in which the first retaining portion 130 can be actuated. In this case, since the first retaining portion 130 is in contact with the first locking portion 236, when the first retaining portion 130 is actuated, the first locking portion 236 will be subjected to an action from the first retaining portion 130 along the actuation direction of the first retaining portion 130 and/or the direction opposite to the actuation direction, thereby causing the moving body 200 to tend to deflect. However, the action of the first limiting portion 180 can suppress the deflection of the moving body 200, thereby always maintaining the assembly accuracy between the moving body 200 and other structural members (for example, the second housing 140, the piercing member 260, etc.).

In some examples, the first limiting portion 180a may be arms respectively provided on both sides of the first retaining portion 130. In some examples, the second limiting portion 180b may be disposed opposite to the first retaining portion 130.

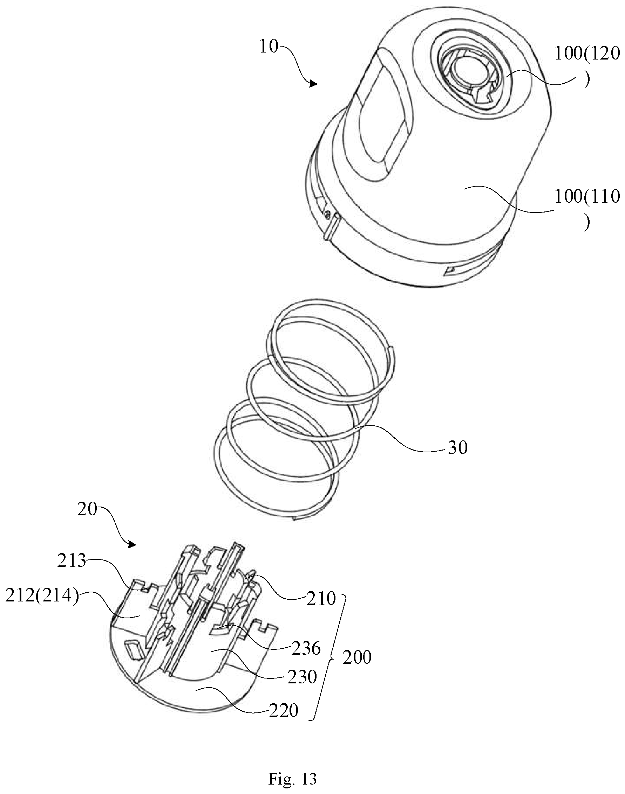

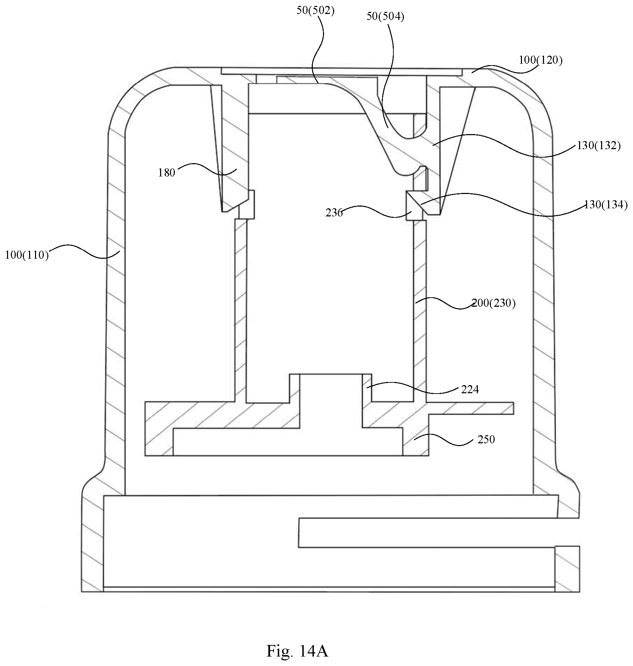

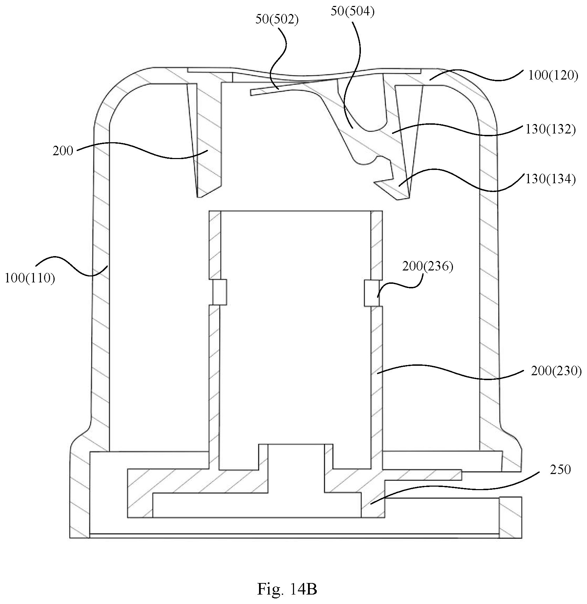

Fig. 13 is an exploded schematic diagram showing the first housing 100, the auxiliary mechanism 20, and the first driving mechanism 30 according to an example of the present disclosure; Fig. 14A is a cross-sectional schematic diagram showing the moving body 200 when retained according to an example of the present disclosure; Fig. 14B is a cross-sectional schematic diagram showing the moving body 200 when released according to an example of the present disclosure. It should be noted that Figs. 14A and 14B mainly show the retaining and release of the moving body 200 by the first retaining portion 130, and do not show the first driving mechanism 30 and other components.

In some examples, as described above, the auxiliary mechanism 20 may include a moving body 200 and an accommodating portion 250 provided on the moving body 200. The moving body 200 may be releasably retained on the first housing 100. In some examples, the moving body 200 may be releasably retained by the first retaining portion 130. After being released, the moving body 200 may be driven toward the proximal end to push the medical device 800 accommodated in the accommodating portion 250 toward the host.

In some examples, referring to Fig. 13, a first driving portion 30 may be provided between the moving body 200 and the first housing 100. The first driving portion 30 may apply an action to the moving body 200 in a direction toward the proximal end. After being released, the moving body 200 may be driven toward the proximal end by the first driving portion 30 to push the medical device 800 accommodated in the accommodating portion 250 toward the host.

In other examples, as described above, the application device 1000 may not include the first driving portion 30. In this case, after the moving body 200 is released, the moving body 200 may also be pushed toward the host by human power, thereby pushing the medical device 800 accommodated in the accommodating portion 250 toward the host.

In some examples, the moving body 200 may include a first bottom 210, a second bottom 220, and a sidewall 230 connecting the first bottom 210 and the second bottom 220 (see Fig. 13). The first bottom 210 may be close to the distal end, the second bottom 220 may be close to the proximal end, and the accommodating portion 250 may be provided on the second bottom 220.

In some examples, a hollow portion may be formed between the first bottom 210, the second bottom 220, and the sidewall 230. In some examples, the piercing member 260 may be provided in the hollow portion of the moving body 200.

In some examples, the first driving mechanism 30 may be provided between the auxiliary mechanism 20 and the first housing 100. In some examples, two ends of the first driving mechanism 30 may abut against the first housing 100 and the auxiliary mechanism 20, respectively.

In some examples, the auxiliary mechanism 20 may include a positioning portion 212 for positioning the first driving mechanism 30. In some examples, the positioning portion 212 may be provided on the second bottom 220. In some examples, the positioning portion 212 may be provided on the second bottom 220 in a manner surrounding the hollow portion. Thus, the first driving mechanism 30 can apply a stable driving action to the moving body 200 through the positioning portion 212.

In some examples, the positioning portion 212 may be a protrusion protruding from the second bottom 220 toward the distal end along the central axis CA. In some examples, the positioning portion 212 may be a columnar protrusion. In some examples, the positioning portion 212 may be cylindrical. In some examples, an end of the positioning portion 212 close to the distal end may have a groove for accommodating the first driving mechanism 30. Thus, the first driving mechanism 30 can stably abut against the auxiliary mechanism 20.

In some examples, the positioning portion 212 may include a plurality of support frames 214 formed on the first bottom 210 and arranged around the hollow portion 200 (see Fig. 13). In some examples, the plurality of support frames 214 may be formed on the sidewall 230 and symmetrically arranged around the hollow portion. In some examples, a side of the support frame 214 close to the distal end may have a groove 213 for supporting the first driving mechanism. Thus, the first driving mechanism 30 can stably abut against the plurality of support frames in a manner of sleeving the moving body to apply a stable driving force to the auxiliary mechanism.

In some examples, the plurality of support frames 214 may be formed on the sidewall 230 of the moving body 200. In some examples, the moving body 200 and the positioning portion 212 may be integrally formed.

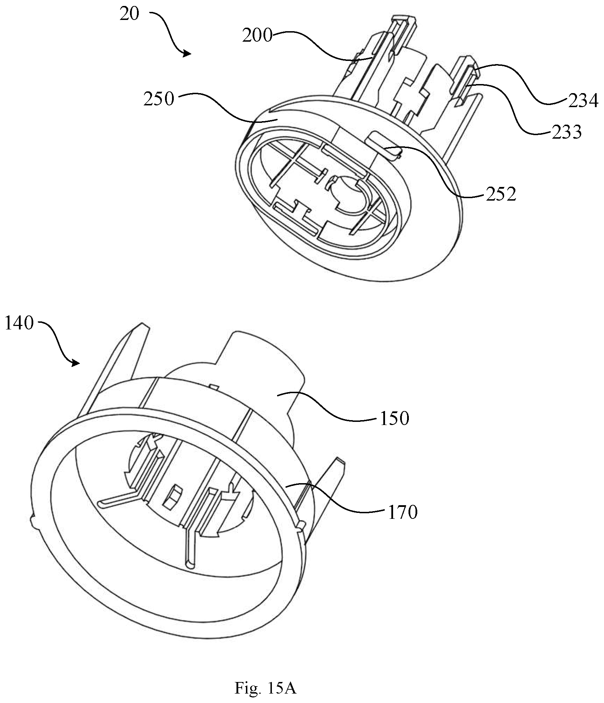

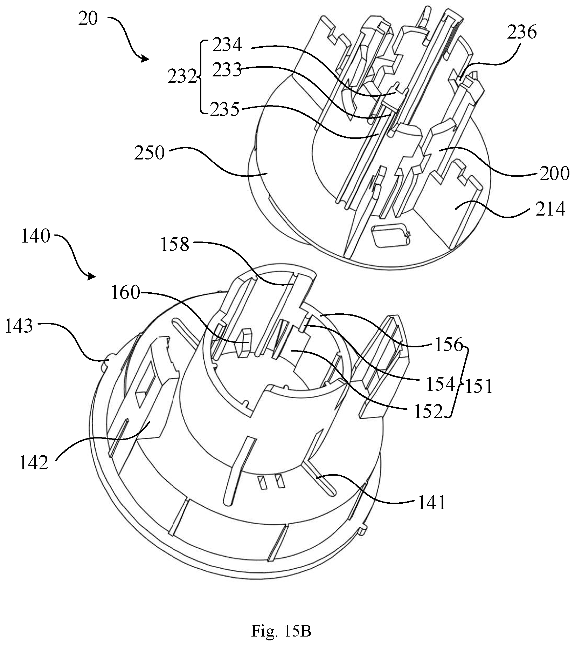

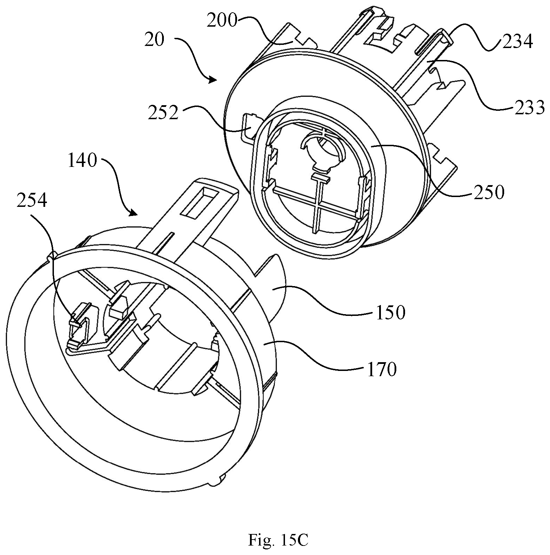

Fig. 15A is an exploded schematic diagram showing the second housing 140 and the auxiliary mechanism 10 from a first perspective according to an example of the present disclosure. Fig. 15B is an exploded schematic diagram showing the second housing 140 and the auxiliary mechanism 10 from a second perspective according to an example of the present disclosure. Fig. 15C is an exploded schematic diagram showing the second housing 140 and the auxiliary mechanism 10 from a third perspective according to an example of the present disclosure.

In some examples, the second housing 140 may have an opening groove 141 through which the positioning portion 212 extends (see Fig. 15A). Thus, during the assembly of the auxiliary mechanism 20 to the second housing 140, the positioning portion 212 can extend through the second housing 140 to abut against the first driving structure 30.

In some examples, the positioning portion 212 may be provided on the first bottom 210. In this case, the positioning portion 212 can also more stably limit the position where the first driving mechanism 30 applies an action to the moving body 200.

In some examples, the first driving mechanism 30 may have an energy storage state and an energy release state. When the first driving mechanism 30 is switched from the energy storage state to the energy release state, the first driving mechanism 30 may release energy and apply an action to the moving body 200 in a direction toward the proximal end. When the moving body 200 is retained, the first driving mechanism 30 may exist between the moving body 200 and the first housing 100 in the energy storage state, and when the moving body 200 is released, the first driving mechanism 30 may be switched from the energy storage state to the energy release state and apply an action to the moving body 200 in a direction toward the proximal end.

In some examples, the first driving mechanism 30 may have a compressed state and an extended state. When the first driving mechanism 30 is switched from the compressed state to the extended state, the first driving mechanism 30 may release energy. In some examples, the first driving mechanism 30 may be a compressible elastic component. In some examples, the first driving mechanism 30 may be a spring. Thus, an actuation mechanism can be provided for the moving body 200 through a simplified structural design.

In some examples, referring to Fig. 12A or Fig. 12B, the distal portion 120 may have a positioning portion 122. In some examples, the positioning portion 122 may be cylindrical. In this case, the other end of the first driving mechanism 30 may be sleeved on the positioning portion 122. In some examples, the positioning portion 122 may be hollow cylindrical. In this case, the other end of the first driving mechanism 30 may be sleeved on or embedded in the positioning portion 122. In some examples, the positioning portion 122 may be composed of at least two arc-shaped columns.

In addition, the application device 1000 of the present disclosure is not limited thereto. In some examples, the application device 1000 may not include the first driving mechanism 30. In this case, when the moving body 200 is released, the moving body 200 may also be moved toward the proximal end by applying an action to the moving body 200 manually.

In some examples, referring to Fig. 13, the moving body 200 may include a first locking portion 236 configured to be releasably interlocked with the first retaining portion 130. Thus, the moving body 200 can be releasably retained by the first retaining portion 130 through the cooperation between the first locking portion 236 and the first retaining portion 130.

In some examples, the first locking portion 236 may be provided on the sidewall 230 of the moving body 200. That is, the first locking portion 236 may be provided on the sidewall 230 of the moving body 200. The first locking portion 236 may cooperate with the first retaining portion 130 to retain the moving body 200 on the first housing 100.

In some examples, the number of first locking portion 236 may be one or more, for example, 1, 2, 3, or 4. In some examples, the number of first locking portion 236 may be the same as the number of first retaining portion 130. In the embodiment shown in Fig. 13, the number of first locking portion 236 is 1.

In some examples, if there are multiple first retaining portions 130, the number of first locking portions 236 is also multiple, and in the projection along the central axis CA, the positions of the multiple first locking portions 236 may be the same as the positions of the multiple first retaining portions 130.

In some examples, the first locking portion 236 may be formed as a buckle structure. Specifically, the first locking portion 236 may have a surface facing the proximal end, and the first retaining portion 130 may retain the moving body 200 by lapping, abutting, or engaging with this surface, and release the moving body 200 by moving away from this surface (see Figs. 14A and 14B). That is, the first retaining portion 130 and the first locking portion 236 may be formed as an interlocking structure, and in the direction along the central axis CA, the projections of the first retaining portion 130 and the first locking portion 236 have overlapping parts, and the first retaining portion 130 retains the first locking portion 236 through this overlapping part, and the first retaining portion 130 and/or the first locking portion 236 may move away from each other to eliminate this overlapping part, thereby releasing the first locking portion 236 by the first retaining portion 130. In some examples, the first retaining portion 130 and/or the first locking portion 236 may be actuated in a manner away from each other, thereby releasing the first locking portion 236 by the first retaining portion 130.

In some examples, the first locking portion 236 may be substantially parallel to the central axis CA or move away from the central axis CA in a direction from the proximal end to the distal end. In this case, the first locking portion 236 may be closer to the central axis CA than the first retaining portion 130, thereby facilitating cooperation with the inwardly folded first retaining portion 130 for interlocking.

In other examples, the first locking portion 236 may be substantially parallel to the central axis CA or approach the central axis CA in a direction from the proximal end to the distal end. In this case, the first locking portion 236 may be farther from the central axis CA than the first retaining portion 130, thereby facilitating cooperation with the outwardly dispersed first retaining portion 130 for interlocking.

In some examples, the first locking portion 236 may be formed in such a way that, for example, the outer surface of the sidewall 230 is concave toward the central axis CA. In some examples, the first locking portion 236 may be formed as a hollow structure, for example, a through hole penetrating the sidewall 230 in a direction substantially orthogonal to the central axis CA. In some examples, a notch communicating with the first locking portion 236 may be formed in the moving body 200, and the actuating portion 504 may be arranged at the distal end in a manner passing through the notch.

In some examples, the first locking portion 236 may also be formed in such a way that the peripheral edge of the first bottom 210 protrudes from the sidewall 230 in a direction orthogonal to the central axis CA. In this case, the first bottom 210 and the sidewall 230 may be fixedly connected. In other examples, the first locking portion 236 may also be in an inverted hook shape or L shape.

The examples of the present disclosure are not limited thereto. As described above, the concept of the present invention is that the first retaining portion 130 and the first locking portion 236 form a structure such as a buckle, a hook, a latch, or a pin for interlocking. In this case, the first locking portion 236 may have a surface facing the proximal end of the application device 1000, and the first retaining portion 130 retains the first locking portion 236 by engaging the surface of the first locking portion 236 facing the proximal end with the surface of the first retaining portion 130 facing the distal end, and the first retaining portion 130 releases the first locking portion 236 by separating the surface of the first locking portion 236 facing the proximal end from the surface of the first retaining portion 130 facing the distal end.

In some examples, referring to Figs. 14A and 14B, the first retaining portion 130 may be formed to be L-shaped, and the first locking portion 236 is formed to be a hollow structure provided on the sidewall 230. When the moving body 200 is retained, that is, as shown in Fig. 14A, the protrusion 134 of the first retaining portion 130 is located in the hollow structure of the first locking portion 236, thereby supporting the moving body 200 to retain the moving body 200. When the moving body 200 is released, that is, as shown in Fig. 14B, the arm 132 of the first retaining portion 130 pivots in a direction away from the central axis CA and moves the protrusion 134 in a direction away from the central axis CA, and the protrusion 134 leaves the hollow structure of the first locking portion 236, thereby releasing the moving body 200, and under the action of the first driving mechanism 30, the moving body 200 moves toward the proximal end.

In addition, in some examples, the application device 1000 may further include a first triggering mechanism 50 configured to separate the first retaining portion 130 and the first locking portion 236 to release the moving body 200 (see Fig. 12A, Fig. 12C, Fig. 14A, or Fig. 14B). Thus, the moving body 200 can be conveniently released through the first triggering mechanism 50.

In some examples, the application device 1000 may further include a first triggering mechanism 50. The first triggering mechanism 50 is configured to trigger the first retaining portion 130, so that the moving body 200 is released by the first retaining portion 130. In some examples, the first triggering mechanism 50 may be configured to actuate the first retaining portion 130 and/or the first locking portion 236 in a manner away from each other to release the moving body 200. For example, the first triggering mechanism 50 may be configured to actuate the first retaining portion 130 to move away from the central axis CA to release the retention of the first locking portion 236, thereby releasing the moving body 200. That is, the first triggering mechanism 50 may be configured to actuate the first retaining portion 130 and/or the first locking portion 236 to move away from each other, thereby releasing the first locking portion 236.

In some examples, the number of first triggering mechanism 50 may be one or more. For example, the number of first triggering mechanism 50 may be 1, 2, 3, or 4. In some examples, the number of first retaining portion 130 may be one or more. For example, the number of first retaining portion 130 may be 1, 2, 3, or 4. In some examples, the number of first retaining portion 130 may be the same as the number of first triggering mechanism 50.

In some examples, the number of first locking portion 236 may be one or more. For example, the number of first locking portion 236 may be 1, 2, 3, or 4. In some examples, the number of first locking portion 236 may be the same as the number of first retaining portion 130.

Hereinafter, the description will be made with the number of first triggering mechanism 50 being 2.

In some examples, the two first triggering mechanisms 50 may be symmetrically arranged. In some examples, the number of notche 290 may be 2. In some examples, the two notches 290 may be symmetrically arranged. In some examples, the moving body 200 may not have the first identification portion 239, and the support base 280 may not have the second identification portion 281.

In some examples, the first triggering mechanism 50 may include a pressing portion 502 and an actuating portion 504 (see Figs. 14A and 14B). In some examples, the first triggering mechanism 50 may include a pressing portion 502 that can be pressed during operation, and an actuating portion 504 configured to be linked with the pressing portion 502 and capable of actuating the first retaining portion 130.

In some examples, the first triggering mechanism 50 may be assembled to the distal portion 120. In some examples, the first triggering mechanism 50 may be formed on the distal portion 120. In some examples, applying an action to the pressing portion 502 may cause the actuating portion 504 to actuate the first retaining portion 130, thereby releasing the retention of the moving body 200 by the first retaining portion 130 to release the moving body 200.

In some examples, the actuating portion 504 may be configured to apply an action to the first retaining portion in a direction substantially orthogonal to the central axis CA of the application device to actuate the first retaining portion 130. In this case, when the actuating portion 504 is driven, the first retaining portion 130 can be actuated to move away from or close to the central axis CA, thereby unlocking the first retaining portion 130 from the locked component (for example, the first locking portion 236) in a manner away from or close to the central axis CA.

In some examples, the actuating portion 504 may extend in a direction having a preset angle with the central axis CA. In some examples, the preset angle may not be zero. In this case, when an action is applied to the actuating portion 504 at an appropriate angle, the actuating portion 504 can apply an action to the first retaining portion 130 in a direction orthogonal to the central axis CA of the application device to actuate the first retaining portion 130.

In some examples, the actuating portion 504 may be applied an action along the extending direction of the actuating portion 504 to actuate the first retaining portion 130. For example, when the preset angle is 90 degrees, an action may be applied to the actuating portion 504 in a direction orthogonal to the central axis CA to actuate the first retaining portion 130. Thus, the actuating portion 504 can actuate the first retaining portion 130 to release the first locking portion 236 in a manner close to or away from the central axis CA.

In some examples, when the preset angle is between 0 degrees and 90 degrees or between 90 degrees and 180 degrees, for example, 20 degrees, 30 degrees, 40 degrees, 45 degrees, 60 degrees, 70 degrees, 80 degrees, 100 degrees, 120 degrees, 130 degrees, 135 degrees, 150 degrees, 160 degrees, or 170 degrees, the action applied to the actuating portion 504 along the extending direction of the actuating portion 504 may be decomposed into an action parallel to the central axis CA and an action orthogonal to the central axis CA. Thus, the action orthogonal to the central axis CA can actuate the first retaining part 130 to release the first locking part 236 in a manner close to or away from the central axis CA.

In some examples, the actuating portion 504 may extend in a direction having a preset angle with the central axis CA and toward the distal end. Thus, the actuating part 504 can be conveniently applied with an action through the distal end.

In some examples, an action may be applied to the actuating portion 504 along the direction of the central axis of the application device 1000 to actuate the first retaining portion 130. In some examples, when an action is applied to the actuating portion 504 along the direction of the central axis CA, the actuating portion 504 may apply an action to the first retaining portion 130 along a direction orthogonal to the central axis CA.

In some examples, the actuating portion 504 may be coupled to the first retaining portion 130. In some examples, in response to applying an action to the actuating portion 504, the first retaining portion 130 may be driven to swing away from or toward the central axis CA of the application device 1000 with the coupling position of the first retaining portion 130 and the distal portion 120 as a fulcrum.

In some examples, an action may be applied to the actuating portion 504 along the direction of the central axis CA of the application device 1000. In this case, the first retaining portion 130 and the actuating portion 504 can form a lever system as a whole with the coupling position of the first retaining portion 130 and the distal portion 120 as a fulcrum. When the actuating portion 504 is driven by the action along the direction of the central axis CA, a torque can be generated to actuate the first retaining portion 130 to swing toward or away from the central axis CA with the coupling position as a fulcrum, thereby enabling the first retaining portion 130 to release the first locking portion 236 in a manner close to or away from the central axis CA.

In some examples, the actuating portion 504 may be coupled to the side of the first retaining portion 130 near the proximal end. In some examples, the actuating portion 504 may be coupled to the arm 134 of the first retaining portion 130. In some examples, the actuating portion 504 may be coupled to the side of the arm of the first retaining portion 130 near the proximal end. This facilitates actuation of the first retaining portion 130.

In some examples, the distal portion 120 may have a through structure. In some examples, the actuating portion 504 may be coupled to the first retaining portion 130 and extend to the through structure along a preset direction. The preset direction may refer to a direction having a preset angle with the central axis CA. This allows an action to be applied to the actuating portion 504 through the through structure, improving operational convenience. In some examples, the through structure may be an opening formed in the distal portion 120.

In some examples, the actuating portion 504 and the first retaining portion 130 may be integrally formed. In this case, the overall rigidity and stability of the actuating portion 504 and the first retaining portion 130 can be improved, which is suitable for transmitting the torque capable of driving the first retaining portion 130 to swing.

In some examples, an end of the actuating portion 504 adjacent to the first retaining portion 130 may have a curved structure. In this case, the length of the force arm when the first retaining portion 130 and the actuating portion 504 are integrated can be increased, thereby generating a torque sufficient to actuate the first retaining portion 130 to swing with a smaller applied force. In other words, applying a smaller force to the actuating portion 504 can actuate the first retaining portion 130. In addition, the curved structure can serve as a support point to improve the overall stability of the first retaining portion 130 and the actuating portion 504; in addition, the curved structure can change the direction of the force applied to the actuating portion 504, so that the force applied to the actuating portion 504 can be effectively transmitted to the first retaining portion 130 and actuate the first retaining portion 130 to swing toward or away from the central axis CA.

In some examples, after the action applied to the first trigger mechanism 50 disappears, the first trigger mechanism 50 and the first retaining portion 130 may return to their initial positions.

As described above, applying an action to the pressing portion 502 can cause the actuating portion 504 to actuate the first retaining portion 130. In some examples, the pressing portion 502 may be coupled to the actuating portion 504.

In some examples, the pressing portion 502 and the actuating portion 504 may be integrally formed. This simplifies the structural design of the first trigger mechanism 50 and improves the stability of the first trigger mechanism 50.

In some examples, the pressing portion 502 may be located at the distal portion 120. In some examples, the pressing portion may be located at the through structure of the distal portion 120. This allows the first retaining portion 130 to release the first locking portion 236 through a simple operation.

In some examples, the application device 1000 may further include a gasket 60 (see Fig. 11) assembled in the through structure and covering the through structure. In some examples, the first trigger structure 50 may be driven by applying an action at the position of the gasket 60.

In some examples, the pressing portion 502 may be generally disc-shaped. In some examples, the pressing portion 502 may be generally frustoconical. In some examples, the pressing portion 502 may be generally flush with the end portion 120. In some examples, the pressing portion 502 may penetrate the end portion 120.

In some examples, the pressing portion 502 may be located at the periphery of the housing.

In some examples, the first trigger mechanism 50, the first retaining portion 130, and the housing may be integrally formed. Thus, the overall structural design of the application device can be simplified.

In some examples, as described above, the actuating portion 504 of the first trigger mechanism 50 may actuate the first retaining portion 130, so that the first retaining portion 130 releases the retention of the moving body 200, thereby releasing the moving body 200.

In some examples, the actuating portion 504 may be formed as a structure extending from the pressing portion 502 toward the first retaining portion 130 (see Fig. 14A). In some examples, the number of actuating portions 504 may be one or more, for example, 1, 2, 3, or 4. In some examples, the number of actuating portions 504 may be the same as the number of first retaining portions 130.

As described above, the first retaining portion 130 may be parallel to the central axis CA in the direction toward the proximal end along the central axis CA. The actuating portion 504 is configured to actuate the first retaining portion 130 to move the end of the first retaining portion 130 close to the proximal end in a direction away from the central axis CA, thereby releasing the retention of the moving body 200.

Before the actuating portion 504 actuates the first retaining portion 130, the first retaining portion 130 and the first locking portion 236 form an interlock to retain the moving body 200 (see Fig. 14A). When the actuating portion 504 actuates the first retaining portion 130 (for example, by pressing the pressing portion 502 in a direction toward the proximal end), under the actuating action of the actuating portion 504, the first retaining portion pivots in a direction away from the central axis CA, so that the protrusion 134 of the first retaining portion 130 is separated from the first locking portion 236, thereby releasing the interlock between the first retaining portion 130 and the first locking portion 236, and thus releasing the moving body 200 (see Fig. 14B).

In some examples, the second housing 140 may include a first limiting mechanism 150 and a second limiting mechanism 170 (see Fig. 15A). In some examples, the first limiting mechanism 150 and the second limiting mechanism 170 may communicate with each other. In some examples, the first limiting mechanism 150 and the second limiting mechanism 170 may be hollow cylindrical structures. In some examples, the first limiting mechanism 150 may releasably retain the moving body 200 and may limit the moving path of the moving body 200. In some examples, the second limiting mechanism 170 may limit the accommodating position of the medical device 800.

In some examples, referring to Fig. 12B, the first housing 100 may have a third retaining portion 124. In some examples, referring to Fig. 15B, the second housing 140 may have a third locking portion 142. In some examples, the third retaining portion 124 may be coupled with the third locking portion 142. In some examples, the third retaining portion 124 may be releasably interlocked with the third locking portion 142 through at least one structure of a buckle, a hook, a latch, or a pin.

In some examples, during the assembly of the first housing 100 and the second housing 140, the third retaining portion 124 may be aligned with the third locking portion 142.

In some examples, the number of third retaining portion 124 may be one or more, for example, 1, 2, 3, or 4. In some examples, if there are multiple third retaining portions 124, the multiple third retaining portions 124 may be symmetrically provided on the inner wall of the first housing 100.

In some examples, the number of third locking portion 142 may be one or more, for example, 1, 2, 3, or 4. In some examples, if there are multiple third locking portions 142, the multiple third locking portions 142 may be symmetrically provided on the peripheral edge of the second housing 140.

In some examples, referring to Fig. 12B, the first housing 100 may have a third identification portion 125. In some examples, referring to Fig. 15B, the second housing 140 may have a fourth identification portion 143. In some examples, during the assembly of the first housing 100 and the second housing 140, the third identification portion 125 may be aligned with the fourth identification portion 143.

In some examples, the third identification portion 125 may be coupled with the fourth identification portion 143. In some examples, the coupling of the third identification portion 125 and the fourth identification portion 143 may be a male-female fit. For example, the shape of the third identification portion 125 may be a groove, and the shape of the fourth identification portion 143 may be a protrusion.

In some examples, the third identification portion 125 may abut against the fourth identification portion 143 in the circumferential direction of the housing 10. Thus, undesired relative rotation between the first housing 100 and the second housing 140 can be suppressed.

In some examples, the number of third identification portion 125 may be one or more. For example, the number of third identification portion 125 may be 1, 2, 3, or 4. In some examples, the number of fourth identification portion 143 may be one or more. For example, the number of fourth identification portion 143 may be 1, 2, 3, or 4. In some examples, the number of third identification portion 125 and the number of fourth identification portion 143 may be the same.

In some examples, during the assembly of the first housing 100 and the second housing 140, the third identification portion 125 may be aligned with the fourth identification portion 143 to align the third retaining portion 124 with the third locking portion 142. Thus, the third retaining portion 124 and the third locking portion 142 can be easily aligned during assembly.

In some examples, in the application device 1000 according to the present disclosure, the housing 10 may include a first housing 100 having a first retaining portion 130 and a second housing 140 that can be assembled to the first housing 100. The second housing 140 may have a first limiting mechanism 150 and a second limiting mechanism 170 that are communicated with each other. The second limiting mechanism 170 may be configured to limit the accommodating position of the medical device 800. The auxiliary mechanism 20 may be assembled to the first limiting mechanism 150 and can move along the first limiting mechanism 150 when the moving body 200 is released by the first retaining portion 130. In this case, the movement of the moving body 200 is limited by the first limiting mechanism 150 of the second housing 140, and the accommodating position of the medical device 800 is limited by the second limiting mechanism 170 of the second housing 140, thereby facilitating more accurate application of the medical device 800 to the host.

In some examples, the first limiting mechanism 150 may have a limiting portion 151 (see Fig. 15B). The limiting portion 151 may limit the moving body 200, thereby suppressing undesired rotation of the moving body 200. In some examples, the limiting portion 151 may be a groove and/or a ridge. In some examples, the limiting portion 151 may include a ridge portion 154 provided on the inner wall of the first limiting mechanism 150 and extending substantially along the central axis CA. In some examples, the limiting portion 151 may further include a groove 152 provided on the inner wall of the first limiting mechanism 150 and extending substantially along the central axis CA. In some examples, the length of the ridge portion 154 and the groove 152 may be less than the height of the first limiting mechanism 150, and the ridge portion 154 and the groove 152 may be substantially collinear in the direction of the central axis CA.

In some examples, the number of limiting portion 151 may be one or more, for example, 1, 2, 3, or 4. In some examples, the multiple limiting portions 151 may be uniformly distributed on the side wall of the first limiting mechanism 150.

In some examples, the ridge portion 154 of the limiting portion 151 may cooperate with the limited portion 232 of the moving body 200 including a groove structure, thereby suppressing undesired rotation of the moving body 200. In some examples, the moving body 200 may be assembled to the first limiting mechanism 150 along the groove 152. Thus, the groove 152 can provide a guiding function during assembly.

In some examples, the limiting portion 151 may further include a notch 156 provided on the first limiting mechanism 150 (see Fig. 15B). In some examples, the notch 156 may be used to provide an engaging space for the protrusion 234 of the moving body 200.

In some examples, the notch 156 may also be configured to expose the first locking portion 236 when the moving body 200 is retained by the first retaining portion 130. In this case, the first retaining portion 130 can retain the first locking portion 236 through the notch 156; in addition, by forming the notch 156 for exposing the first locking portion 236 in the first limiting mechanism 150, a sufficient movement stroke can be provided for the moving body 200 with a small space structure, which is beneficial to improve the integration degree of the application device.

In some examples, the first limiting mechanism 150 may further include a reinforcing rib 158 provided on the inner wall (see Fig. 15B). In some examples, the reinforcing rib 158 may extend substantially along the direction of the central axis CA. In some examples, the number of reinforcing rib 158 may be multiple, for example, 2, 3, 4, 6, etc. In some examples, the reinforcing rib 158 may be uniformly distributed on the inner wall of the first limiting mechanism 150. In some examples, the reinforcing rib 158 may provide line contact when the moving body moves relative to the first housing.

In some examples, the first limiting mechanism 150 may limit the stroke of the moving body 200 in the direction along the central axis CA. In this case, the stroke of the moving body 200 in the direction along the central axis CA is limited by the first limiting mechanism 150, thereby more accurately pushing the medical device 800 accommodated in the accommodating portion 250 by a predetermined distance, and thus more accurately applying the medical device 800 to the host.

In addition, in some examples, in the projection in the direction along the central axis CA, the accommodating portion 250 may at least partially overlap with the wall of the first limiting mechanism 150, and the end of the moving body 200 close to the distal end may at least partially overlap with the wall of the first limiting mechanism 150 (see Fig. 14A). In this case, by at least partially overlapping the accommodating portion 250 and the moving body 200 with the wall of the first limiting mechanism 150, it is possible to effectively limit the stroke of the moving body 200 along the first limiting mechanism 150 while simplifying the structure.

In addition, in some examples, a protrusion protruding in a direction substantially orthogonal to the central axis CA and away from the central axis CA may be provided on the end of the moving body 200 close to the distal end. In this case, a buckle is formed between this protrusion and the wall of the first limiting mechanism 150, thereby effectively limiting excessive movement of the moving body 200 toward the proximal end.

As described above, the auxiliary mechanism may include a positioning portion 212 for positioning the first driving mechanism 30. In some examples, the positioning portion 212 may be provided on a side of the accommodating portion 250 facing the distal end.

In some examples, the second limiting mechanism 170 may have an opening groove 141 through which the positioning portion 212 extends. In some examples, the opening groove 141 may extend from the second limiting mechanism 170 to the first limiting mechanism 150. In this case, the movement stroke of the moving body 200 along the central axis CA can be further limited through the cooperation of the positioning portion 212 and the moving body 200.

In some examples, the number of opening groove 141 may be the same as the number of support frame of the positioning portion 212. Thus, the positioning portion 212 can extend through the second housing 140 to abut against the first driving mechanism 30.

In addition, in some examples, the first limiting mechanism 150 may suppress rotation of the moving body 200. In this case, by suppressing rotation of the moving body 200, undesired rotation of the piercing member 260 during application can be suppressed.

In addition, in some examples, the first limiting mechanism 150 may include grooves and/or ridges continuously or discontinuously provided on the inner wall in a direction substantially along the central axis CA. In this case, by engaging the moving body 200 with the grooves and/or ridges of the first limiting mechanism 150, undesired rotation of the moving body 200 in the first limiting mechanism 150 can be effectively suppressed.

In addition, in some examples, the moving body 200 may include grooves and/or ridges provided on the outer wall of the sidewall 230 in a direction substantially along the central axis CA. In this case, by engaging the grooves and/or ridges of the moving body 200 with the grooves and/or ridges of the first limiting mechanism 150, undesired rotation of the moving body 200 in the first limiting mechanism 150 can be effectively suppressed.

In addition, in some examples, the first limiting mechanism 150 and the second limiting mechanism 170 may be hollow cylindrical, and the inner diameter of the first limiting mechanism 150 may be not greater than the inner diameter of the second limiting mechanism 170. In this case, by providing the first limiting mechanism 150 and the second limiting mechanism 170 as hollow cylindrical, the auxiliary mechanism 20 can be easily moved along the first limiting mechanism 150 and the second limiting mechanism 170.

In addition, in some examples, when the moving body 200 is released, the moving body 200 may move along the first limiting mechanism 150 and the accommodating portion 250 may move along the second limiting mechanism 170. In this case, by moving the moving body 200 along the first limiting mechanism 150 and the accommodating portion 250 along the second limiting mechanism 170, the medical device 800 accommodated in the accommodating portion 250 can be more accurately applied to a desired position on the host's body surface.

In some examples, a limited portion 232 (see Fig. 15B) may be provided on the sidewall 230 of the moving body 200. The limited portion 232 may cooperate with the limiting portion 151 of the first limiting mechanism 150 to suppress undesired rotation of the moving body 200.

In some examples, the limited portion 232 may include a groove 235 formed on the outer wall of the sidewall 230. In some examples, the groove 235 may be formed between two side-by-side ridges. In some examples, the limited portion 232 may further include an arm 233 formed by extending the sidewall 230 in the direction of the groove 235 toward the distal end. In some examples, the limited portion 232 may further include a protrusion 234 formed by protruding the arm 233 in a direction substantially orthogonal to the central axis CA and away from the central axis CA. In some examples, the number of limited portion 232 may be one or more, for example, 1, 2, 3, or 4. In some examples, the number of limited portion 232 may be the same as the number of limiting portion 151. In some examples, the multiple limited portions 232 may be uniformly distributed on the sidewall 230. In some examples, the arm 233 may have elasticity.

In some examples, in the projection in a direction substantially orthogonal to the central axis CA, the projection surface of the protrusion 234 of the limited portion 232 may be farther from the central axis CA than the projection of the notch 156 of the limiting portion 151. In addition, the projection of the ridge 154 may substantially engage with the projection of the groove 235. In addition, the projection of the protrusion 234 may substantially engage with the projection of the groove 152. In some examples, the length and/or width of the accommodating portion 250 may be greater than the diameter of the first limiting mechanism 150.

In some examples, the sidewall 230 of the moving body 200 may have a moving body limiting portion 238 (to be described later). The moving body limiting portion 238 may limit the piercing member 260 provided in the moving body 200, thereby suppressing undesired rotation of the piercing member 260. In some examples, the moving body limiting portion 238 may be formed as a groove and/or a ridge. In some examples, the moving body limiting portion 238 may be two side-by-side ridges and a groove between the two ridges.

In some examples, referring to Fig. 15C, the second housing 140 may include a magnet accommodating portion 252. As described above, the application device 1000 may include a magnet. In some examples, the magnet accommodating portion 252 may be configured to accommodate the magnet.

In some examples, the auxiliary mechanism 20 may have a passage portion 254 through which the magnet accommodating portion 252 extends. In some examples, during the assembly of the auxiliary mechanism 20 to the second housing 140, the magnet accommodating portion 252 may pass through the passage portion 254.

In some examples, the passage portion 254 may be provided on a side of the auxiliary mechanism 20 having the accommodating portion 250. In this case, by making the passage portion 254 close to the accommodating portion 250, when the medical device 800 has not been applied to the host (that is, the auxiliary mechanism 20 is assembled to the second housing 140), the magnet accommodated in the passage portion 254 can be closer to the medical device 800 accommodated in the accommodating portion 250, thereby helping to keep the switch module 884 of the electronic device 880 in an off state.

Embodiments of the present disclosure include:
B. An instrument kit for applying a medical device, the instrument kit including an application device for applying the medical device to a host, the application device including a housing and an auxiliary mechanism, the housing including a proximal end close to the host during operation and a distal end away from the host, the auxiliary mechanism releasably retaining the medical device and being movable relative to the housing when released, the auxiliary mechanism configured to be driven in a direction toward the proximal end when released to at least partially place the medical device under the skin of the host.

In some examples, Embodiment B may be combined with any one or more of the following additional elements. Element 1: The auxiliary mechanism includes a moving body releasably retained by the housing and configured to be movable relative to the housing when released, and an accommodating portion provided on the moving body and configured to receive and accommodate the medical device. Element 2: The housing includes a first retaining portion configured to releasably retain the moving body, and the moving body includes a first locking portion configured to be releasably interlocked with the first retaining portion. Element 3: The application device further includes a first triggering mechanism configured to separate the first retaining portion and the first locking portion to release the moving body. Element 4: It further including a sharp object and a support base configured to support the sharp object and assembled to the moving body, when the moving body is released, the moving body is driven toward the proximal end and at least partially places the medical device under the skin of the host through the sharp object.

Element 5: The application device further includes a first driving mechanism configured to apply an action to the auxiliary device in a direction toward the proximal end and drive the moving body to move toward the host when the auxiliary device is released. Element 6: The housing includes a first housing having the first retaining portion and a second housing that can be assembled to the first housing, the second housing having a first limiting mechanism and a second limiting mechanism that are communicated with each other, the auxiliary mechanism is assembled to the first limiting mechanism and can move along the first limiting mechanism when the moving body is released by the first retaining portion. Element 7: The first retaining portion has a surface facing the distal end, the first locking portion has a surface facing the proximal end, when the moving body is retained, the surface of the first retaining portion facing the distal end engages with the surface of the first locking portion facing the proximal end, and when the moving body is released, the surface of the first retaining portion facing the distal end separates from the surface of the first locking portion facing the proximal end. Element 8: The first retaining portion has an arm extending substantially along the direction of the central axis of the application device, and a protrusion linked with the arm and protruding substantially along a direction orthogonal to the central axis of the application device. Element 9: The arm of the first retaining portion has elasticity in a direction substantially orthogonal to the central axis of the application device. Element 10: The arm of the first retaining portion can pivot or swing toward or away from the central axis of the application device. Element 11: The arm of the first retaining portion approaches the central axis of the application device in a direction from the distal end to the proximal end, and the protrusion of the first retaining portion protrudes toward the central axis of the application device. Element 12: The arm of the first retaining portion moves away from the central axis of the application device in a direction from the distal end to the proximal end, and the protrusion of the first retaining portion protrudes away from the central axis of the application device. Element 13: The first locking portion is substantially parallel to the central axis of the application device or moves away from the central axis of the application device in a direction from the proximal end to the distal end. Element 14: The first locking portion is substantially parallel to the central axis of the application device or approaches the central axis of the application device in a direction from the proximal end to the distal end. Element 15: The first housing includes a hollow cylindrical peripheral portion and a distal portion provided at one end of the peripheral portion close to the distal end, and the first retaining portion is provided on the distal portion.

Element 16: The first housing further includes a first limiting portion extending substantially along the direction of the central axis of the application device, the first limiting portion configured to maintain the balance state of the moving body in a plane orthogonal to the central axis of the application device. Element 17: The first limiting portion is provided on the distal portion, and the first retaining portion, the first limiting portion, and the first housing are integrally formed. Element 18: The first triggering mechanism includes a pressing portion that can be pressed during operation, and an actuating portion configured to be linked with the pressing portion and capable of actuating the first retaining portion. Element 19: The actuating portion extends in a direction having a preset angle with the central axis of the application device. Element 20: The actuating portion extends in a direction having a preset angle with the central axis of the application device and toward the distal end.

Element 21: The first retaining portion is actuated in response to applying an action to the actuating portion along the extending direction of the actuating portion. Element 22: The first retaining portion is actuated in response to applying an action to the actuating portion in a direction along the central axis of the application device. Element 23: The actuating portion is coupled to the first retaining portion. Element 24: The actuating portion and the first retaining portion are integrally formed. Element 25: The first retaining portion is driven to swing away from or close to the central axis of the application device with the coupling position of the first retaining portion and the distal portion as a fulcrum in response to applying an action to the actuating portion. Element 26: The distal portion has a through structure, and the actuating portion is coupled to the first retaining portion and extends to the through structure along a preset direction. Element 27: An end of the actuating portion close to the first retaining portion has a curved structure. Element 28: The pressing portion is located in the through structure. Element 29: The first triggering mechanism, the first retaining portion, and the first housing are integrally formed. Element 30: The first triggering portion is configured to be actuated in a direction along the central axis of the application device. Element 31: When the moving body is released, the moving body can move along the first limiting mechanism and the accommodating portion can move along the second limiting mechanism. Element 32: The first limiting mechanism limits the stroke of the moving body in the direction along the central axis of the application device. Element 33: In the projection in the direction along the central axis of the application device, the accommodating portion at least partially overlaps with the wall of the first limiting mechanism, and the end of the moving body close to the distal end at least partially overlaps with the wall of the first limiting mechanism. Element 34: The first triggering mechanism and the first retaining mechanism are integrally formed on the housing.

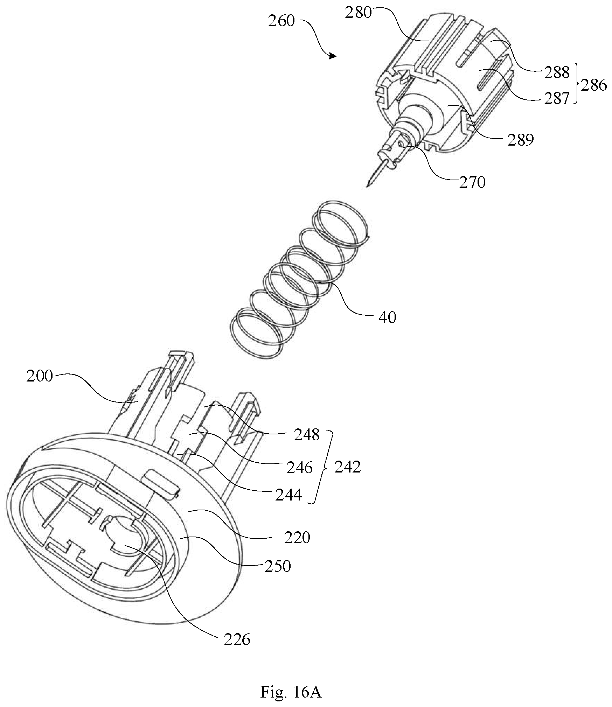

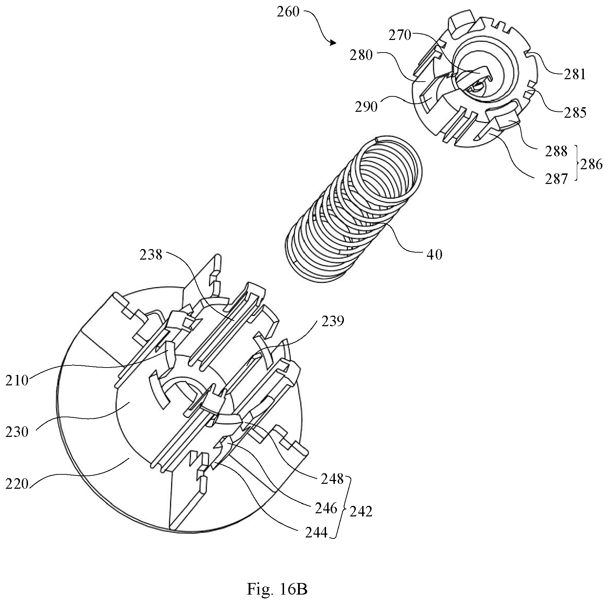

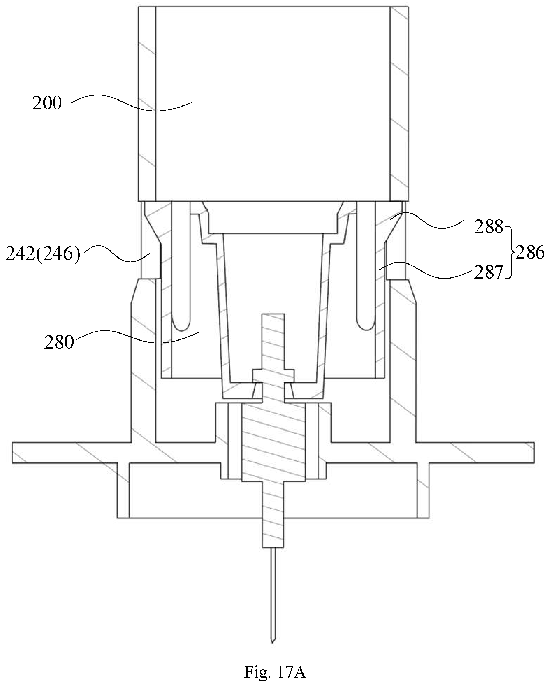

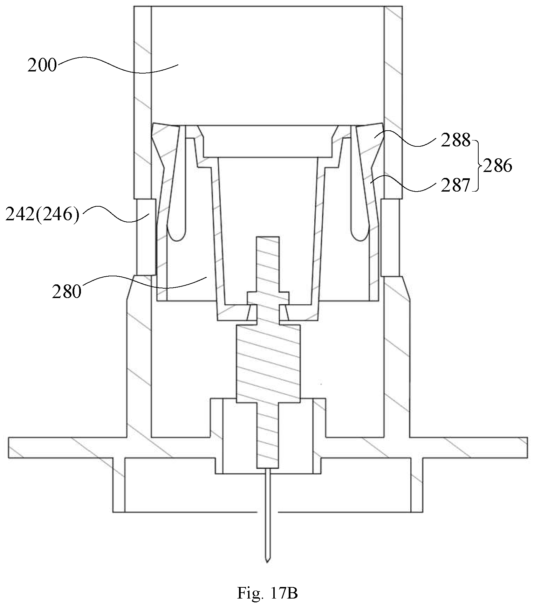

Fig. 16A is an exploded schematic diagram showing the moving body 200, the second driving mechanism 40, and the piercing member 260 from a first perspective according to an example of the present disclosure; Fig. 16B is an exploded schematic diagram showing the moving body 200, the second driving mechanism 40, and the piercing member 260 from a second perspective according to an example of the present disclosure; Fig. 17A is a cross-sectional schematic diagram showing the piercing member 260 when retained according to an example of the present disclosure; Fig. 17B is a cross-sectional schematic diagram showing the piercing member 260 when released according to an example of the present disclosure.

In some examples, the moving body 200 and the piercing member 260 may be integrally fixedly connected. In other examples, the moving body 200 and the piercing member 260 may be detachably assembled and combined. In some examples, the piercing member 260 may be releasably retained by the moving body 200.

In some examples, as described above, the moving body 200 may have a first bottom 210 close to the distal end, a second bottom 220 close to the proximal end, and a sidewall 230 connecting the first bottom 210 and the second bottom 220. In some examples, a hollow portion may be formed between the first bottom 210, the second bottom 220, and the sidewall 230. In some examples, referring to Fig. 16A, the accommodating portion 250 may be provided on the second bottom 220 and face the proximal end. In some examples, the piercing member 260 may be provided in the hollow portion. In some examples, the second bottom 220 may have a through hole 226. In addition, in some examples, the piercing member 260 may pass through the through hole 226.

In some examples, the piercing member 260 may be releasably provided on the moving body 200, and the piercing member 260 may be configured to be movable relative to the accommodating portion 250 when released. For example, when the piercing member 260 is released, it may move relative to the accommodating portion 250 in a direction away from the host.

In some examples, the application device 1000 may further include a second driving mechanism 40 (see Fig. 16A). The second driving mechanism 40 may be configured to apply an action to the piercing member 260 in a direction toward the distal end. When the piercing member 260 is released, the piercing member 260 may be driven by the second driving mechanism 40 toward the distal end to move the piercing member 260 away from the host.

In other examples, the application device 1000 may not include the second driving mechanism 40. In this case, the piercing member 260 may be manually driven to move away from the host. For example, in some examples, the piercing member 260 may also be integrally connected to the moving body 200, and the piercing member 260 may be moved away from the host by manually moving the moving body 200 in a direction away from the host.

In some examples, the piercing member 260 may be releasably provided in the hollow portion of the moving body 200. In some examples, when the piercing member 260 is released, it may move along the hollow portion of the moving body 200. In this case, the hollow portion is formed by the first bottom 210, the second bottom 220, and the sidewall 230, the accommodating portion 250 is provided on the second bottom 220, and the piercing member 260 is provided in the hollow portion, thereby facilitating movement of the piercing member 260 relative to the accommodating portion 250 when released. Thus, after the medical device 800 is applied to the host, the piercing member 260 is released and moves relative to the accommodating portion 250, thereby moving the piercing member 260 away from the host.

In other examples, the piercing member 260 may also be fixedly provided on the moving body 200. In this case, after the medical device 800 is applied to the host, the moving body 200 may be manually moved away from the host as a whole, thereby moving the piercing member 260 away from the host.

In addition, in some examples, the first limiting mechanism 150 may further include a protrusion 160 provided on the inner wall (see Fig. 15B, to be described in detail later).

In some examples, a second driving mechanism 40 may be provided between the second bottom 220 of the moving body 200 and the piercing member 260. The second driving mechanism 40 is configured to apply an action to the piercing member 260 toward the distal end. When the piercing member 260 is released, the second driving mechanism 40 may apply an action to the piercing member 260 toward the distal end to move the piercing member 260 away from the host. In some examples, the second driving mechanism 40 may be provided between the support base 280 of the piercing member 260 and the second bottom 220 of the moving body 200. Thus, the second driving mechanism 40 can apply an action to the piercing member 260 to move away from the accommodating portion 250.

In some examples, as described above, the moving body 200 may include a sidewall 230. In some examples, the moving body 200 may include a limiting portion 238 provided on the sidewall 230 (see Fig. 16B). The piercing member 260 may have a limited portion 285 (see Fig. 16B). When the piercing member 260 is assembled and combined with the moving body 200, the moving body 200 may limit the limited portion 285 through the limiting portion 238 to limit the movement of the piercing member 260. In some examples, the number of limiting portion 238 may be one or more, for example, 1, 2, 3, or 4. In some examples, the multiple limiting portions 238 may be uniformly distributed on the inner wall of the sidewall 230.

In some examples, the moving body 200 may further include a second retaining portion 242 (see Figs. 16A and 16B). The piercing member 260 may have a second locking portion 286 (see Figs. 16A and 16B). When the piercing member 260 is assembled and combined with the moving body 200, the moving body 200 may interlock with the piercing member 260 through the second retaining portion 242 to retain the piercing member 260. In some examples, the retention of the piercing member 260 by the second retaining portion 242 may be releasable. In some examples, the number of second retaining portion 242 may be one or more, for example, 1, 2, 3, or 4. In one example, the multiple second retaining portions 242 may be uniformly distributed on the sidewall 230.

In addition, in some examples, the moving body 200 may be configured to suppress rotation of the piercing member 260. In this case, by suppressing undesired rotation of the piercing member 260 during application, the user experience during application of the medical device 800 can be improved. In some examples, the moving body 200 may inhibit undesired rotation of the piercing member 260 during application through the limiting portion 238.

In addition, in some examples, the moving body 200 may include grooves and/or ridges provided on the inner wall of the sidewall 230 substantially along the direction of the central axis CA. In this case, by cooperating the grooves and/or ridges on the inner wall of the side wall of the moving body 200 with the grooves and/or ridges of the piercing member 260, undesired rotation of the piercing member 260 during application can be effectively inhibited. That is, in some examples, the limiting portion 238 of the moving body 200 may be grooves and/or ridges provided on the inner wall of the sidewall 230 substantially along the direction of the central axis CA.

In some examples, the limiting portion 238 of the moving body 200 may be formed as a groove structure. In some examples, the limiting portion 238 may include two ridges arranged side by side on the inner wall of the sidewall 230 substantially along the direction of the central axis CA, and a groove formed between the two ridges.

In addition, in some examples, the moving body 200 may have a notch 242 along the direction of the central axis CA (see Figs. 16A and 16B). In this case, by providing the notch 242 along the direction of the central axis CA on the moving body 200, it is convenient to apply an action to the piercing member 260 provided in the hollow portion of the moving body 200.

In addition, in some examples, a protrusion 160 protruding toward the central axis CA via the notch 242 may be provided on the inner wall of the first limiting mechanism 150. In this case, when the moving body 200 moves along the first limiting mechanism 150, the protrusion 160 provided on the inner wall of the first limiting mechanism 150 can apply an action to the piercing member 260 via the notch 242 of the moving body 200, thereby providing a triggering mechanism for the piercing member 260 with a simplified structure.

In some examples, the second retaining portion 242 of the moving body 200 may be a notch formed on the sidewall 230 (see Figs. 16A and 16B). In some examples, the second retaining portion 242 may include a first notch 244, a second notch 246, and a third notch 248 that extend substantially along the central axis CA and communicate with each other. The first notch 244 may be close to the proximal end, and the third notch 248 may be close to the distal end. In some examples, the width of the second notch 246 may be different from the width of the first notch 244 and the width of the third notch 248. In some examples, the width of the second notch 246 may be greater than the width of the first notch 244 and the width of the third notch 264.

In some examples, the piercing member 260 may include a sharp object 270 and a support base 280 for supporting the sharp object 270 (see Figs. 16A and 16B). In some examples, the support base 280 of the piercing member 260 may be provided in the hollow portion of the moving body 200, the accommodating portion 250 may be provided on the second bottom 220, the second bottom 220 may have a through hole 226, and the sharp object 270 may pass through the through hole 226 of the second bottom 220. In this case, by arranging the sharp object 270 to pass through the through hole 226 of the second bottom 220, the sharp object 270 can be engaged with the medical device 800 accommodated in the accommodating portion 250, thereby facilitating at least partially placing the medical device 800 under the host's skin through the piercing member 260.

In addition, in some examples, the moving body 200 may further include a positioning structure 224 provided on the second bottom 220 for positioning the second driving mechanism 40 (see Fig. 14A). In some examples, the positioning structure 224 may be columnar. In some examples, the positioning structure 224 may be hollow cylindrical. In this case, the second driving mechanism 40 may be positioned by being sleeved on the positioning structure 224, thereby limiting the driving position of the second driving mechanism 40.

In addition, in some examples, when the piercing member 260 is retained, the distance between the support base 280 and the first bottom 210 may be not less than the length of the sharp object 270 protruding from the accommodating portion 250. In this case, by setting the distance between the support base 280 and the first bottom 210 to be not less than the length of the sharp object 270 protruding from the accommodating portion 250, a moving space can be provided for the piercing member 260 to move away from the host, thereby reducing the risk of undesired injury to the host by the piercing member 260.

In other examples, the sharp object 270 and the support base 280 may be configured to be detachably assembled and combined. Thus, it is convenient to separately sterilize the sharp object 270.

In some examples, the limited portion 285 of the piercing member 260 may be formed as a ridge structure (see Fig. 16B). In some examples, the limited portion 285 may include two grooves arranged side by side on the side wall of the support base 280 substantially along the direction of the central axis CA, and a ridge formed between the two grooves. In some examples, the number of limited portion 285 may be one or more, for example, 1, 2, 3, or 4. In some examples, the number of limited portion 285 may be equal to the number of limiting portion 238.

Additionally, in some examples, the support base 280 may include grooves and/or ridges disposed on the outer wall substantially along the direction of the central axis CA. In this case, by coordinating the grooves and/or ridges of the support base 280 with the grooves and/or ridges on the inner wall of the side wall of the moving body 200, it can effectively suppress unintended rotation of the piercing member 260. That is, the limited portion 285 of the piercing member 260 may be formed to be grooves and/or ridges disposed on the outer wall substantially along the central axis CA.The limited portion 285 of the piercing member 260 can cooperate with the limiting portion 238 of the moving body 200 to suppress the piercing member 260 from rotating unexpectedly.

In some examples, the piercing member 260 may include a second locking portion 286 configured to releasably interlock with the second retaining portion 242 (see Fig. 17A). Thus, the piercing member 260 can be releasably retained by the second retaining portion 242 through cooperation between the second locking portion 286 and the second retaining portion 242. In some examples, the second locking portion 286 may be disposed on the support base 280 of the piercing member 260.

In some examples, the second locking portion 286 may include an arm 287 extending substantially along the central axis CA, and a protrusion 288 linked to the arm 287 and protruding away from the central axis CA in a direction substantially orthogonal to the central axis CA (see FIGS. 16A and 16B). In this case, when the piercing member 260 is placed in the hollow portion of the moving body 200, the second locking portion 286 can lap with the notch 242 of the moving body 200 (see Fig. 17A), enabling retention of the piercing member 260 with a simplified structure. In some examples, the arm 287 of the second locking portion 286 may have elasticity in a direction substantially orthogonal to the central axis CA of the application device 1000.

As described above, the moving body 200 may have a notch 242 extending substantially along the central axis CA. In some examples, when the piercing member 260 is retained, the protrusion 288 of the second locking portion 286 may pass through the notch 242. In this case, lapping the protrusion 288 of the second locking portion 286 with the notch 242 of the moving body 200 allows retention of the second locking portion 286 with a simplified structure.

Additionally, in some examples, when the piercing member 260 is released, the arm 288 of the second locking portion 286 may be pressed toward the central axis CA. This enables easy release of the second locking portion 286.

Furthermore, in some examples, the second retaining portion 242 and second locking portion 286 may be releasably interlocked via at least one structure of a buckle, hook, latch, or pin. In this case, the second retaining portion 242 and the second locking portion 286 are interlocked in a releasable manner through at least one structure of a buckle, hook, latch, or pin, thereby providing an easily releasable interlocking mechanism.

In some examples, the second locking portion 286 of the piercing member 260 may be formed as a shoulder structure. In some examples, the second locking portion 286 may include an arm 287 extending toward the distal end substantially along the central axis CA, and a protrusion 288 protruding from the end of the arm 287 in a direction substantially orthogonal to and away from the central axis CA. In some examples, the arm 287 may be elastic. In some examples, the arm 287 may gradually move away from the central axis CA in the direction toward the distal end along the central axis CA. In some examples, when projected along the central axis CA, the projection of the protrusion 288 may fully or partially overlap with the projection of the first notch 244. In some examples, the number of second locking portion 286 may be one or more, such as 1, 2, 3, or 4. In some examples, the number of second locking portion 286 may equal the number of second retaining portion 242.

In some examples, the width of the surface of the protrusion 288 facing the distal end may be smaller than the width of the second notch 246 and larger than the widths of the first notch 224 and the second notch 246.

In some examples, when assembling the first limiting mechanism 150 and the moving body 200, a protrusion 160 disposed on the inner wall of the first limiting mechanism 150 can sequentially pass through the third notch 248, the second notch 246, and the first notch 244. In this case, by providing the first notch 244, the protrusion 160 can protrude beyond the moving body 200; by providing the second notch 246, a retention mechanism for the piercing member 260 can be provided; additionally, by providing the third notch 248, a structure facilitating assembly can be provided. The protrusion 160 enters the first notch 244 after passing through the third notch 248 and the second notch 246, and the protrusion 288 of the second locking portion 286 projects outward through the second notch 246, thereby providing a retention mechanism for the piercing member 260. Furthermore, the protrusion 160 can pass through the first notch 244 to abut against the protrusion 288 of the second locking portion 286, thus also providing, via the protrusion 160 and the first notch 244, a triggering mechanism for releasing the second locking portion 286. In some examples, the widths of the second notch 246 and the first notch 244 may be the same.

In some examples, the protrusion 160 can act on the second locking portion 286 in a direction orthogonal to the central axis CA. In this case, the second locking portion 286 is biased inward and separates from the second locking portion 286 to move away from the host (refer to Fig. 17B).

In some examples, after the piercing member 260 is released by the moving body 200, the second driving mechanism 40 drives the piercing member 260 distally to cause the piercing member 260 to exit the host.

In some examples, the second driving mechanism 40 and the first driving mechanism 30 may have the same or similar configurations; for features of the second driving mechanism 40, refer to the description of the features of the first driving mechanism 30. In some examples, the second driving mechanism 40 may be a spring. Thereby, an actuation mechanism for the piercing member 260 can be provided through a simplified structural design.

In some examples, the second driving mechanism 40 may be disposed between the support base 280 and the second bottom 220, and when the piercing member 260 is retained, the distance between the support base 280 and the first bottom 210 is not less than the depth at which the sensor 820 is implanted subcutaneously. In this case, by providing sufficient movement space for the piercing member 260, it is advantageous for causing the piercing member 260 to exit the host.

In some examples, the moving body 200 further includes a first identification portion 239 disposed on the sidewall 230 (refer to Fig. 16B). In some examples, the first identification portion 239 may be used to restrict the assembly direction of the support base 280 into the moving body 200.

In some examples, the support base 280 may include a second identification portion 281 (refer to Fig. 16B) that can be coupled with the first identification portion 239. In this case, the moving body 200 and the support base 280 can have a unique assembly direction.

In some examples, the first identification portion 239 may be a rib or groove formed in the sidewall 230 and extending along the central axis CA, and the second identification portion 281 may be a groove or rib formed on the support base 280.

In some examples, after the piercing member 260 is triggered to unlock, the piercing member 260 can be driven to a position near the distal portion 120. In some examples, the piercing member 260 can be driven to a position abutting the distal portion 120. Thereby, the piercing member 260 can retract with the maximum stroke, reducing the risk of exposure of the piercing member 260.

In some examples, when the piercing member 260 is released, it can transition from a first path defined by the moving body 200 to a second path defined by the first housing 100.

In some examples, the process of the piercing member 260 being released and moving relative to the moving body 200 may include a first state where its movement path is defined by the moving body 200, a second state where its movement path is defined by both the moving body 200 and the first housing 100, and a third state where its movement path is defined by the first housing 100. Thereby, the piercing member 260 can exit the host along a predetermined path.

In some examples, referring to Fig. 12B, the first housing 100 may include a path-limiting portion 123 extending from the distal portion 120. The path-limiting portion 123 can be used to receive the piercing member 260 and define the movement path of the piercing member 260.

In some examples, the support base 280 may further include a notch 290 (refer to Fig. 16B). When the support base 280 is assembled into the application device 1000, the notch 290 may be disposed on the same side as the first retaining portion 130. In this case, when the piercing member 260 transitions from the first path defined by the moving body 200 to the second path defined by the first housing 100, the first retaining portion 130 and/or the actuation portion 504 can pass through the notch, thereby allowing the piercing member 260 to enter the second path unobstructed.

In some examples, the piercing member 260 may include a positioning portion 289 disposed on the support base 280 (refer to Fig. 16A). In some examples, the positioning portion 289 may be a columnar structure. One end of the second driving mechanism 40 may be sleeved over the positioning portion 289 of the piercing member 260, and the other end may be sleeved over a positioning portion 224 of the moving body 200 (refer to Fig. 14A). In this way, the second driving mechanism 40 is disposed between the moving body 200 and the piercing member 260.

In some examples, the application device 1000 further provides a triggering mechanism to cause the second retaining portion 242 to disengage the interlock with the second locking portion 286, thereby releasing the piercing member 260 from the moving body 200. That is, in some examples, the application device 1000 further includes a second triggering mechanism configured to cause the second retaining portion 242 and the second locking portion 286 to separate, thereby releasing the piercing member 260. Thereby, the piercing member 260 can be conveniently released via the second triggering mechanism.

As described above, the second locking portion 286 is formed as a shoulder structure capable of abutting against the second retaining portion 242, thereby being retained by the second retaining portion 242 in a buckle form. In some examples, such a triggering mechanism is provided, that is, it can actuate the second retaining portion 242 or the second locking portion 286 to cause them to separate.

In some examples, the triggering mechanism acting on the second retaining portion 242 and the second locking portion 286 may be disposed on the movement path of the second locking portion 286. When the moving body 200 moves proximally, this triggering mechanism can be activated. In some examples, this triggering mechanism may be a protrusion disposed on the movement path of the second locking portion 286. In this case, when the second locking portion 286 passes the protrusion, the arm 287 of the second locking portion 286 may be squeezed toward the central axis CA and elastically deform, causing the protrusion 288 of the second locking portion 286 to disengage from the second notch 246 and the third notch 248.

In some examples, as described previously, a protrusion 160 is provided on the inner wall of the first limiting mechanism 150 (refer to Fig. 15B). When the moving body 200 moves along the first limiting mechanism 150, the first notch 244, second notch 246, and third notch 248 of the moving body 200 can pass this protrusion. Moreover, when the piercing member 260 is retained within the hollow portion of the moving body 200, the second locking portion 286 of the piercing member 260 is squeezed by this protrusion 160 and deforms, moving away from the junction of the second notch 246 and the third notch 248, thereby releasing the piercing member 260.

Furthermore, when assembling the moving body 200 and the first limiting mechanism 150, the protrusion 160 can sequentially pass through the third notch 248, the second notch 246, and the first notch 244 until the moving body 200 is assembled and combined with the first limiting mechanism 150. By providing the protrusion 160 on the inner wall of the first limiting mechanism 150 and the first notch 244, second notch 246, and third notch 248 on the sidewall 230 of the moving body 200, it is possible to provide an in-motion triggering mechanism without adversely affecting the assembly of the moving body 200 and the first limiting mechanism 150.

In some examples, after the use of the instrument kit 1 is completed, an action can be applied to the moving body 200 in the proximal-to-distal direction to cause the moving body 200 to be locked onto the first retaining portion 130 again.

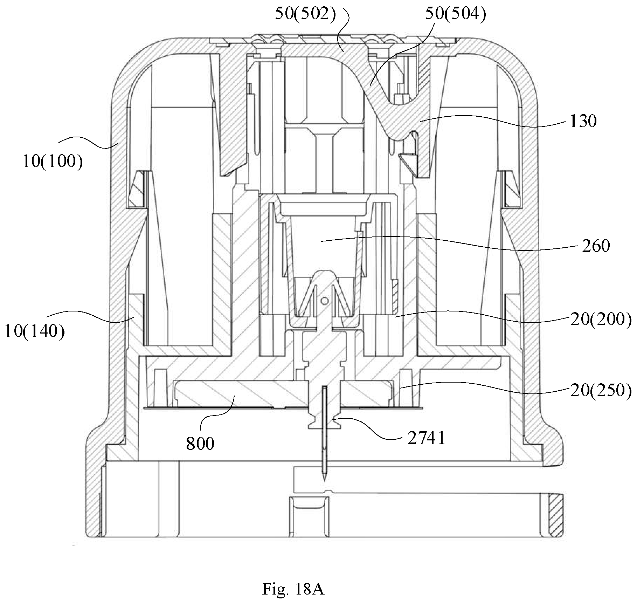

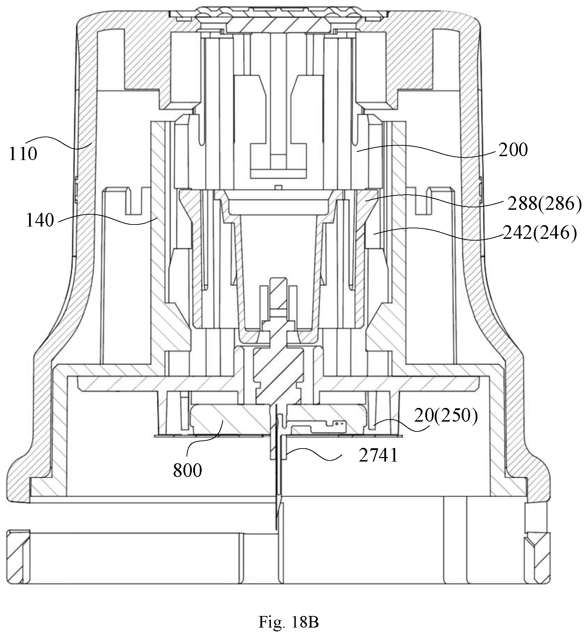

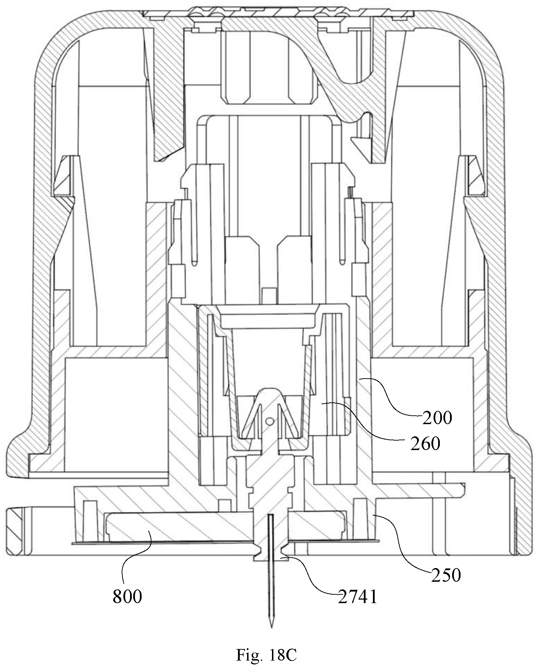

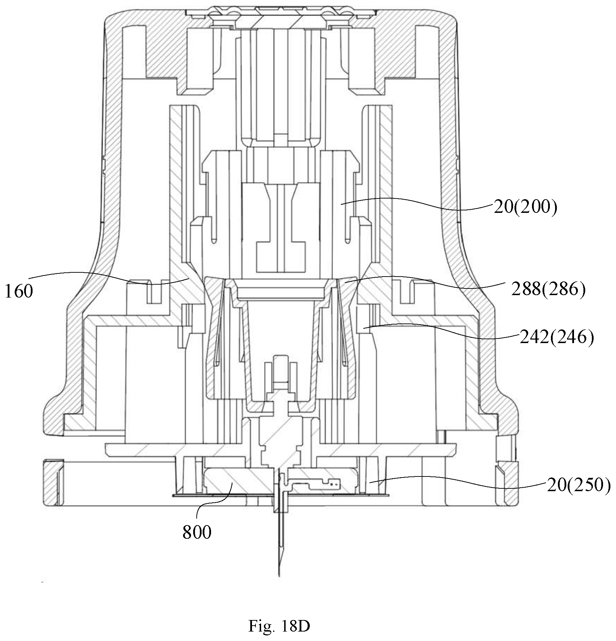

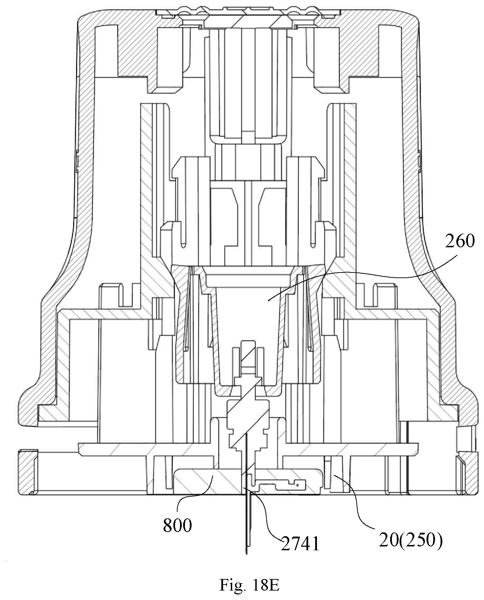

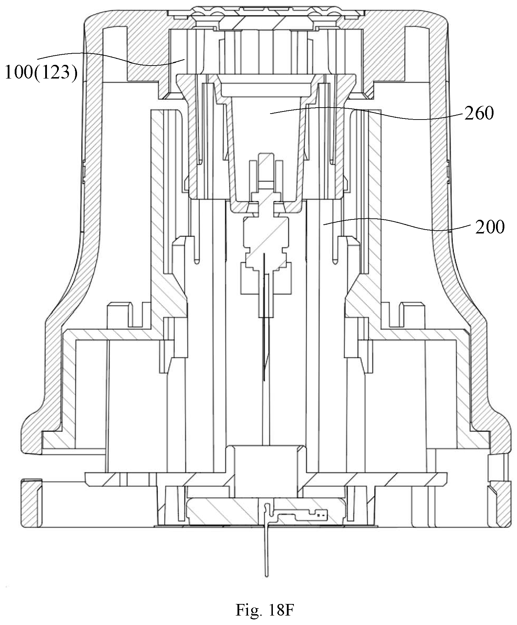

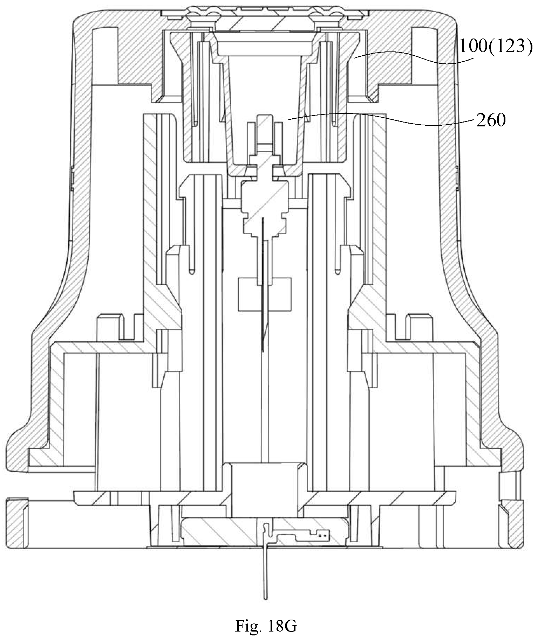

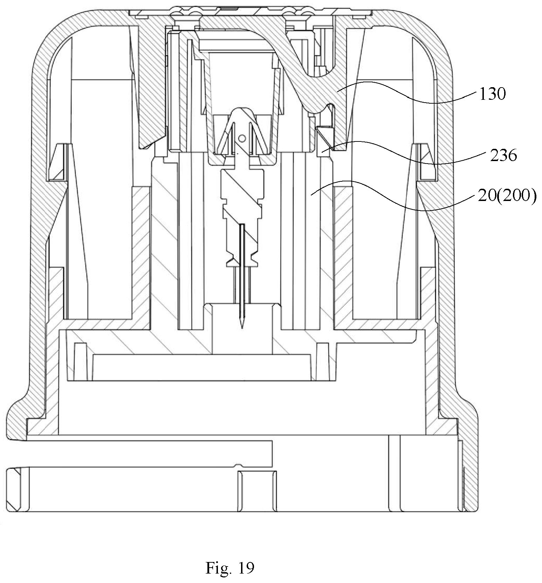

Fig. 18A is a cross-sectional view showing a first perspective of the instrument kit 1 according to an example of the present disclosure before applying the medical device 800; Fig. 18B is a cross-sectional view showing a second perspective of the instrument kit 1 according to an example of the present disclosure before applying the medical device 800. Fig. 18C is a cross-sectional view showing a first perspective of the instrument kit 1 according to an example of the present disclosure during application of the medical device 800; Fig. 18D is a cross-sectional view showing a second perspective of the instrument kit 1 according to an example of the present disclosure during application of the medical device 800; Fig. 18E is a cross-sectional view of the instrument kit 1 according to an example of the present disclosure when applying the medical device 800 to a host; Fig. 18F is a cross-sectional view of the instrument kit 1 according to an example of the present disclosure after applying the medical device 800, when the piercing member 260 is in a second state; Fig. 18G is a cross-sectional view of the instrument kit 1 according to an example of the present disclosure after applying the medical device 800, when the piercing member 260 is in a third state; Fig. 19 is a cross-sectional view of the instrument kit 1 according to an example of the present disclosure after applying the medical device 800, when the moving body 200 is locked again onto the first retaining portion 130. Note: Figs. 18A to 19 do not show the first driving mechanism 30 and the second driving mechanism 40.

Now, the application process of the application device 1000 is described again: Applying the medical device 800 to the host using the application device 1000 may include an application phase and a retraction phase. In the application phase, the medical device 800 is applied to the host; in the retraction phase, the piercing member 260 exits the host.

In some examples, referring to Figs. 18A and 18B, before applying the medical device 800, the moving body 200 is locked onto the first retaining portion 130.

In the application phase, by pressing the pressing portion 502 of the first triggering mechanism 50, the actuation portion 504 of the first triggering mechanism 50 actuates the first retaining mechanism 130 to pivot in a direction away from the central axis CA, causing the first retaining portion 130 to disengage from the first locking portion 236, thereby releasing the moving body 200; subsequently, the first driving mechanism 30 applies a force to the moving body 200 in the proximal direction to cause the moving body 200 to move proximally; then, under the action of the piercing member 260, the medical device 800 accommodated in the accommodating portion 250 is at least partially placed subcutaneously in the host.

In some examples, the application device 1000 may not include the first driving mechanism 30. In this case, after the moving body 200 is released, the moving body 200 can also be driven toward the host by manual force, thereby applying the medical device 800 accommodated in the accommodating portion 250 to the host via the piercing member 260.

In the retraction phase, as the moving body 200 moves proximally along the first limiting mechanism 150, the protrusion 160 disposed on the inner wall of the first limiting mechanism 150 passes the second notch 246 disposed on the moving body 200 and approaches the second locking portion 286 of the piercing member 260, and the protrusion 160 squeezes the second locking portion 286 to cause the second locking portion 286 to pivot in a direction toward the central axis CA, causing the second locking portion 286 to disengage from the second retaining portion 242 (refer to Figs. 18C and 18D); subsequently, the second driving mechanism 40 applies a force to the piercing member 260 in the distal direction to cause the piercing member 260 to move distally and exit the host.

In some examples, referring to Figs. 18A and 18B, the piercing member 260 may include an engaging portion 2741 projecting beyond the bottom of the medical device 800. In some examples, when the second locking portion 286 disengages from the second retaining portion 242, the medical device 800 has not yet been applied to the host (refer to Figs. 18C and 18D). At this moment, the moving body 200 and the accommodating portion 250 may continue to be driven in the proximal direction, while the piercing member 260 may be driven in the distal direction. In this case, because the piercing member 260 is triggered to unlock earlier, when the medical device 800 is applied to the host, the piercing member 260 has already moved a certain distance relative to the moving body 200 in the distal direction. Consequently, the engaging portion 2741 can assist in applying the medical device 800 to the host in a posture where it does not project beyond the bottom of the medical device 800, thereby improving user comfort. For example, referring to Figs. 18D and 18E, when the medical device 800 is applied to the host, the bottom of the engaging portion 2741 is flush with the bottom of the medical device 800. In some examples, when the medical device 800 is applied to the host, the bottom of the engaging portion 2741 may not project beyond the bottom of the medical device 800.

In some examples, by appropriately setting the timing when the puncture mechanism 260 is triggered to unlock, during the entire application process, the bottom of the engaging portion 2741 may not project beyond the proximal end of the housing 10. That is, the unlocking of the puncture mechanism 260 can be triggered when the bottom of the engaging portion 2741 moves to the proximal end of the housing 10 or before it moves there. In this case, the bottom of the engaging portion 2741 will not act on the host, improving user comfort.

In some examples, referring to Fig. 18E, when the medical device 800 is applied to the host, a protrusion 234 of the moving body 200 may abut against a notch 156. Specifically, the lower surface of the protrusion 234 of the moving body 200 may abut against the upper surface of the notch 156. Thereby, further proximal movement of the moving body 200 can be limited, facilitating subsequent distal movement of the piercing member 260 away from the host.

In some examples, the piercing member 260 and the moving body 200 may also be fixedly connected. Additionally, in some examples, the application device 1000 may not include the second driving mechanism 40. In this case, after the medical device 800 is applied to the host, the moving body 200 can be driven entirely away from the host by manual force, and consequently, the piercing member 260 fixedly connected to the moving body 200 can follow the moving body 200 to exit the host.

Additionally, it should be noted that during the retraction phase, the first driving mechanism 30 (or manual force) can still apply a force to the moving body 200 toward the host. In other words, for a first driving mechanism 30 configured as an elastic component (e.g., a spring), when the moving body 200 is driven toward the host and the medical device 800 accommodated in the accommodating portion 250 is adhered to the host's body surface, the first driving mechanism 30 may still be in an energy-stored state. In this case, during the retraction phase, the medical device 800 adheres tightly to the host's body surface under the action of the first driving mechanism 30, thereby effectively reducing the possibility of the medical device 800 detaching from the host during retraction.

In some examples, referring to Figs. 18F and 18G, the piercing member 260 can be driven to a position abutting the first housing 100. In some examples, the path-limiting portion 123 can receive the protrusion 288 of the second locking portion 286. In some examples, the size of the protrusion 288 of the second locking portion 286 may be equal to or slightly smaller than the size of the path-limiting portion 123. Here, Fig. 18F shows the piercing member 260 in a second state where its movement path is limited by both the moving body 200 and the path-limiting portion 123 of the first housing 100; Fig. 18G shows the piercing member 260 in a third state where its movement path is limited by the path-limiting portion 123 of the first housing 100.

In some examples, referring to Fig. 19, the moving body 200 can be locked again onto the first retaining portion 130. In some examples, an action can be applied to the moving body 200 in the distal direction to cause the moving body 200 to be locked again onto the first retaining portion 130. In some examples, when the moving body 200 can be locked again onto the first retaining portion 130, the cap 700 can be coupled to the housing 10. Thereby, facilitating the organization and storage of the instrument kit 1.

Embodiments of the present disclosure include:
C. An instrument kit, the instrument kit comprising a piercing member, and an application device cooperating with the piercing member to apply the medical device to a host, the application device comprising a housing and an auxiliary mechanism, the housing comprising a proximal end configured to be proximate to the host during operation and a distal end configured to be distal from the host, the auxiliary mechanism releasably retaining the medical device and being movable relative to the housing when released, the auxiliary mechanism configured to be driven towards the proximal end when released to position at least a portion of the medical device subcutaneously within the host via the piercing member, responsive to an action applied to the application device in a distal direction to drive the piercing member out of the host.

In some examples, embodiment C can be combined with any one or more of the following additional elements. Element 1: The auxiliary mechanism comprises a moving body releasably retained by the housing and configured to be movable relative to the housing when released, and a accommodating portion disposed on the moving body and configured to receive and accommodate the medical device. Element 2: The piercing mechanism is releasably retained by the moving body and configured to be movable relative to the accommodating portion when released. Element 3: It further comprising a second drive mechanism configured to apply an action to the piercing member in the distal direction. Element 4: When the piercing mechanism is released, the piercing member is driven out of the host responsive to the action applied to the piercing member by the second drive mechanism. Element 5: The piercing member comprises a sharp object having a groove, and a support base for supporting the sharp object, the medical device being entirely or partially placeable in the groove of the sharp object, the second drive mechanism configured to apply an action to the support base.

Element 6: The moving body comprises a second retaining portion for retaining the piercing member, the piercing member comprising a second locking portion configured to be releasably interlockable with the second retaining portion. Element 7: The moving body has a first bottom near the distal end, a second bottom near the proximal end, a side wall connecting the first bottom and the second bottom, and a hollow portion formed between the first bottom, the second bottom, and the side wall, the accommodating portion disposed on the second bottom and movable along the hollow portion when the piercing member is released. Element 8: The second locking portion comprises an arm extending substantially along a direction of a central axis of the application device, and a protrusion disposed on the arm and protruding substantially orthogonally to the central axis of the application device and away from the central axis of the application device. Element 8: The moving body has a notch substantially along the direction of the central axis of the application device, and when the piercing member is retained, the protrusion of the second locking portion passes through the notch.

Element 9: The piercing member comprises a second locking portion configured to be releasably interlockable with the second retaining portion and disposed on the support base, the second locking portion comprising an arm extending substantially along the direction of the central axis of the application device, and a protrusion linked to the arm and protruding substantially orthogonally to the central axis of the application device and away from the central axis of the application device. Element 10: A protrusion is provided on the inner wall of the first limiting mechanism, which can protrude towards the central axis of the application device via the notch. Element 2: The arm of the second locking portion has elasticity in a direction substantially orthogonal to the central axis of the application device.

Element 11: The moving body has a notch substantially along the direction of the central axis of the application device, and when the piercing member is retained, the protrusion of the second locking portion passes through the notch. Element 12: When the piercing member is released, the arm of the second locking portion is squeezed towards the central axis of the application device.

Element 13: When the piercing member is released, it transitions to a second path defined by the first housing along a first path defined by the moving body. Element 14: The first housing comprises a path-limiting portion disposed at the distal end for defining the second path. Element 15: In projection along the direction of the central axis of the application device, the path-limiting portion defining the second path at least partially overlaps with the moving body. Element 16: The process of the piercing member being released and moving relative to the moving body includes a first state where its movement path is defined by the moving body, a second state where its movement path is defined by both the moving body and the housing, and a third state where its movement path is defined by the housing. Element 17: The support base has a notch substantially along the direction of the central axis of the application device, when the piercing member transitions to the second path, the first retaining portion passes through the notch. Element 18: The moving body can be actuated in the direction towards the distal end to cause the first locking portion to lock onto the first retaining portion. Element 17: The support base is configured to be assembled to the moving body in a predetermined direction.

In some examples, embodiment B, and embodiments combining embodiment B with any one or more of corresponding elements 1 to 17, can be combined with embodiment A or combinations combining embodiment A with its corresponding elements.

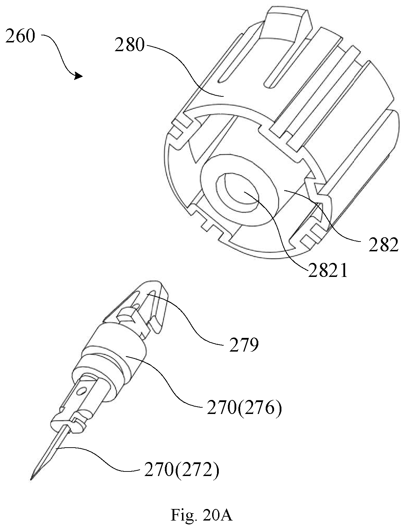

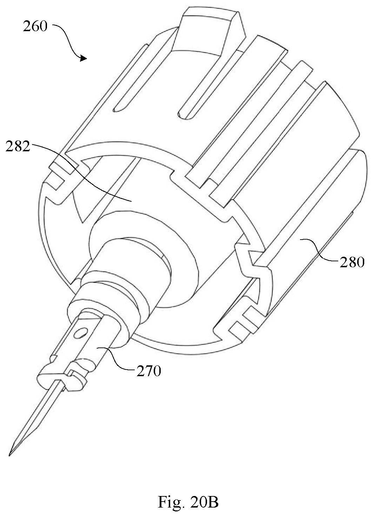

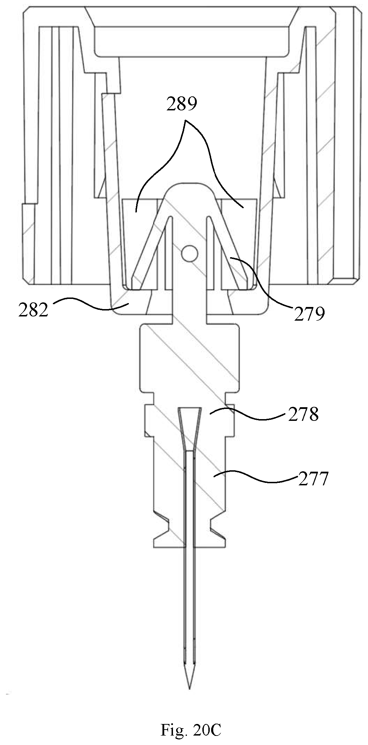

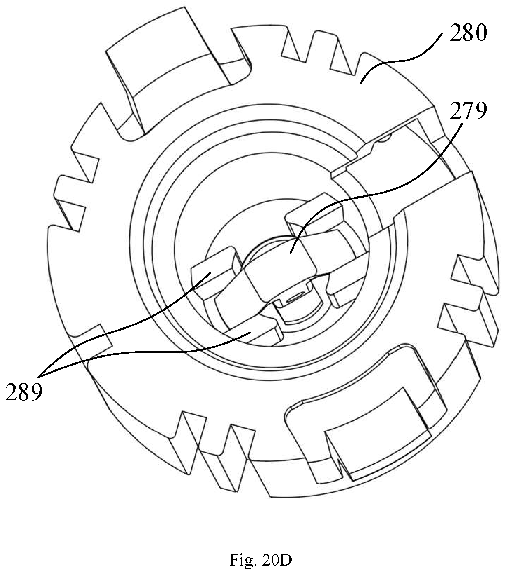

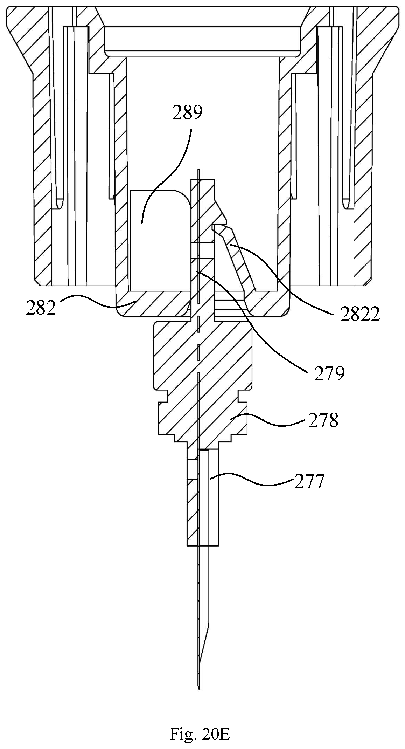

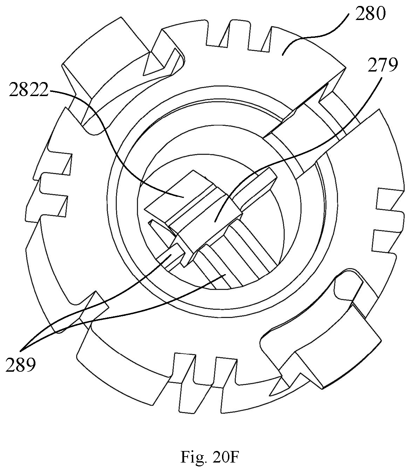

Fig. 20A is an exploded schematic diagram illustrating a piercing member 260 according to an example of the present disclosure; Fig. 20B is a schematic diagram illustrating a first perspective view of the assembled piercing member 260 according to an example of the present disclosure; Fig. 20C is a cross-sectional view illustrating the piercing member 260 according to an example of the present disclosure; Fig. 20D is a schematic diagram illustrating a second perspective view of the assembled piercing member 260 according to an example of the present disclosure. Fig. 20E is a cross-sectional view illustrating another structure of the piercing member 260 according to an example of the present disclosure. Fig. 20F is an appearance view illustrating another structure of the piercing member 260 according to an example of the present disclosure.

As mentioned above, referring to Figs. 20A and 20B, the piercing member 260 may comprise a sharp object 270 and a support base 280 supporting the sharp object 270. In some examples, the sharp object 270 and the support base 280 may be integrally formed. In other examples, the sharp object 270 may be separably assembled and combined with the support base 280. In some examples, the sharp object 270 may have a groove, and the medical device 800 may be entirely or partially placed within this groove. In some examples, a second drive mechanism 40 may apply an action to the support base 280. In this case, by placing the medical device 800 entirely or partially within the groove of the sharp object 270, it facilitates positioning at least a portion of the medical device 800 subcutaneously within the host via the piercing member 260. In some examples, an implantable portion of a sensor 820 may be placed within a groove 274 of the sharp object 270.

In some examples, the sharp object 270 may comprise a needle portion 272 (see Fig. 20A). In some examples, a groove extending along the length direction of the needle portion 272 may be provided on the needle portion 272. The medical device 800 may be at least partially disposed within a groove of the sharp object 270. In this case, the sensor 820 can be received within the groove, facilitating the sensor 820 to follow the sharp object 270 and be positioned subcutaneously within the host.

In some examples, the sharp object 270 may comprise a cap portion 276 connected to the needle portion 272 (see Fig. 20A). In some examples, the needle portion 272 may be assembled to the support base 280 via the cap portion 276.

In some examples, referring to Fig. 20A, the support base 280 may comprise a hole 2821 configured to receive the cap portion 276. The cap portion 276 may extend through the hole 2821 and be assembled to the support base 280. In some examples, the support base 280 may comprise a sharp object fixing portion 282 having the hole 2821. In some examples, the cap portion 276 may be fixed to the sharp object fixing portion 282.

In some examples, the cap portion 276 may comprise a second part 278 and a third part 279 connected sequentially (see Fig. 20C). In some examples, the sharp object 270 may be fixed to the support base 280 via the third part 279.

In some examples, the third part 279 has multiple fingers configured to snap-fit onto the support base 280. In some examples, the cap portion 276 has a top end distal from a sensor control device and an end opposite the top end. The multiple fingers are formed at the top end and extend away from a central axis of the sharp object 270 in a direction from the top end towards the bottom end. Thereby, the sharp object 270 can be fixed to the support base 280 in a unidirectional locking manner.

In some examples, the number of fingers may be 2, 3, 4, 5, or 6, etc.

In some examples, the third part 279 may have elasticity. In some examples, the sharp object fixing portion 282 may have elasticity.

In some examples, during the assembly process, the multiple fingers may be squeezed towards the central axis CA, and after the multiple fingers extend through the hole 2821, they may restore to an expanded posture. Thereby, they can snap-fit onto the support base 280.

In some examples, the sharp object fixing portion 282 may further comprise a position restricting portion 289 configured to inhibit rotation of the sharp object 270 (see Figs. 20C and 20D). In some examples, the position restricting portion 289 may be formed as a clamping groove restricting circumferential movement of the fingers. In some examples, the position restricting portion 289 may be formed by two adjacent protrusions creating a clamping groove that restricts circumferential movement of the fingers.

In some examples, when the sharp object 270 is assembled to the support base 280, the fingers may snap-fit into grooves. Thereby, rotation of the sharp object 270 can be inhibited.

In some examples, referring to Figs. 20E and 20F, the third part 279 may have fixing teeth configured to snap-fit onto the support base 280. In some examples, the fixing teeth may have an abutting surface inclined away from the central axis CA of the sharp object 270 in the direction from the top end to the bottom end. Thereby, the sharp object 270 can be fixed to the support base 280 in a unidirectional locking manner.

In some examples, the sharp object fixing portion 282 may have a sharp object fixing arm 2822. In some examples, the sharp object fixing arm 2822 may have elasticity.

In some examples, during the assembly process, the fixing teeth may squeeze the sharp object fixing arm 2822 away from the central axis CA, and after the fixing teeth extend beyond an end of the sharp object fixing arm 2822 distal from the hole 2821, they may restore to an expanded posture. Thereby, they can snap-fit onto the support base 280.

In some examples, referring to Fig. 20F, there may be multiple position restricting portions 289. In some examples, the multiple position restricting portions 289 may abut against three planes of the third part 279 that do not have fixing teeth. Thereby, rotation of the sharp object 270 can be inhibited.

In some examples, the second part 248 may be a structure where its central geometric dimension is larger than the diameter of the hole 2821, such as a cylinder with a diameter larger than that of the hole 2821. Thereby, the second part 248 can limit the axial travel of the sharp object 270. Further, the cooperation of the second part 248 and the third part 279 can axially fix the sharp object 270 to the support base 280.

In some examples, the cap portion 276 may comprise a first part 277, a second part 278, and a third part 279 connected sequentially. Among them, the first part 277 may be used to cooperate with the medical device 800.

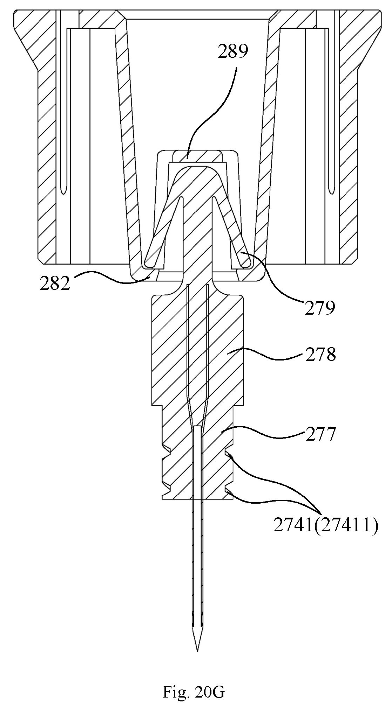

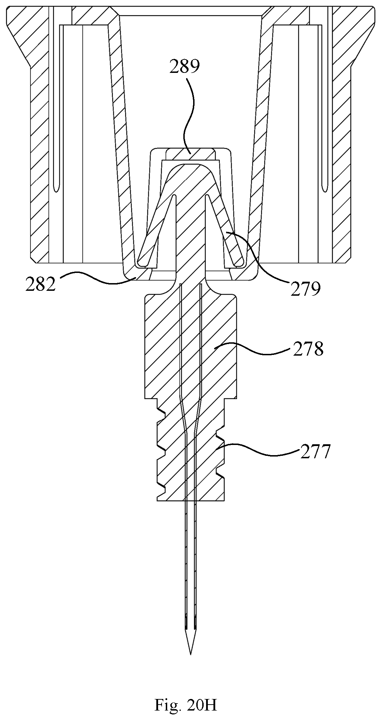

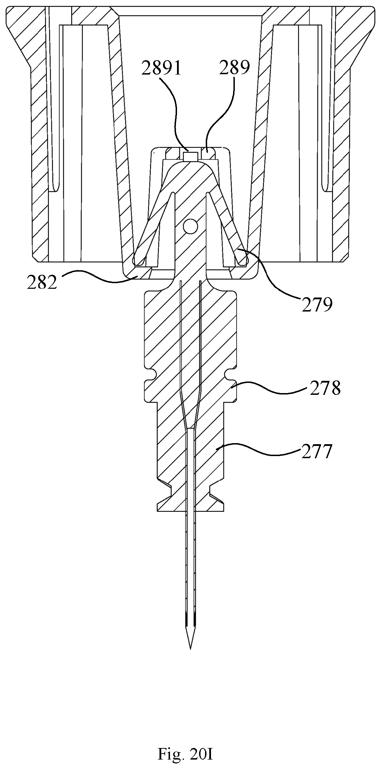

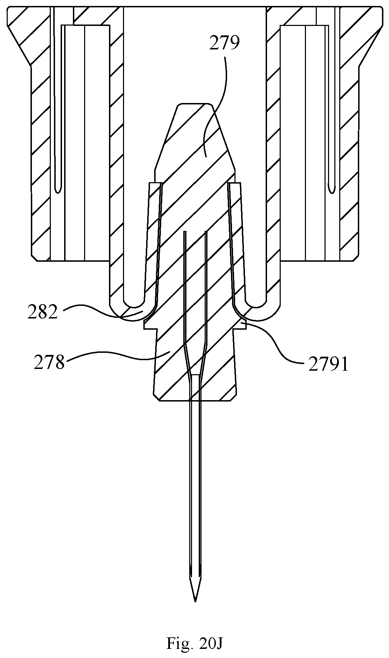

Fig. 20G is a cross-sectional view illustrating another structure of the piercing member 260 according to an example of the present disclosure. Fig. 20H is a cross-sectional view illustrating another structure of the piercing member 260 according to an example of the present disclosure. Fig. 20I is a cross-sectional view illustrating another structure of the piercing member 260 according to an example of the present disclosure. Fig. 20J is a cross-sectional view illustrating another structure of the piercing member 260 according to an example of the present disclosure. It should be noted that descriptions of some identical or similar structures refer to the above descriptions and will not be repeated here.

In some examples, referring to Figs. 20G and 20H, the position restricting portion 289 may be a semi-enclosed structure. In some examples, the position restricting portion 289 may be disposed at least partially above the third part 279. Thereby, it can help prevent the third part 279 from excessively extending into the support base 280.

In some examples, the number of first engagement structure 27411 may be one or more (see Figs. 20G and 20H). For example, the number of first engagement structure 27411 may be 1, 2, 3, or 4. In some examples, the number of second engagement structure 721 may also be one or more. For example, the number of second engagement structure 721 may be 1, 2, 3, or 4. In some examples, the number of first engagement structure 27411 and the number of second engagement structure 721 may be equal.

In some examples, referring to Fig. 20I, the position restricting portion 289 may have a limiting hole 2891. In some examples, the sharp object 270 may extend at least partially into the limiting hole 2891. In some examples, the needle portion 272 may extend at least partially into the limiting hole 2891. Thereby, wobbling of the sharp object 270 within the support base 280 can be reduced.

In some examples, the limiting hole 2891 may be aligned with the hole 2821.

In some examples, referring to Fig. 20J, the third part 279 may have a preset taper. In some examples, along the central axis CA and towards the proximal direction, the side wall of the third part 279 may gradually move away from the central axis CA. Thereby, it facilitates the assembly of the sharp object 270 to the support base 280.

In some examples, the stiffness of the third part 279 may be greater than the stiffness of the sharp object fixing portion 282. In some examples, the third part 279 may have a deformation portion. In some examples, the deformation portion may be formed by the third part 279 being recessed towards the central axis CA. In this case, during the process of assembling the sharp object 270 to the support base 280, because the third part 279 and the sharp object fixing portion 282 undergo squeezing, by forming the deformation portion, space can be provided for the deformation of the sharp object fixing portion 282 due to compression, thus facilitating the assembly of the sharp object 270 to the support base 280. In some examples, the deformation portion may be a notch formed in the third part 279.

In other examples, the stiffness of the third part 279 may also be less than or equal to the stiffness of the sharp object fixing portion 282.

In some examples, the side wall of the third part 279 may partially abut against the inner wall of the sharp object fixing portion 282. In some examples, the size of the side wall of the third part 279 may be smaller than the size of the inner wall of the sharp object fixing portion 282.

In some examples, referring to Fig. 20J, the third part 279 may have a limiting flange 2791. In some examples, the limiting flange 2791 may be configured to limit the length by which the third part 279 extends into the support base 280.

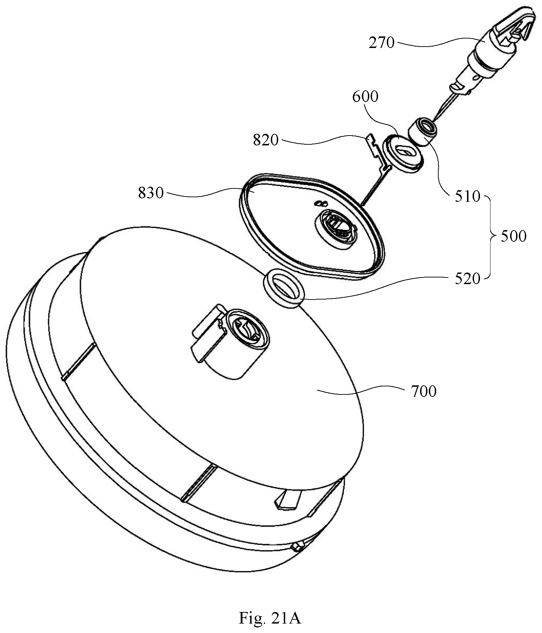

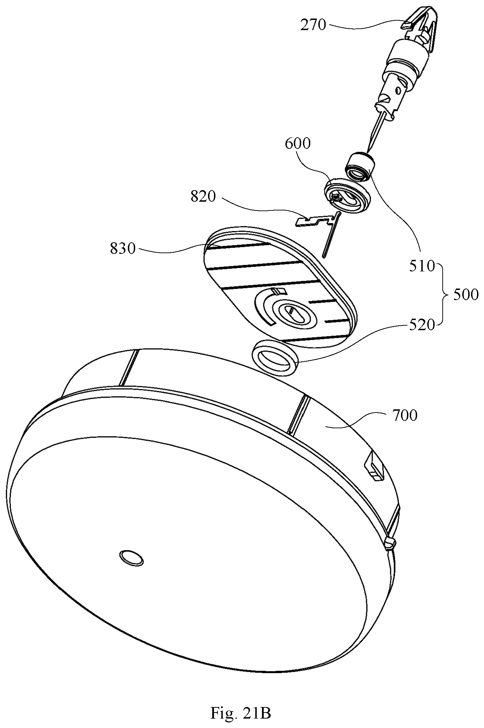

Fig. 21A is an exploded view illustrating a first perspective of a sterilization assembly according to an example of the present disclosure. Fig. 21B is an exploded view illustrating a second perspective of the sterilization assembly according to an example of the present disclosure.

As mentioned above, during the user's use of the analyte monitoring system 1, the sharp object 270 and the sensor 820 may at least partially pierce the host's subcutaneous tissue. In some examples, the sharp object 270 and the sensor 820 may be sterilized. In some examples, radiation sterilization may be used to sterilize the sensor 820. Radiation sterilization methods may include, for example, electron beam radiation, gamma radiation, X-ray radiation, or a combination thereof. Thereby, it can contribute to the health and safety of the host.

In some examples, after sterilizing the sharp object 270 and the sensor 820, the sharp object 270 and the sensor 820 may be preserved in a sealed space. In this case, prior to the user using the analyte monitoring system 1, the sharp object 270 and the sensor 820 can remain within a sterile space.

In some examples, referring to Figs. 21A and 21B, the analyte monitoring system 1 may comprise a sharp object 270, a sealing assembly 500, a sealing cover 600, a sensor 820, a base 830, and a cap 700. In some examples, the sharp object 270, sealing assembly 500, sealing cover 600, sensor 820, base 830, and cap 700 may form the sterilization assembly. In some examples, the entire sterilization assembly may be sterilized. In some examples, when sterilizing the entire sterilization assembly, components within it, such as the cap 700, should at least be made of a material that permits radiation transmission. In some examples, suitable materials for making the cap 700 may include non-magnetic metals, thermoplastics, ceramics, rubber, composite materials, or combinations thereof.

In some examples, the sterilization assembly may have a sealed space. In some examples, the sharp object 270 and the sensor 820 may be at least partially located within the sealed space of the sterilization assembly.

In some examples, the sharp object 270, sealing assembly 500, sealing cover 600, sensor 820, base 830, and cap 700 may be assembled together to form the aforementioned sealed space.

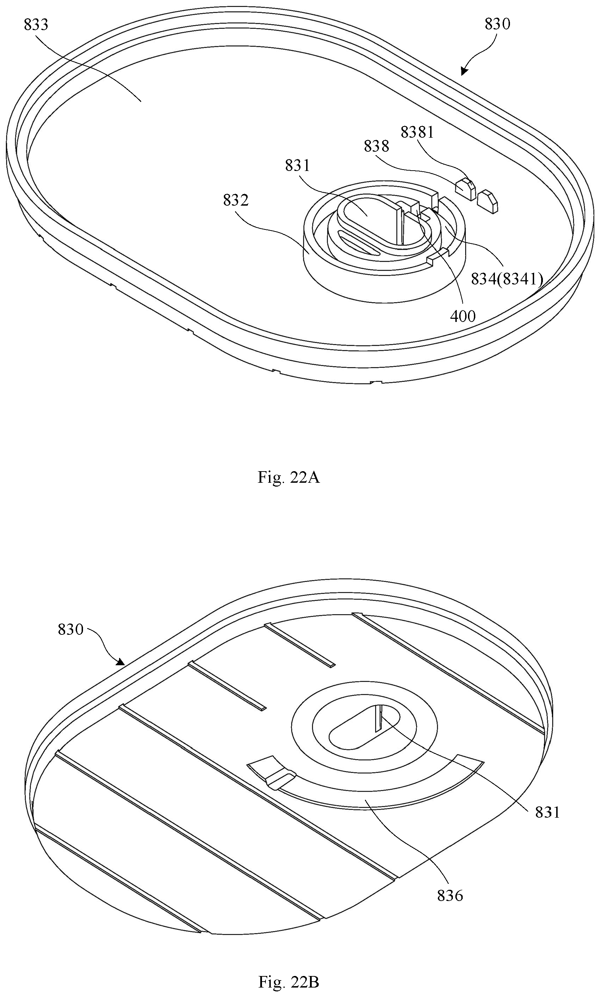

Fig. 22A is a structural schematic diagram illustrating a first perspective of a base 830 according to an example of the present disclosure. Fig. 22B is a structural schematic diagram illustrating a second perspective of the base 830 according to an example of the present disclosure.

In some examples, referring to Figs. 22A and 22B, an application portion 860 may comprise a base 830. In some examples, the contour of the base 830 may be substantially racetrack-shaped.

In some examples, the base 830 may comprise an opening portion 831. In some examples, the sensor 820 may be supported on the base 830. In some examples, a central axis of a tail portion 821 of the sensor 820 may extend through the opening portion 831.

In some examples, the contour of the opening portion 831 may be the same as the contour of a first part 277 of the sharp object 270. Thereby, wobbling of the sharp object 270 within the opening portion 831 can be reduced.

In some examples, the contour of the opening portion 831 may be substantially racetrack-shaped. In this case, because the contour of the opening portion 831 is the same as the contour of the first part 277 of the sharp object 270, undesired rotation of the sharp object 270 within the opening portion 831 can be inhibited.

In some examples, the contour of the opening portion 831 may also be polygonal, square, fan-shaped, or other non-circular shapes.

In some examples, the opening portion 831 may be a through-hole. Thereby, it facilitates the sharp object 270 and the sensor 820 passing through the opening portion 831 and extending out of the bottom surface of the base 830, thus facilitating the implantation of the sharp object 270 and the sensor 820 subcutaneously within the host.

In some examples, referring to Fig. 22A, the base 830 may comprise a support portion 832. In some examples, the support portion 832 may be a boss. In some examples, the support portion 832 may be configured to support at least a portion of the sensor 820.

In some examples, referring to Figs. 22A and 22B, the base 830 may comprise a substrate 833. In some examples, the support portion 832 may be disposed on the substrate 833. In some examples, the opening portion 831 may penetrate through the support portion 832 and the substrate 833.

In some examples, referring to Fig. 22A, the base 830 may comprise an assembly portion 834. In some examples, the assembly portion 834 may be disposed on the support portion 832.

In other examples, the assembly portion 834 may also be disposed on the substrate 833.

In some examples, referring to Fig. 22A, the base 830 may comprise a fixing portion 838. In some examples, the fixing portion 838 may be configured to fix the sensor 820. In some examples, the fixing portion 838 may be disposed on the substrate 833. In some examples, the fixing portion 838 may be a protrusion formed on the substrate 833.

In some examples, referring to Fig. 22A, there may be two fixing portions 838. In some examples, the two fixing portions 838 may be disposed opposite each other.

In other examples, the number of fixing portion 838 may be one. For example, the fixing portion 838 may be a fork structure with a groove.

In other examples, the number of fixing portion 838 may be greater than two. For example, the number of fixing portion 838 may be 4, 5, 6, or 7, etc. Thereby, the contact portion 823 can be fixed more stably.

In some examples, referring to Fig. 22A, the fixing portion 838 may have an inclined surface 8381. In some examples, the inclined surface 8381 may be inclined towards a direction away from the fixing portion 838 along the direction from a top end to a bottom end of the fixing portion 838. Thereby, it facilitates aligning a slot on the electronic device 880, which accommodates the sensor 820, with the sensor 820. (To be described in detail later)

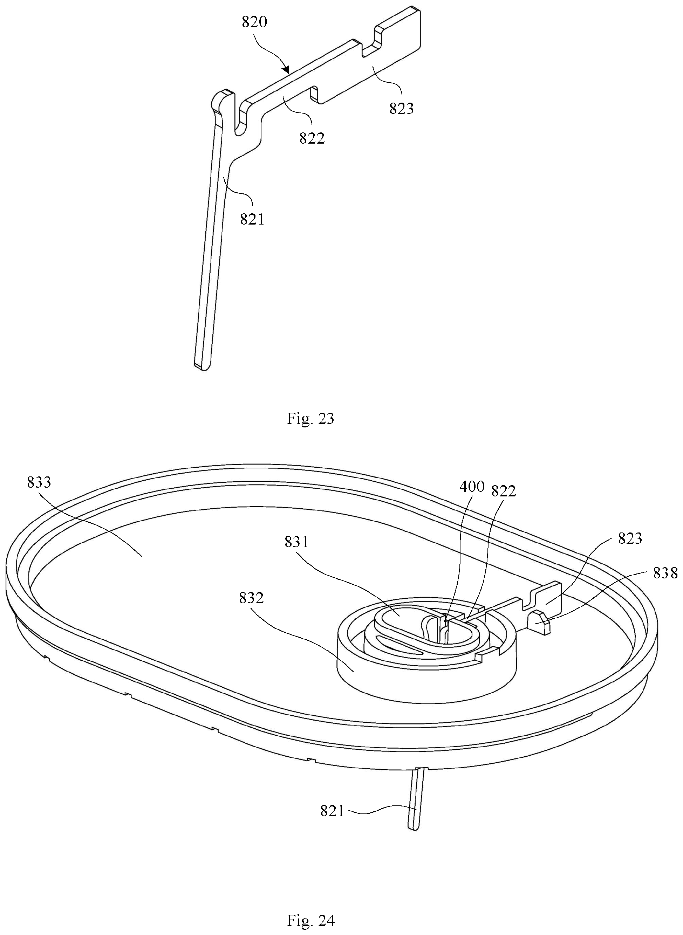

Fig. 23 is a structural schematic diagram illustrating a sensor 820 according to an example of the present disclosure.

In some examples, referring to Fig. 23, the medical device 800 may comprise a sensor 820. In some examples, the sensor 820 may be configured to generate analyte level information of the host. In some examples, the sensor 820 may be partially or entirely implanted subcutaneously within the host. In some examples, after being implanted subcutaneously, the sensor 820 may react with analytes in the subcutaneous interstitial fluid to generate the host's analyte level information.

In some examples, referring to Fig. 23, the sensor 820 may comprise a tail portion 821. In some examples, the tail portion 821 may be used for implantation into the host.

In some examples, the sensor 820 may comprise a contact portion 823 for electrical connection (see Fig. 23). In some examples, the contact portion 823 may be configured to connect to an electronic device 880. In some examples, the contact portion 823 may have electrical contacts.

In some examples, referring to Fig. 23, an angle between 0° to 90° may be formed between the contact portion 823 and the tail portion 821 of the sensor 820. Thereby, it facilitates placing the tail portion 821 into the opening portion 831 or ensuring the contact portion 823 electrically connects to the electronic device 880 when the tail portion 821 is implanted into the host.

In some examples, referring to Fig. 23, the sensor 820 may comprise a connection portion 822. In some examples, the connection portion 822 may connect the tail portion 821 and the contact portion 823. That is, the tail portion 821, connection portion 822, and contact portion 823 may be connected sequentially. Thereby, analyte level signals generated by the tail portion 821 can be transmitted to the contact portion 823 via the connection portion 822.

Fig. 24 is a schematic diagram illustrating the sensor 820 disposed on the base 830 according to an example of the present disclosure.

In some examples, referring to Fig. 24, the sensor 820 may be assembled to the base 830. Thereby, it facilitates retaining the sensor 820 subcutaneously within the host.

In some examples, referring to Fig. 24, the sensor 820 may be at least partially located within the opening portion 831. In some examples, the tail portion 821 may be at least partially located within the opening portion 831. In some examples, the tail portion 821 may extend out of the bottom surface of the base 830 via the opening portion 831. Thereby, it facilitates the sensor 820 being at least partially implanted subcutaneously within the host. Additionally, because the sharp object 270 extends through the opening portion 831, it facilitates the sharp object 270 accommodating the tail portion 821 of the sensor 820 and carrying the tail portion 821 for implantation into the host.

In some examples, the tail portion 821 may be aligned with the opening portion 831. Thereby, it further facilitates the sharp object 270 accommodating the tail portion 821.

In some examples, referring to Fig. 24, the sensor 820 may be at least partially located outside the opening portion 831. In some examples, the connection portion 822 and the contact portion 823 may also be located outside the opening portion 831.

In some examples, referring to Fig. 24, the application portion 860 may comprise a channel 400. In some examples, the channel 400 may extend towards the opening portion 831. In some examples, referring to Fig. 24, the sensor 820 may pass through the channel 400 and be located at the opening portion 831. In some examples, the connection portion 822 may extend through the channel 400.

In some examples, referring to Fig. 24, the channel 400 may be disposed on the support portion 832. In some examples, the sensor 820 may enter the opening portion 831 via the channel 400 disposed on the support portion 832.

In some examples, the channel 400 may have a fixed dimension adapted to the size of the connection portion 822. This fixed dimension may be equal to or similar to the size of the connection portion 822. Thereby, the gap between the connection portion 822 and the channel 400 can be minimized as much as possible, helping to form a seal at the channel 400.

In some examples, the sealing assembly 500 may form a seal at the channel 400 or in the direction extending from the channel 400. In some examples, the sealing assembly 500 may be disposed at the channel 400. In some examples, the sealing assembly 500 may be at least partially disposed at the channel 400.

As mentioned above, the fixing portions 838 may be disposed opposite each other. In some examples, the fixing portions 838 may be symmetrically distributed on both sides of the sensor 820. In some examples, the fixing portions 838 may clamp the contact portion 823. Thereby, wobbling of the sensor 820 during assembly can be reduced.

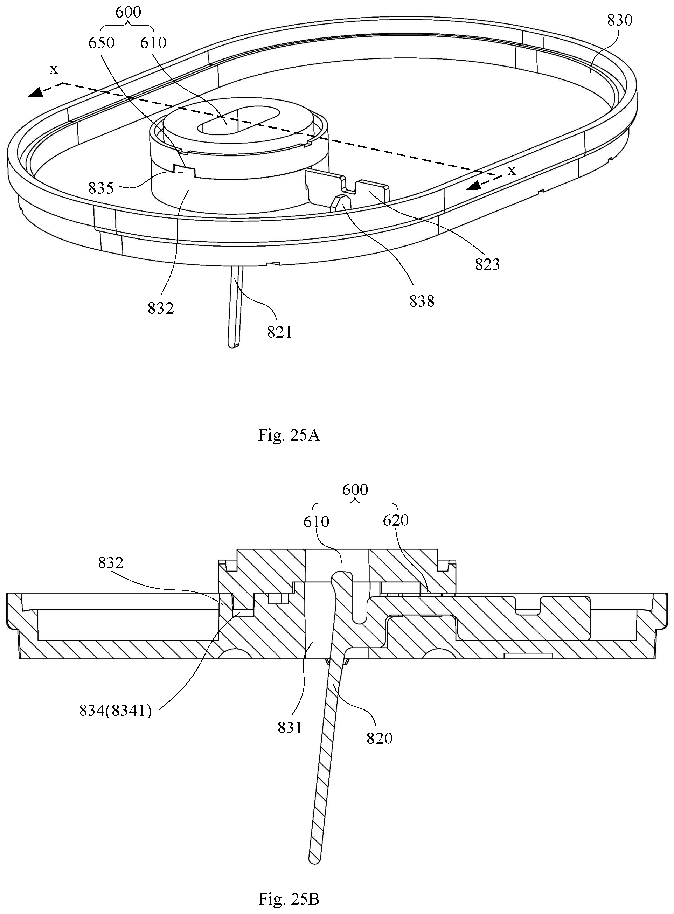

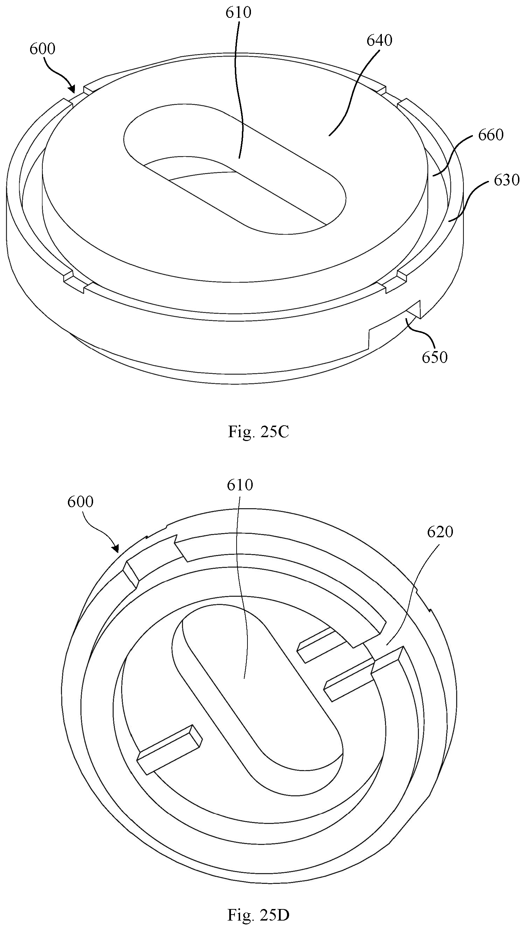

Fig. 25A is a schematic diagram illustrating the sealing cover 600 covering the support portion 832 according to an example of the present disclosure. Fig. 25B is a cross-sectional view taken along line X-X in Fig. 25A. Fig. 25C is a structural schematic diagram illustrating a first perspective of the sealing cover 600 according to an example of the present disclosure. Fig. 25D is a structural schematic diagram illustrating a second perspective of the sealing cover 600 according to an example of the present disclosure.

In some examples, referring to Fig. 25A, the application portion 860 may comprise a sealing cover 600. In some examples, the sealing cover 600 may cover the base 830 and the sensor 820. In some examples, the sealing cover 600 may cover the support portion 832.

In some examples, the sealing cover 600 may completely cover the support portion 832. In other words, in some examples, projecting along the axial direction of the support portion 832, the projection of the sealing cover 600 and the projection of the support portion 832 may completely overlap. Thereby, structural compactness is improved, facilitating subsequent sealing of the upper interface.

In some examples, the sealing cover 600 may at least partially cover the channel 400. Thereby, it helps form a sealing interface at the channel 400. Additionally, it helps retain the sensor 820 within the channel 400.

In some examples, the sealing cover 600 and the support portion 832 may be fixedly connected via one or more methods such as adhesive bonding, ultrasonic welding, etc. Thereby, the sealing performance of the sealing cover 600 can be enhanced.

In some examples, referring to Figs. 25A and 25B, the sealing cover 600 may have a central hole 610 aligned with the opening portion 831. In some examples, the central hole 610 may be configured to allow the sharp object 270 to extend through.

In some examples, referring to Figs. 25B and 25C, the contour of the central hole 610 may be the same as the contour of the opening portion 831. That is, the contour of the central hole 610 may be the same as the contour of the first part 277 of the sharp object 270. Thereby, undesired rotation of the sharp object 270 within the central hole 610 can be inhibited.

In other examples, the contour of the central hole 610 may also be different from the contour of the opening portion 831.

In some examples, referring to Fig. 25D, the sealing cover 600 may have a first notch 620. In some examples, the first notch 620 may be aligned with the channel 400. In some examples, the first notch 620 may at least partially overlap with the channel 400. Thereby, it facilitates the connection portion 822 passing through the channel 400.

Additionally, in some examples, the channel 400 may also be disposed on the sealing cover 600. In some examples, the channel 400 may extend towards the central hole 610. In some examples, the channel 400 may be disposed on a side of the sealing cover 600 close to the support portion 832.

As mentioned above, the base 830 may comprise an assembly portion 834. In some examples, referring to Fig. 22A, the assembly portion 834 may have a first pocket 8341. In some examples, referring to Fig. 22A, the first pocket 8341 may surround the opening portion 831. In some examples, the first pocket 8341 may be an annular groove.

In some examples, referring to Fig. 25B, the assembly portion 834 and the sealing cover 600 may mate in a male-female fit. In some examples, the sealing cover 600 may be at least partially received within the first pocket 8341.

In some examples, before assembling the sealing cover 600 to the assembly portion 834, an adhesive may be injected into the first pocket 8341. Thereby, it helps seal the interface between the assembly portion 834 and the sealing cover 600.

In some examples, referring to Fig. 25B, there may be a gap between the portion of the sealing cover 600 received within the first pocket 8341 and the bottom of the first pocket 8341. In this case, by leaving a certain flow space for the adhesive between the sealing cover 600 and the first pocket 8341, it helps the adhesive flow to fill gaps between components, thereby aiding in sealing the interface between the assembly portion 834 and the sealing cover 600.

In some examples, referring to Fig. 22A, the first pocket 8341 may be in communication with the channel 400. Thereby, the adhesive can seal the channel 400. In some examples, adhesive may be injected into the channel 400 to seal the channel 400.

In some examples, referring to Fig. 25A, the base 830 may have a first positioning portion 835. In some examples, the sealing cover 600 may have a second positioning portion 650. In some examples, during the process of assembling the sealing cover 600 to the assembly portion 834, the first positioning portion 835 may be aligned with the second positioning portion 650. Thereby, the sealing cover 600 can be more accurately assembled to the assembly portion 834.

In some examples, referring to Fig. 25A, the first positioning portion 835 may be disposed on the assembly portion 834, and the second positioning portion 650 may be disposed on the sealing cover 600. In some examples, the shape of the first positioning portion 835 may be complementary to the shape of the second positioning portion 650. For example, the first positioning portion 835 may be a groove, and the second positioning portion 650 may be a protrusion.

Additionally, in some examples, the first positioning portion 835 may also be disposed on the substrate 833, and the second positioning portion 650 may also be disposed on the sealing cover 600.

In some examples, when the first positioning portion 835 is aligned with the second positioning portion 650, the first notch 620 may be aligned with the connection portion 822. In this case, by aligning the first positioning portion 835 and the second positioning portion 650, the first notch 620 can be aligned with the connection portion 822, thus facilitating the assembly of the sealing cover 600.

In some examples, referring to Fig. 25A, the first positioning portion 835 may abut against the second positioning portion 650. In some examples, the first positioning portion 835 may abut against the second positioning portion 650 circumferentially around the assembly portion 834. Thereby, it can prevent the sealing cover 600 from rotating relative to the assembly portion 834, reducing the risk of damaging the sensor 820 due to rotation of the sealing cover 600.

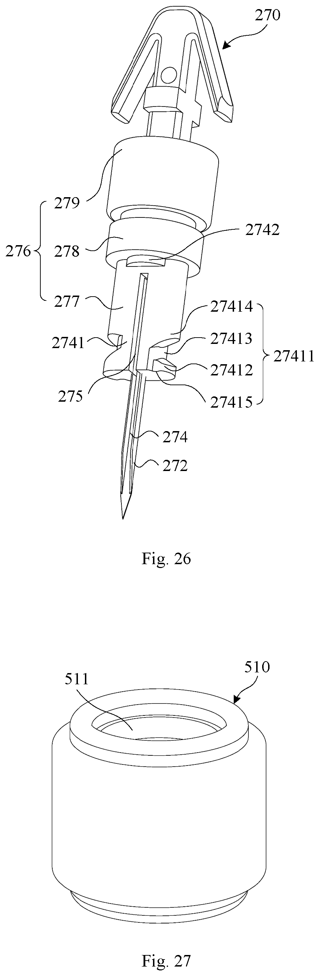

Fig. 26 is a structural schematic diagram illustrating a sharp object 270 according to an example of the present disclosure.

As mentioned above, referring to Fig. 26, the analyte monitoring system 1 may comprise a sharp object 270. In some examples, the sharp object 270 may be configured to implant the sensor 820 at least partially subcutaneously within the host.

In some examples, referring to Fig. 26, the sharp object 270 may comprise a needle portion 272. Thereby, it facilitates the sharp object 270 piercing tissue.

In some examples, referring to Fig. 26, the sharp object 270 may comprise a groove 274. In some examples, the groove 274 may be configured to accommodate the tail portion 821. In some examples, the groove 274 may be disposed on the needle portion 272. In some examples, the groove 274 may extend along the length direction of the needle portion 272.

In some examples, the opening direction of the groove 274 may face towards the tail portion 821. Thereby, it facilitates the tail portion 821 entering the groove 274.

In some examples, referring to Fig. 26, the sharp object 270 may comprise a cap portion 276. In some examples, the cap portion 276 may be at least partially located within the opening portion 831.

In some examples, referring to Fig. 26, the cap portion 276 may comprise a first part 277. In some examples, the outer contour of the first part 277 may be substantially the same as the inner contour of the opening portion 831. As mentioned earlier, the contour of the opening portion 831 may be substantially racetrack-shaped. In some examples, the contour of the first part 277 may also be substantially racetrack-shaped. Thereby, undesired rotation of the first part 277 within the opening portion 831 can be inhibited.

In some examples, the contour of the first part 277 may also be non-circular shapes such as polygonal, square, or fan-shaped.

In some examples, referring to Fig. 26, the cap portion 276 may include a second part 278. In some examples, the second part 278 may be configured to secure a first sealing element 510.

In some examples, referring to Fig. 26, the cap portion 276 may include a third part 279. In some examples, the third part 279 may be configured to make the sharp object 270 fix to the support base 280.

In some examples, referring to Fig. 26, the needle portion 272 may be connected to the cap portion 276. In some examples, the needle portion 272 may be fixed to the cap portion 276. In some examples, the cap portion 276 may clamp the needle portion 272. Thereby, the needle portion 272 may be made to carry the sensor 820 more stably to pierce the tissue.

In some examples, the needle portion 272 may penetrate the cap portion 276. In some examples, the needle portion 272 may be fixed to the first part 277 of the cap portion 276.

In some examples, the sharp object 270 may have a grooved portion 275 (refer to Fig. 26). In some examples, the grooved portion 275 may be disposed on a side of the first part 277 close to the tail portion 821. Thereby, the tail portion 821 may be conveniently guided into the grooved portion 275.

In some examples, the opening direction of the groove 274 may face the grooved portion 275. In some examples, the groove 274 may be in communication with the grooved portion 275. Thereby, the tail portion 821 may be conveniently guided through the grooved portion 275 into the groove 274.

Fig. 27 is a structural schematic diagram of a first sealing element 510 according to an example of the present disclosure.

In some examples, referring to Fig. 27, the sealing assembly 500 may include a first sealing element 510. In some examples, the first sealing element 510 may be configured to seal the opening portion 831. In some examples, the first sealing element 510 may be configured to seal the upper interface of the opening portion 831.

In some examples, referring to Fig. 21A or Fig. 21B, the first sealing element 510 may be disposed between the sharp object 270 and the sealing cover 600. In some examples, the first sealing element 510 may seal the interface between the sharp object 270 and the sealing cover 600. In some examples, the first sealing element 510 may seal the central hole 610. In some examples, the first sealing element 510 may seal the opening portion 831 by sealing the central hole 610. It should be noted that since there is already a sealed interface between the sealing cover 600 and the base 830, the upper interface of the central hole 610 may also be regarded as the upper interface of the opening portion 831. Sealing the opening portion 831 is equivalent to sealing the central hole 610.

In some examples, the first sealing element 510 may be made of an elastic material. In some examples, the first sealing element 510 may be made of materials such as silicone resin, thermoplastic elastomer (TPE), polytetrafluoroethylene, or rubber. Thereby, the sealing performance of the first sealing element 510 may be improved.

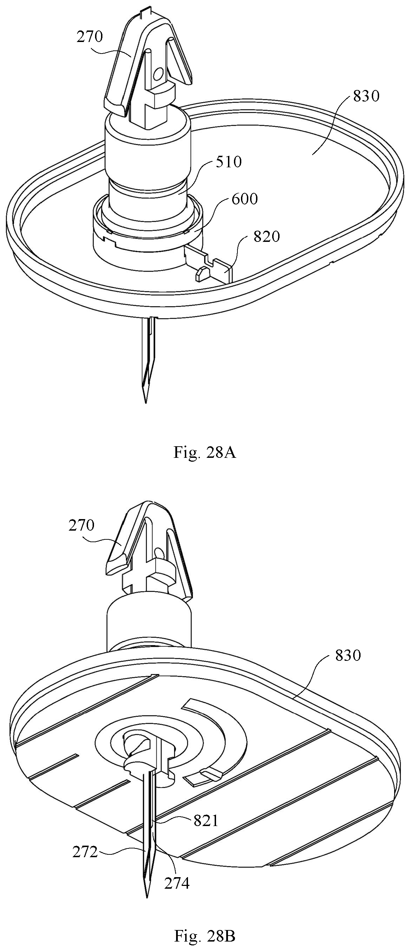

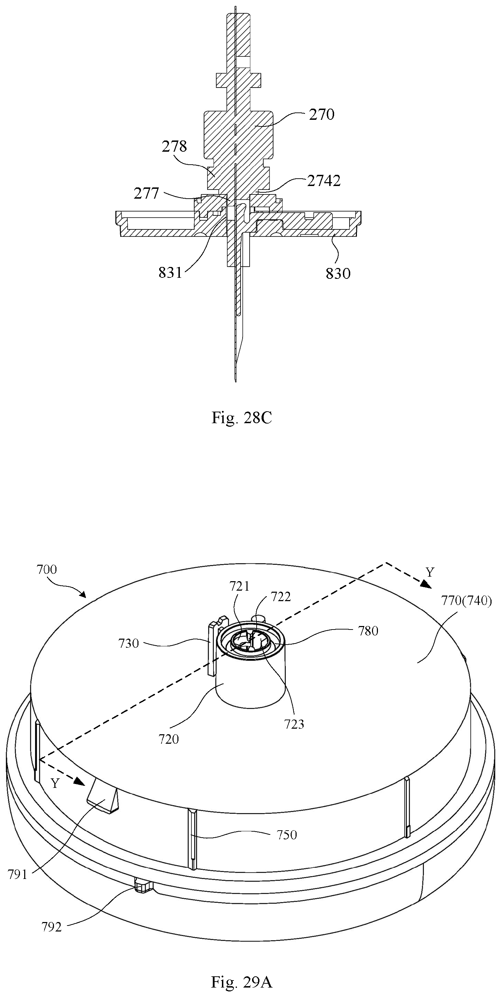

Fig. 28A is a schematic diagram of a first view of the sharp object 270 assembled to the base 830 according to an example of the present disclosure. Fig. 28B is a schematic diagram of a second view of the sharp object 270 assembled to the base 830 according to an example of the present disclosure. Fig. 28C is a schematic diagram of a depth-limiting portion 2742 abutting the sealing cover 600 according to an example of the present disclosure.

In some examples, referring to Fig. 28A and Fig. 28B, the sharp object 270 may be assembled to the base 830. In some examples, the sharp object 270 may extend through the opening portion 831. In some examples, the sharp object 270 may be at least partially located within the opening portion 831.

In some examples, referring to Fig. 28A and Fig. 28B, the first part 277 may extend at least partially out of the opening portion 831. Thereby, it may facilitate the coupling of the sharp object 270 with the cap 700.

In some examples, referring to Fig. 28A and Fig. 28B, the needle portion 272 may extend at least partially out of the opening portion 831. Thereby, it may facilitate the needle portion 272 entering the subcutaneous tissue of the host.

In some examples, referring to Fig. 28B, the sharp object 270 may be configured to accommodate at least a portion of the sensor 820. In some examples, the sharp object 270 may accommodate the tail portion 821 of the sensor 820. In some examples, the sharp object 270 may also be configured to carry at least a portion of the sensor 820 to pierce the tissue. In some examples, the sharp object 270 may carry the tail portion 821 to pierce the tissue. Thereby, it may facilitate implanting the sensor 820 into the host.

As described above, the groove 274 may be configured to accommodate the tail portion 821. In some examples, the needle portion 272 provided with the groove 274 may also be configured to carry the tail portion 821 to pierce the tissue. In this case, by accommodating the tail portion 821 in the groove 274, the needle portion 272 may conveniently carry the tail portion 821 to pierce the tissue.

In some examples, referring to Fig. 28A, the first sealing element 510 may be sleeved over the cap portion 276. In some examples, both ends of the first sealing element 510 may be embedded in the cap portion 276. In this case, when the sharp object 270 moves, the risk of the first sealing element 510 falling off from the cap portion 276 may be reduced.

As described above, the cap portion 276 may include a second part 278. In some examples, the first sealing element 510 may be sleeved over the second part 278. In some examples, the first sealing element 510 may have a first hole 511 (refer to Fig. 26). In some examples, the sharp object 270 may pass through the first hole 511 so that the first sealing element 510 is sleeved over the second part 278.

In some examples, the first sealing element 510 may be sleeved over the second part 278 in an enveloping manner. In some examples, the outer contour of the second part 278 may be the same as the inner contour of the first sealing element 510. In some examples, the second part 278 may be an annular protrusion on the cap portion 276, and the first sealing element 510 may be a collar with a C-shaped vertical cross-section (refer to Fig. 26 and Fig. 27).

In some examples, the size of the first sealing element 510 may be slightly smaller than the size of the second part 278. Thereby, the first sealing element 510 may be more tightly sleeved over the second part 278.

In some examples, referring to Fig. 28A, one side of the first sealing element 510 may engage with the sharp object 270, and the other side of the first sealing element 510 may engage with the sealing cover 600.

In some examples, referring to Fig. 28A, the first sealing element 510 may surround the central hole 610. Thereby, it may facilitate the first sealing element 510 sealing the central hole 610. In some examples, the first sealing element 510 may be annular.

In some examples, the first sealing element 510 may be a sealing washer or an O-ring.

In some examples, referring to Fig. 28A, the sharp object 270 may enter the opening portion 831 via the first hole 511. In some examples, the first hole 511 may be aligned with the opening portion 831. Thereby, it may facilitate the sharp object 270 passing through the first hole 511 and entering the opening portion 831.

In some examples, the sharp object 270 may apply pressure to the first sealing element 510. In some examples, the sharp object 270 may compress the first sealing element 510 against the sealing cover 600 to deform the first sealing element 510. In some examples, the first sealing element 510 may deform under compression to seal the interface between the sharp object 270 and the sealing cover 600. In some examples, the first sealing element 510 surrounding the opening portion 831 may deform under compression to seal the upper interface of the opening portion 831. Thereby, the sealing performance of the first sealing element 510 may be improved.

In some examples, referring to Fig. 26 and Fig. 28C, the cap portion 276 may include a depth-limiting portion 2742. In some examples, the depth-limiting portion 2742 may be configured to limit the depth to which the first part 277 is embedded in the opening portion 831. In some examples, the depth-limiting portion 2742 may be disposed on the first part 277. In some examples, the depth-limiting portion 2742 may protrude outward from the first part 277.

In other examples, the depth-limiting portion 2742 may also be disposed on the bottom surface of the second part 278 and protrude outward from the second part 278.

In some examples, referring to Fig. 28C, when the first part 277 passes through the opening portion 831 and protrudes from the base 830, the depth-limiting portion 2742 may abut the sealing cover 600. Thereby, it may help to assemble the sharp object 270 more stably to the base 830.

In other examples, the depth-limiting portion 2742 may also abut the support base 832.

It should be noted that when the sharp object 270 is assembled to the base 830, since the connection between the sharp object 270 and the sealing cover 600 is through the first sealing element 510, i.e., the connection between the sharp object 270 and the sealing cover 600 is an elastic connection, and the sharp object 270 applies pressure to the first sealing element 510, if the pressure applied to the first sealing element 510 is uneven, different parts of the first sealing element 510 will have varying degrees of deformation, making it unable to provide stable support for the sharp object 270, possibly causing the sharp object 270 to wobble. Therefore, by having the depth-limiting portion 2742 abut the sealing cover 600, rigid support may be provided for the sharp object 270, reducing its wobble.

In some examples, the thickness of the first sealing element 510 before compression may be greater than the thickness of the depth-limiting portion 2742. Thereby, it may help to fully compress the first sealing element 510 to perform its sealing function.

In some examples, the depth-limiting portion 2742 may be integrally formed with the first part 277 or the second part 278. Additionally, in some examples, the depth-limiting portion 2742 may also be detachably connected to the first part 277 or the second part 278.

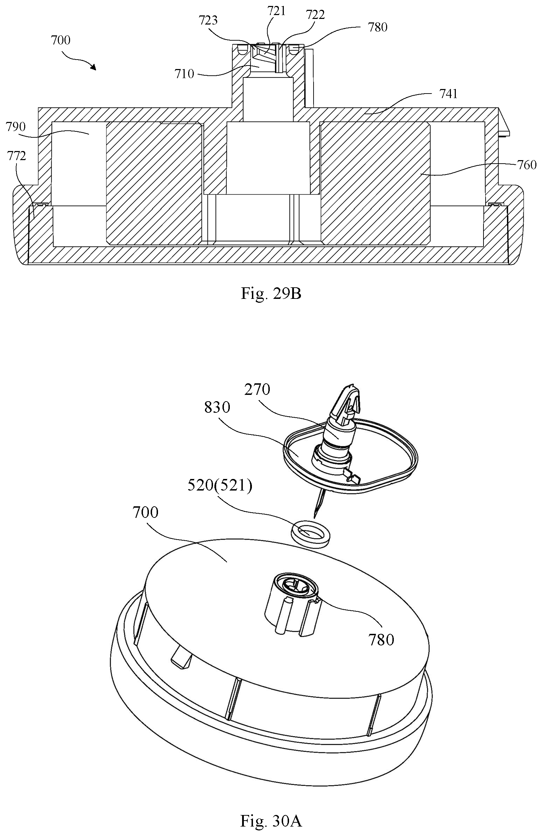

Fig. 29A is a structural schematic diagram of the cap 700 according to an example of the present disclosure. Fig. 29B is a cross-sectional schematic diagram of the cap 700 according to an example of the present disclosure.

As described above, referring to Fig. 29A, the analyte monitoring system 1 may include the cap 700. In some examples, the cap 700 may be configured to accommodate the tail portion 821. In some examples, the cap 700 may also be configured to seal the opening portion 831. In some examples, the cap 700 may be assembled to the base 830.

In some examples, referring to Fig. 29A, the cap 700 may include an engagement pedestal 740. In some examples, the engagement pedestal 740 may be part of a bottom cover 770. In some examples, the cap 700 may be coupled to the housing 10 via the engagement pedestal 740. In some examples, the engagement pedestal 740 may be embedded in the proximal end portion of the housing 10.

In some examples, when the cap 700 is coupled to the housing 10, the first engagement feature 191 may surround the engagement pedestal 740.

In some examples, the engagement pedestal 740 may be elastic. In some examples, the engagement pedestal 740 may include an elastic wall 741. In some examples, a mating portion 720 may be disposed on the wall 741. In some examples, the elasticity of the wall 741 may be adjusted by changing its thickness. For example, the elasticity of the wall 741 may be increased by reducing its thickness. In this case, since the first engagement feature 191 is a straight thread and the engaging portion 2741 is a tapered thread, when screwing the cap 700, in the direction of the central axis CA, the travel distance of the mating portion 720 is different from that of the engagement pedestal 740. This causes deformation at the junction between the mating portion 720 and the engagement pedestal 740. By adjusting the elasticity of the wall 741 by changing its thickness, the junction between the mating portion 720 and the engagement pedestal 740 may be made more easily deformable, thereby facilitating decoupling the mating portion 720 from the engaging portion 2741.

In some examples, referring to Fig. 29A, the cap 700 may include a second rib 750. In some examples, the second rib 750 may be arranged on the side surface of the engagement pedestal 740. In this case, the second rib 750, by making the contact between the housing 10 and the engagement pedestal 740 a line contact, may reduce the force required for relative screwing between the housing 10 and the cap 700, thereby facilitating decoupling the housing 10 and the cap 700. In some examples, the second rib 750 may surround the engagement pedestal 740. In some examples, there may be multiple second ribs 750.

In some examples, referring to Fig. 29B, the cap 700 may have a chamber 710. In some examples, the chamber 710 may be configured to accommodate the tail portion 821. In some examples, the tail portion 821, the cap portion 276, and the needle portion 272 may be at least partially located within the chamber 710.

In some examples, referring to Fig. 29B, the cap 700 may include a desiccant 760. In some examples, the desiccant 760 may be located in the chamber 710. Thereby, it may help maintain dryness inside the chamber 710.

In some examples, the desiccant 760 may be accommodated in a chamber 790 of the bottom cover 770 (refer to Fig. 29B). Specifically, the cap 700 may also include a bottom plate 772 engaged with the engagement pedestal 740. The engagement pedestal 740 and the bottom plate 772 may cooperate to form the chamber 790 (refer to Fig. 29B). In some examples, the chamber 710 and the chamber 790 may be in communication.

In some examples, the bottom plate 772 may be connected to the engagement pedestal 740. In some examples, the bottom plate 772 and the engagement pedestal 740 may be fixedly connected by one or more methods such as adhesive bonding or ultrasonic welding. Thereby, it may help seal the chamber 710.

In some examples, the bottom plate 772 may be made of a transparent material. In this case, after the cap 700 is assembled to the base 830, it is convenient to confirm whether the sensor 820 is accommodated in the groove 274 through the bottom plate 772.

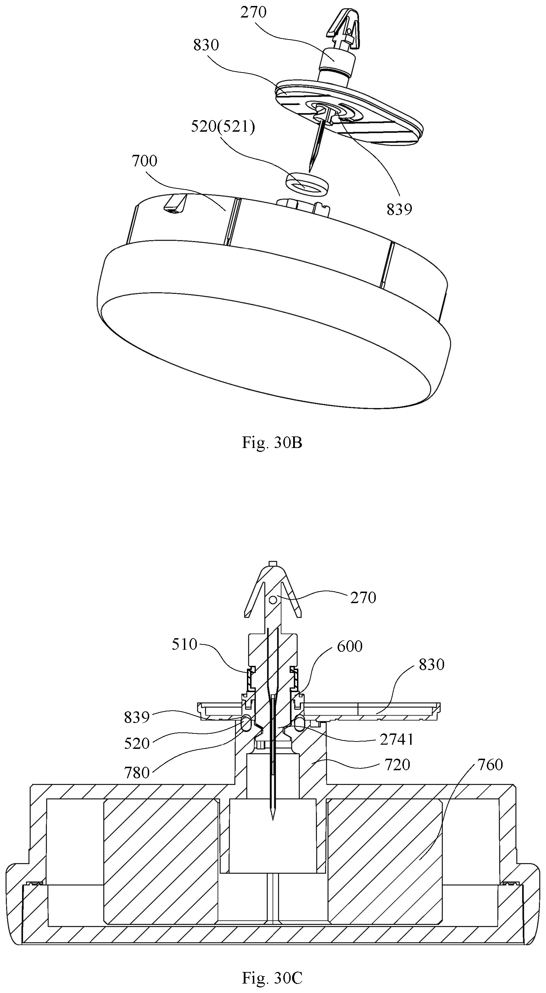

Fig. 30A is a schematic diagram of a first view of the cap 700 assembled to the base 830 according to an example of the present disclosure. Fig. 30B is a schematic diagram of a second view of the cap 700 assembled to the base 830 according to an example of the present disclosure. Fig. 30C is a cross-sectional view of the sterilization assembly according to an example of the present disclosure.

In some examples, the cap 700 may be assembled to the base 830. In some examples, when the cap 700 is assembled to the base 830, the cap 700 may be coupled to the sharp object 270. In some examples, the cap 700 may apply a pulling force to the sharp object 270. In some examples, the cap 700 may apply a pulling force to the sharp object 270 by coupling with the sharp object 270. In other words, the sharp object 270 may also apply a pulling force to the cap 700 by coupling with the cap 700. In this case, by the sharp object 270 applying a pulling force to the cap 700, the cap 700 may fit more tightly against the bottom surface of the base 830, thereby helping to seal the interface between the cap 700 and the base 830. Additionally, through the interaction force between the cap 700 and the sharp object 270, it may help maintain the assembly relationship between the cap 700 and the base 830.

In some examples, the coupling between the cap 700 and the sharp object 270 may be a threaded coupling. Thereby, the coupling between the cap 700 and the sharp object 270 may be more stable.

As described above, referring to Fig. 30B, the sharp object 270 may extend through the opening portion 831. In some examples, referring to Fig. 30C, a side of the sharp object 270 protruding from the opening portion 831 may be coupled to the cap 700. In some examples, the cap portion 276 may protrude from the opening portion 831 and couple to the cap 700.

As described above, the cap 700 may be removably coupled to the housing 10. In some examples, referring to Fig. 30C, the cap 700 may accommodate the sharp object 270 and the tail portion 821. In some examples, the size of the bottom cover 770 may be larger than the size of the mating portion 720. For example, the diameter of the bottom cover 770 may be larger than the diameter of the mating portion 720. In some examples, the size of the engagement pedestal 740 may be slightly smaller than the size of the proximal end portion of the housing 10, and the size of the bottom cover 770 may be equal to the size of the proximal end portion of the housing 10. In this case, by the cap 700 covering the bottom of the housing 10 to provide a sealed space for the sharp object 270 and the tail portion 821, a larger accommodation space may be conveniently formed, thereby accommodating more desiccant 760 and helping to maintain the dryness of the sealed space.

In some examples, referring to Figs. 30A and 30B, the sealing assembly 500 may include a second sealing element 520. In some examples, the second sealing element 520 may be configured to seal an opening portion 831. In some examples, the second sealing element 520 may be configured to seal the lower interface of the opening portion 831. In some examples, the interface between the cap 700 and the base 830 may be the same as the lower interface of the opening portion 831.

In some examples, referring to Figs. 30A and 30B, the second sealing element 520 may be disposed between the cap 700 and the base 830. Thereby, it helps to seal the interface between the cap 700 and the base 830.

In some examples, the second sealing element 520 may surround the opening portion 831. Thereby, it facilitates the sealing of the opening portion 831 by the second sealing element 520. In some examples, the second sealing element 520 may be annular.

In some examples, referring to Figs. 30A and 30B, the second sealing element 520 may be a gasket or an O-ring.

In some examples, the second sealing element 520 may be made of an elastic material. In some examples, the second sealing element 520 may be made of materials such as silicone, Thermoplastic Elastomer (TPE), polytetrafluoroethylene, or rubber. Thereby, the sealing performance of the second sealing element 520 can be improved.

In some examples, the second sealing element 520 may be sleeved on the cap 700. In some examples, the second sealing element 520 may be sleeved on the end of the cap 700 near the base 830.

In some examples, referring to Fig. 30C, the side of the cap 700 near the base 830 may have a first sealing groove 780. In some examples, the second sealing element 520 may be at least partially embedded in the first sealing groove 780.

In some examples, referring to Fig. 30B, the side of the base 830 near the cap 700 may have a second sealing groove 839. In some examples, the second sealing element 520 may be at least partially embedded in the second sealing groove 839.

In some examples, the second sealing element 520 may first be assembled into the first sealing groove 780 of the cap 700, and then the cap 700 may be assembled onto the base 830. In other examples, the second sealing element 520 may first be assembled into the second sealing groove 839 of the base 830, and then the cap 700 may be assembled onto the base 830.

In some examples, the depth of the first sealing groove 780 may be greater than the depth of the second sealing groove 839. In this case, when assembling the cap 700 onto the base 830 from below the base 830, it helps prevent the second sealing element 520 from falling off during assembly.

In some examples, the shape of the second sealing element 520 may be identical or similar to the contour of the first sealing groove 780 and the second sealing groove 839. In this case, by making the first sealing groove 780 and the second sealing groove 839 have a shape closer to the shape of the second sealing element 520, the adhesion between the second sealing element 520 and the first sealing groove 780 and the second sealing groove 839 can be improved, thereby enhancing the sealing performance of the second sealing element 520.

In some examples, the size of the first sealing groove 780 and the second sealing groove 839 may be slightly smaller than the size of the second sealing element 520. Thereby, the second sealing element 520 can be compressed more fully, improving its sealing performance.

In some examples, referring to Figs. 30A and 30B, the second sealing element 520 may have a second hole 521. In some examples, the needle portion 272 and the tail portion 821 may at least partially pass through the opening portion 831 and enter the second hole 521.

In some examples, the second hole 521 may be aligned with the opening portion 831. Thereby, it facilitates the passage of the needle portion 272 and the tail portion 821 through the second hole 521.

In some examples, referring to Fig. 30, one side of the second sealing element 520 may engage with the cap 700, and the other side of the second sealing element 520 may engage with the base 830.

In some examples, the cap 700 may apply pressure to the second sealing element 520. In some examples, the cap 700 may press the second sealing element 520 tightly against the base 830 to cause deformation of the second sealing element 520. In some examples, the second sealing element 520 may deform under pressure to seal the interface between the cap 700 and the base 830. In some examples, the second sealing element 520 surrounding the opening portion 831 may deform under pressure to seal the lower interface of the opening portion 831. Thereby, the sealing performance of the second sealing element 520 can be improved.

As described above, the sharp object 270 may apply a pulling force to the cap 700 through coupling with the cap 700. In some examples, the pressure applied by the cap 700 to the second sealing element 520 may be the pulling force applied by the sharp object 270 to the cap 700.

As described above, the cap portion 276 may be coupled to the cap 700. In some examples, referring to Figs. 26 and 30C, the cap portion 276 may have an engaging portion 2741. In some examples, the engaging portion 2741 may be located at the end of the cap portion 276 near the cap 700. In some examples, the engaging portion 2741 may be located on the part of the cap portion 276 protruding through the bottom surface of the base 830. Thereby, it facilitates the coupling of the engaging portion 2741 with the cap 700.

In some examples, referring to Figs. 29A and 30C, the cap 700 may have a mating portion 720. In some examples, the mating portion 720 may be located at the end of the cap 700 near the base 830. In some examples, the mating portion 720 may be disposed on the engagement pedestal 740. In some examples, the mating portion 720 and the engagement pedestal 740 may be integrally formed.

In some examples, the engaging portion 2741 may be coupled to the mating portion 720. In some examples, the engaging portion 2741 may be coupled to the mating portion 720 by relatively screwing them together.

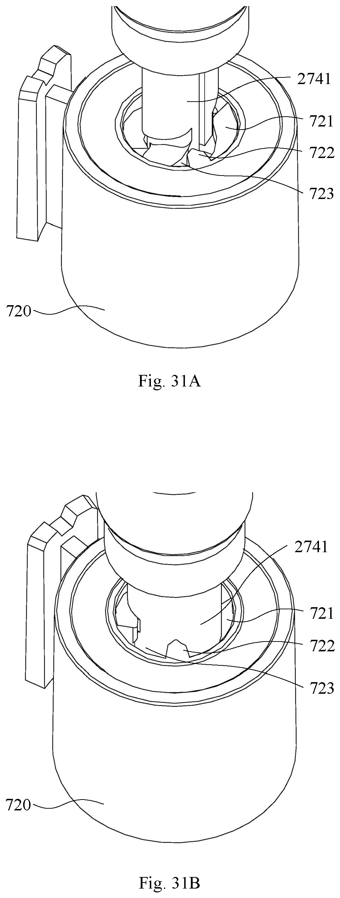

Fig. 31A is a schematic diagram showing the cap 700 and the sharp object 270 before coupling according to an example of the present disclosure. Fig. 31B is a schematic diagram showing the cap 700 and the sharp object 270 after coupling according to an example of the present disclosure.

In some examples, referring to Fig. 26 and Fig. 31A, the engaging portion 2741 may have a first engagement structure 27411. In some examples, the mating portion 720 may have a second engagement structure 721. In some examples, the first engagement structure 27411 may be a groove structure. In some examples, the second engagement structure 721 may be an arc-shaped protrusion structure. In some examples, during the relative screwing process of the engaging portion 2741 and the mating portion 720, the first engagement structure 27411 may rotate relative to the second engagement structure 721 under the constraint of the second engagement structure 721, and the second engagement structure 721 may also rotate relative to the first engagement structure 27411 under the constraint of the first engagement structure 27411.

In some examples, referring to Figs. 31A and 31B, the coupling between the engaging portion 2741 and the mating portion 720 may be a threaded coupling. In some examples, the first engagement structure 27411 may be a first thread. In some examples, the second engagement structure 721 may be a second thread. In some examples, the second thread may have a preset angle. In some examples, the preset angle may be 90 degrees. In some examples, by coupling the first thread with the second thread, the engaging portion 2741 can be guided to rotate 90 degrees relative to the mating portion 720 to couple with it.

In some examples, referring to Fig. 30C, the width of the first thread may be equal to or similar to the width of the second thread. Thereby, the tightness of the coupling between the first engagement structure 27411 and the second engagement structure 721 can be improved.

In some examples, the number of turns of the first thread and the second thread can be determined based on comprehensive consideration of the tightness of the connection between the sharp object 270 and the cap 700 and the thickness of the base 830, and set accordingly.

In some examples, the first thread and the second thread may be tapered threads. Thereby, the tightness of the coupling between the engaging portion 2741 and the mating portion 720 can be improved.

In other examples, the first thread and the second thread may also be parallel threads.

As described above, the wall 741 may be elastic. For example, when the first and second threads are tapered threads, the elasticity of the movable wall 741 may be greater; when the first and second threads are parallel threads, the elasticity of the wall 741 may be smaller.

In some examples, referring to Fig. 26, the first engagement structure 27411 may include a first engagement end 27412, a second engagement end 27414, and an engagement groove 27413. In some examples, the first engagement end 27412 and the second engagement end 27414 may be located on both sides of the engagement groove 27413, respectively.

In some examples, referring to Fig. 30C, the size of the engagement groove 27413 may match the size of the second engagement structure 721. In some examples, in the vertical direction, the width of the engagement groove 27413 and the width of the second engagement structure 721 may be equal or similar. In some examples, the width of the engagement groove 27413 may be the distance between the first engagement end 27412 and the second engagement end 27414.

In some examples, when the first engagement structure 27411 is the first thread, the distance between the first engagement end 27412 and the second engagement end 27414 may be the width of the first thread.

In some examples, referring to Fig. 31A, the mating portion 720 may have an entry guide groove 723. In some examples, the entry guide groove 723 may be configured to guide the engaging portion 2741 into the mating portion 720 along its axial direction. In some examples, the first engagement end 27412, the engagement groove 27413, and the second engagement end 27414 of the engaging portion 2741 may sequentially enter the entry guide groove 723.

In some examples, the entry guide groove 723 may extend along the axial direction of the mating portion 720. In some examples, the axial direction of the mating portion 720 may be the vertical direction. Thereby, it facilitates the coupling of the engaging portion 2741 to the mating portion 720.

In some examples, referring to Fig. 26, the first engagement end 27412 may have an engagement notch 27415. In some examples, the contour of the engagement notch 27415 may be the same as the contour of the end of the second engagement structure 721 near the entry guide groove 723. In some examples, during the process of the first engagement end 27412 entering the entry guide groove 723, the end of the second engagement structure 721 near the entry guide groove 723 may pass through the engagement notch 27415, enter the engagement groove 27413, and abut against the second engagement end 27414.

In some examples, referring to Figs. 31A and 31B, when the second engagement structure 721 abuts against the second engagement end 27414, relative screwing of the engaging portion 2741 and the mating portion 720 may be allowed.

In some examples, when the second engagement structure 721 abuts against the second engagement end 27414, the engagement groove 27413 may be aligned with the second engagement structure 721. Thereby, it facilitates the relative screwing of the engaging portion 2741 and the mating portion 720.

In some examples, referring to Fig. 31B, after relatively screwing the engaging portion 2741 and the mating portion 720, the first engagement structure 27411 may be coupled to the second engagement structure 721.

In some examples, referring to Fig. 31B, the mating portion 720 may have a first rib 722. In some examples, the first rib 722 may be disposed at the end point of the second engagement structure 721 (i.e., the end of the second engagement structure 721 away from the entry guide groove 723). In some examples, the first rib 722 may be configured to prevent further relative screwing of the engaging portion 2741 and the mating portion 720 after they have been relatively screwed by the preset angle. In some examples, after the engaging portion 2741 and the mating portion 720 have been relatively screwed by the preset angle, the first rib 722 may abut against the first engagement end 27412 and the second engagement end 27414 to prevent further relative screwing. In some examples, the first rib 722 may extend along the axial direction of the mating portion 720.

In some examples, the angle between the first rib 722 and the entry guide groove 723 in the circumferential direction of the mating portion 720 may be equal to the preset angle.

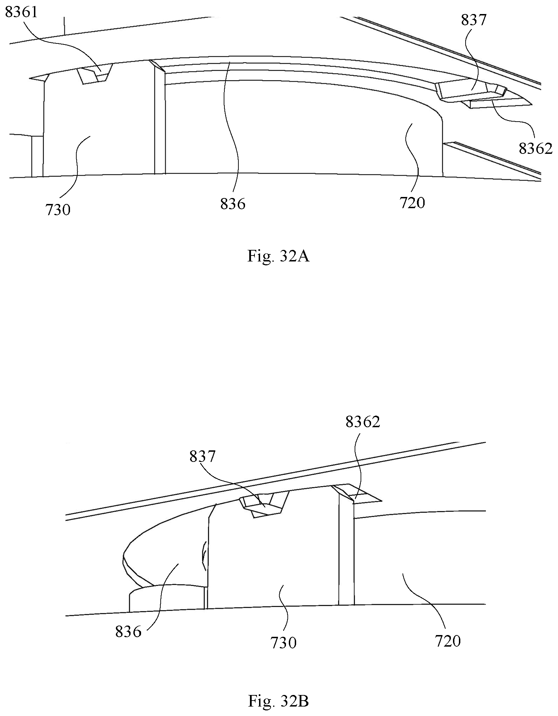

Fig. 32A is a schematic diagram showing the cap 700 and the base 830 before coupling according to an example of the present disclosure. Fig. 32B is a schematic diagram showing the cap 700 and the base 830 after coupling according to an example of the present disclosure.

In some examples, referring to Figs. 22B and 32A, the base 830 may include a guide portion 836. In some examples, referring to Figs. 32A and 32B, the cap 700 may include a guided portion 730. In some examples, the guide portion 836 may be a guide rail. In some examples, when the cap 700 rotates, the guided portion 730 may move along the guide portion 836. Thereby, it enables the cap 700 to rotate along the desired motion path.

In some examples, referring to Fig. 29A, the guided portion 730 may be disposed on the engagement pedestal 740. In some examples, the guided portion 730 may be connected to the mating portion 720.

In some examples, referring to Figs. 32A and 32B, the guided portion 730 may cooperate with the guide portion 836. In some examples, the guide portion 836 may guide the guided portion 730. In some examples, during screwing, the guided portion 730 may move along the guide portion 836.

In some examples, referring to Fig. 22B, the guide portion 836 may be formed by recessing inward from the bottom surface of the base 830. In some examples, the guide portion 836 may be a guide groove formed by inward recessing of the bottom surface of the base 830.

In some examples, referring to Fig. 22B, the guide portion 836 may be arc-shaped. In some examples, the guide portion 836 may have a predetermined arc. In some examples, the angle corresponding to the predetermined arc may match the preset angle. For example, the angle corresponding to the predetermined arc may be equal to the preset angle. It should be noted that since the guided portion 730 has a certain thickness in the guiding direction, the angle corresponding to the predetermined arc may also be slightly larger than the preset angle.

In some examples, the guide portion 836 may have a predetermined radius. In some examples, the predetermined radius may match the radius corresponding to a first engagement feature 191. Thereby, it enables the cap 700 to be decoupled from the applicator device 1000 while simultaneously releasing the coupling between the cap 700 and the cap portion 276.

In some examples, referring to Figs. 32A and 32B, the base 830 may have a third limiting portion 837. In some examples, the third limiting portion 837 may be disposed on the guide portion 836 or on an extension line of the guide portion 836.

In some examples, the third limiting portion 837 may cooperate with the guided portion 730. Thereby, it can limit the rotational travel of the cap 700. In some examples, the third limiting portion 837 may fixedly engage with the guided portion 730. Thereby, it can restrict the position of the movement endpoint of the guided portion 730.

In some examples, the third limiting portion 837 may be a protrusion. In some examples, the guided portion may have a groove. In some examples, the groove of the guided portion 730 may couple with the third limiting portion 837 configured as a protrusion.

In some examples, referring to Fig. 32A, the guide portion 836 may have a starting position and an ending position. In some examples, during the coupling process of the engaging portion 2741 and the mating portion 720, the starting position of the guided portion 730 may be the starting position of the guide portion 836, and the ending position of the guided portion 730 may be the ending position of the guide portion 836.

In some examples, the third limiting portion 837 may be disposed at an end of a guide portion 836 close to an ending position. Thereby, the guided portion 730 can be restricted at the ending position.

In some examples, referring to Fig. 32A, both ends of the third limiting portion 837 in the guiding direction of the guide portion 836 may have engagement inclined surfaces. Thereby, the coupling between the third limiting portion 837 and a groove of the guided portion 730 can be facilitated.

In some examples, by relatively twisting the engaging portion 2741 and the mating portion 720, the relative position between the engaging portion 2741 and the mating portion 720 can be changed from that in Fig. 32A to that in Fig. 32B.

In some examples, the sensor 820 may be assembled to the assembly portion 834, an adhesive may be injected into the assembly portion 834, and a sealing cover 600 may be mounted on the assembly portion to seal the channel 400. In some examples, the first sealing element 510 may be assembled to the sharp object 270, and the sharp object 270 may be assembled to the sealing cover 600 to seal the upper interface of the opening portion 831. In some examples, the second sealing element 520 may be assembled to the cap 700, and the cap 700 may be assembled to a base 830 to seal the lower interface of the opening portion 831. Thereby, a sealed space for sealing the sharp object 270 and the sensor 820 can be formed.

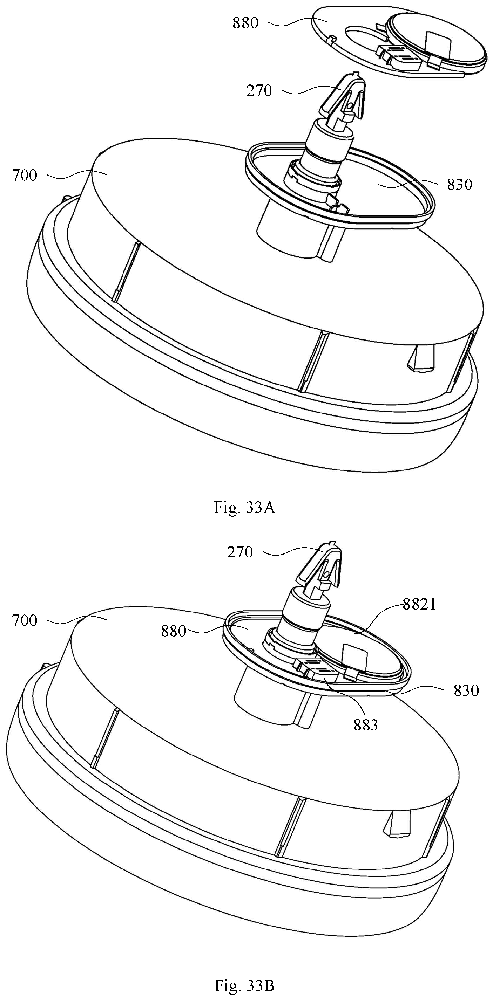

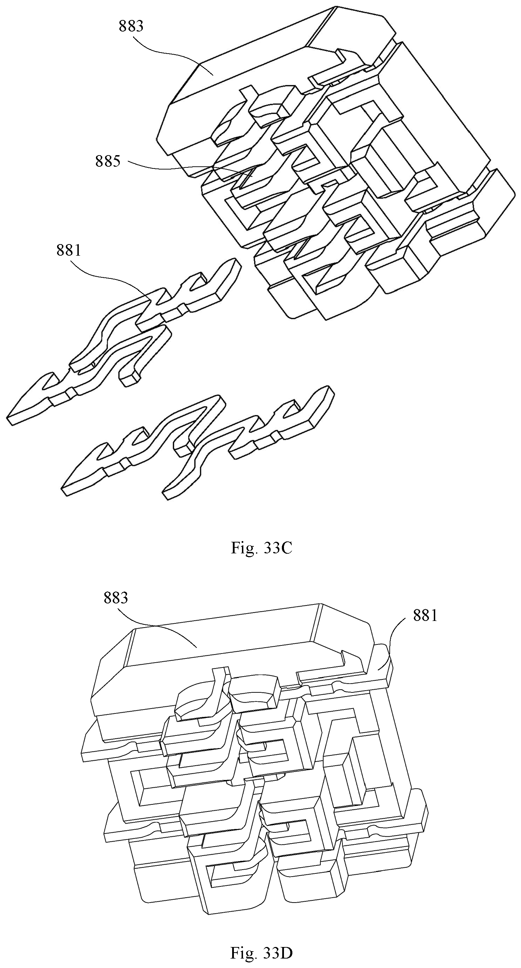

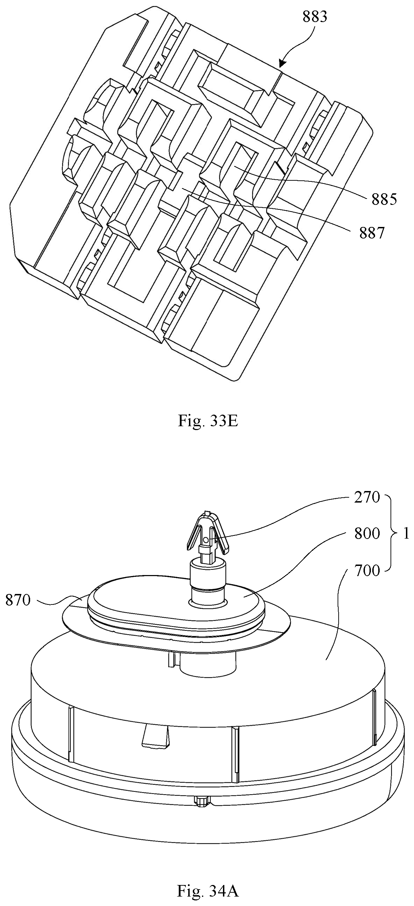

Fig. 33A is a schematic diagram showing an electronic device 880 and the base 830 before assembly according to an example of the present disclosure. Fig. 33B is a schematic diagram showing the electronic device 880 and the base 830 after assembly according to an example of the present disclosure. Fig. 33C is a schematic diagram showing a spring plate 881 and a connector 883 before assembly according to an example of the present disclosure. Fig. 33D is a schematic diagram showing the spring plate 881 and the connector 883 after assembly according to an example of the present disclosure. Fig. 33E is a schematic diagram showing a structure of the connector 883 according to an example of the present disclosure.

In some examples, the electronic device 880 may be assembled to the base 830. In some examples, after being assembled to the base 830, the electronic device 880 may connect to the sensor 820.

In some examples, referring to Figs. 33A and 33B, the electronic device 880 may have a space for the sharp object 270 to pass through. Thereby, assembly of the electronic device 880 to the base 830 can be facilitated.

In some examples, referring to Figs. 33A and 33B, the sensor 820 may be connected to the electronic device 880. As described above, a contact portion 823 of the sensor 820 may be connected to the electronic device 880.

In some examples, referring to Figs. 33C and 33D, the electronic device 880 may include a spring plate 881. In some examples, the spring plate 881 may be configured to conductively connect the contact portion 823 with a circuit board of the electronic device 880. In some examples, the spring plate 881 may contact the contact portion 823. In some examples, the spring plate 881 may be made of a conductive material. In some examples, multiple spring plates 881 may be provided, e.g., the number of spring plates 881 may be 2, 3, 4, 5, or 6.

In some examples, the spring plate 881 may be fixedly connected to the circuit board of the electronic device 880. For example, the spring plate 881 may be soldered to the circuit board. Thereby, electrical connection between the sensor 820 and the electronic device 880 can be facilitated.

In other examples, the spring plate 881 may also be fixedly connected to the base 830.

In some examples, referring to Figs. 33D and 33E, the electronic device 880 may include a connector 883. In some examples, the connector 883 may be configured to fix the spring plate 881.

In some examples, referring to Figs. 33C and 33D, the spring plate 881 may be assembled to the connector 883. In some examples, referring to Fig. 33E, the connector 883 may have a recess 885. In some examples, the recess 885 may have a contour and size similar to those of the spring plate 881. Thereby, when the connector 883 is mounted on the spring plate 881, the relative positions among multiple spring plates 881 can be constrained.

In some examples, referring to Figs. 33A and 33B, the connector 883 may be mounted on the contact portion 823 and a fixing portion 838. In some examples, the connector 883 may be aligned with the contact portion 823 and the fixing portion 838.

In some examples, referring to Fig. 33E, the connector 883 may have a cross-shaped recess 887. In some examples, the cross-shaped recess 887 may have a contour and size similar to those of the contact portion 823 and the fixing portion 838. In some examples, the size of the cross-shaped recess 887 may be slightly smaller than that of the contact portion 823 and the fixing portion 838. Thereby, the connector 883 can be more tightly mounted on the contact portion 823 and the fixing portion 838.

In other examples, the connector 883 may also be fixedly connected to the base 830 via adhesive or ultrasonic welding.

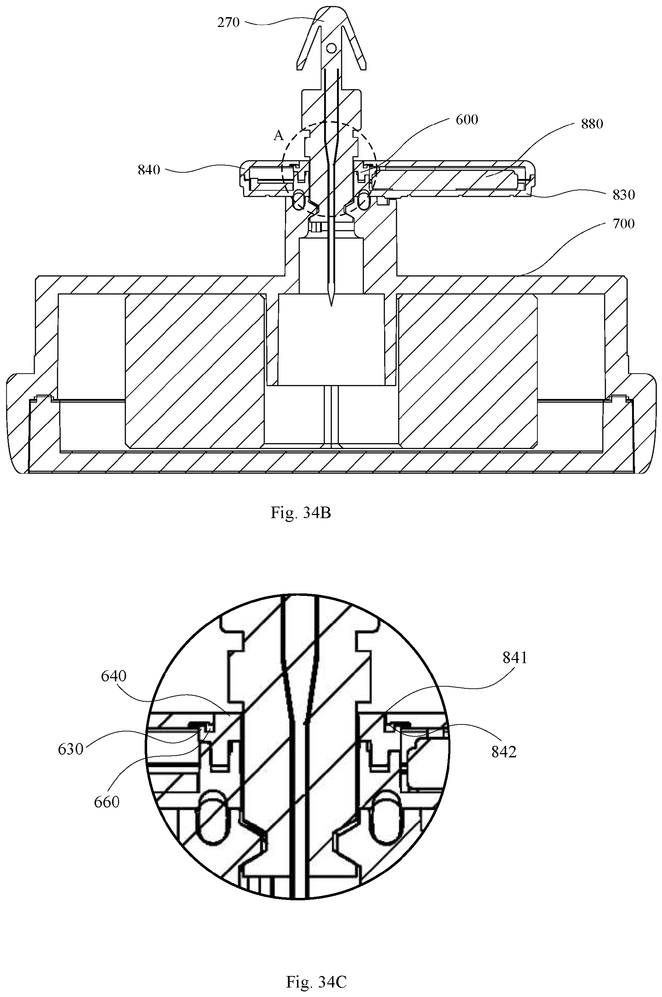

Fig. 34A is a schematic diagram showing assembly of an upper cover 840 and the base 830 according to an example of the present disclosure. Fig. 34B isa schematic diagram showing assembly of an upper cover 840 and the base 830 according to an example of the present disclosure. Fig. 34C is an enlarged view of region A in Fig. 34B.

In some examples, referring to Fig. 34A, an application portion 860 may include the upper cover 840. In some examples, the upper cover 840 may be assembled to the base 830. In some examples, the upper cover 840 may be mounted on the base 830. In some examples, the contour of the upper cover 840 may be identical to that of the base 830. In some examples, the outermost contour of the upper cover 840 may be identical to the outermost contour of the base 830.

In some examples, referring to Fig. 34B, the upper cover 840 and the base 830 may cooperate to form a first space. In some examples, the electronic device 880 may be accommodated in the first space. Thereby, sealing of the electronic device 880 can be facilitated.

In some examples, the upper cover 840 may be fixed to the base 830. For example, the upper cover 840 may be fixed to the base 830 via adhesion. Thereby, sealing of the first space can be facilitated.

In some examples, the upper cover 840 may be detachably connected to the base 830. For example, the upper cover 840 may be connected to the base 830 via detachable methods such as snap-fit or screw-fastening.

In some examples, referring to Figs. 34A and 34B, the sharp object 270 may penetrate the upper cover 840 and be assembled onto the sealing cover 600.

In some examples, referring to Fig. 34C, the upper cover 840 may have a third hole 841. In some examples, the third hole 841 may be configured to allow the sharp object 270 to pass through. In some examples, the third hole 841 may be aligned with the opening portion 831. In some examples, the third hole 841 may be aligned with a central hole 610. Thereby, assembly of the sharp object 270 onto the sealing cover 600 can be facilitated.

In some examples, a sealing interface may be formed around the opening portion 831 between the upper cover 840 and the base 830. In some examples, the sensor 820 may extend through the sealing interface.

In some examples, referring to Fig. 25C, the sealing cover 600 may have a central boss 640. In some examples, the central boss 640 may be disposed on a side of the sealing cover 600 close to the upper cover 840. In some examples, the central boss 640 may be at least partially embedded in the upper cover 840. Thereby, the relative position between the sealing cover 600 and the upper cover 840 can be constrained.

In some examples, referring to Fig. 34C, the central boss 640 may be embedded in the third hole 841. In some examples, the central boss 640 may be aligned with the third hole 841. Thereby, embedding of the central boss 640 into the third hole 841 can be facilitated. In some examples, an outer edge of the central boss 640 may fit against an inner edge of the third hole 841.

In some examples, referring to Fig. 34C, the upper cover 840 has an annular ridge portion 842. In some examples, the annular ridge portion 842 may be disposed on a side of the upper cover 840 close to the sealing cover 600. In some examples, the annular ridge portion 842 may surround the third hole 841. In some examples, the annular ridge portion 842 may fit against the outer edge of the central boss 640. In this case, by increasing the contact area between the upper cover 840 and the sealing cover 600, sealing of the interface between the upper cover 840 and the sealing cover 600 can be facilitated.

In some examples, referring to Figs. 25C and 34C, the sealing cover 600 may have an annular flange 630. In some examples, the annular flange 630 may be located outermost on the sealing cover 600. In some examples, the annular flange 630 may surround the central boss 640. In some examples, the annular flange 630 may extend toward the upper cover 840 and abut against the upper cover 840.

In some examples, referring to Fig. 34C, the annular flange 630 and the central boss 640 may form a second pocket 660. In some examples, the annular ridge portion 842 may be at least partially received in the second pocket 660.

In some examples, the upper cover 840 and the sealing cover 600 may be sealingly engaged. In some examples, an adhesive may be injected into the second pocket 660. In some examples, the interface between the upper cover 840 and the sealing cover 600 may be sealed by injecting adhesive into the second pocket 660.

In some examples, referring to Fig. 34C, the central hole 610 may penetrate the central boss 640. Thereby, the sharp object 270 can easily pass through the sealing cover 600.

In some examples, an upper surface of the upper cover 840 may be substantially coplanar with an upper surface of the sealing cover 600. In other words, the upper surface of the upper cover 840 may be substantially flush with the upper surface of the sealing cover 600. In this case, the overall flatness of the upper surface of the application portion 860 can be improved, thereby simplifying the sealing of the upper interface (refer to the method of sealing the upper interface herein).

In some examples, to facilitate assembly of a sterilization assembly, the assembly process may generally include a first step (i.e., assembling the sharp object 270 and the first sealing element 510), a second step (i.e., assembling the sensor 820, the base 830, and the sealing cover 600), a third step (i.e., assembling the cap 700 and the second sealing element 520), and a fourth step.

In some examples, in the first step, the sharp object 270 may pass through a first hole 511 of the first sealing element 510 so that the first sealing element 510 is sleeved on a second part 278 of the sharp object 270. In some examples, in the second step, a tail portion 821 of the sensor 820 may be aligned with the opening portion 831 of the base 830, a connection portion 822 may be aligned with the channel 400, and the contact portion 823 may be aligned with the fixing portion 838, so that the sensor 820 is assembled to the base 830. In some examples, in the second step, an adhesive may be injected into a first pocket 8341 of the assembly portion 834. After aligning a second limiting portion 650 of the sealing cover 600 with a first limiting portion 835 of the base 830 and aligning a first notch 620 with the channel 400 and the connection portion 822, the sealing cover 600 may be assembled to the assembly portion 834, so that the sealing cover 600 is at least partially received in the first pocket 8341. In some examples, in the second step, an adhesive sheet 870 may also be assembled to a bottom surface of the base 830 (i.e., a side close to the cap 700). Thereby, assembly of the sharp object 270, first sealing element 510, sealing cover 600, sensor 820, and base 830 can be completed.

In some examples, in the third step, the second sealing element 520 may be assembled to a first sealing groove 780 of the cap 700. Thereby, assembly of the second sealing element 520 and the cap 700 can be completed.

In some examples, in the fourth step, the sharp object 270 assembled with the first sealing element 510 may first be assembled to the base 830 assembled with the sensor 820 and sealing cover 600. Then, the sharp object 270 and the base 830 may be assembled to the cap 700 assembled with the second sealing element 520. Thereby, assembly of the sealing assembly can be completed.

In other examples, in the fourth step, the base 830 assembled with the sensor 820 and sealing cover 600 may first be assembled to the cap 700 assembled with the second sealing element 520. Then, the base 830 and the cap 700 may be assembled to the sharp object 270 assembled with the first sealing element 510.

In some examples, in the fourth step, the assembled sharp object 270 and base 830 may be aligned with the cap 700. In some examples, during alignment of the sharp object 270, base 830, and cap 700, a second sealing groove 839 may be aligned with a second hole 521 of the second sealing element 520 and the first sealing groove 780. A first engagement structure 27411 of the engaging portion 2741 may be aligned with an entry guide groove 723 of the mating portion 720.

In some examples, during assembly of the sharp object 270 and base 830 to the cap 700, the engaging portion 2741 of the sharp object 270 extending out of the bottom surface of the base 830 may be aligned with the mating portion 720 of the cap 700. The guided portion 730 may be aligned with a starting position 8361 of the guide portion 836. In some examples, after aligning the engaging portion 2741 with the mating portion 720 and the guided portion 730 with the starting position 8361, the cap 700 may be twisted relative to the base 830 by a preset angle along the extending direction of the guide portion 836, so that the guided portion 730 moves to an ending position 8362 of the guide portion 836.

In some examples, when the guided portion 730 is located at the ending position 8362, the sealed space is formed.

In some examples, after moving the guided portion 730 to the ending position 8362, the guided portion 730 may couple with the third limiting portion 837 at the ending position 8362 to restrict further movement of the guided portion 730 and maintain it at the ending position 8362. In this case, by limiting the position of the guided portion 730 via the third limiting portion 837, the relative positions among the sharp object 270, base 830, and cap 700 can be fixed, thereby facilitating assembly of the sterilization assembly to an application device 1000. Additionally, since the pulling force between the sharp object 270 and the cap 700 increases with the relative twisting angle, limiting the position of the guided portion 730 via the third limiting portion 837 can restrict the magnitude of the pulling force between the sharp object 270 and the cap 700, reducing the risk of deformation or damage to components due to excessive pulling force between the sharp object 270 and the cap 700.

In some examples, after sterilizing the entire sterilization assembly, the electronic device 880 may be assembled to the sterilized base 830. In some examples, before assembling the electronic device 880 to the sterilized base 830, the spring plate 881 may first be fixedly connected to the circuit board of the electronic device 880. Then, the connector 883 may be mounted on the spring plate 881.

In some examples, after assembling the electronic device 880, the upper cover 840 may be assembled to the base 830.

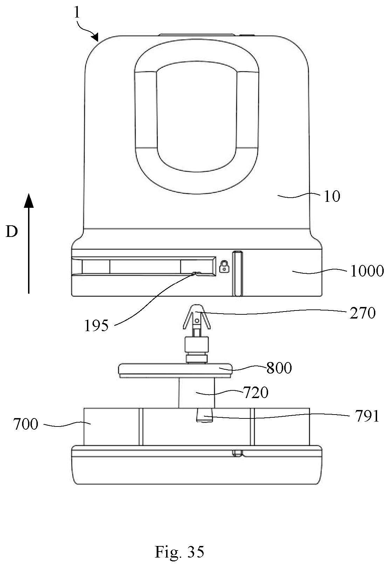

Fig. 35 is a schematic diagram showing assembly of a housing 10 and the sterilization assembly assembled with the electronic device 880 and upper cover 840 according to an example of the present disclosure.

In some examples, the sterilization assembly assembled with the electronic device 880 and upper cover 840 may be referred to as a pre-assembled module.

In some examples, a cap portion 276 of the pre-assembled module may be removably and sealingly engaged with the application portion 860 above the opening portion 831. In some examples, the cap 700 of the pre-assembled module may be removably and sealingly engaged with the application portion 860 below the opening portion 831.

In some examples, the mating portion 720 and a bottom cover 770 may be integrally formed. Thereby, the cap 700 can both form a sealed space receiving at least a portion of the sensor 820 and couple with the application device 1000.

In some examples, referring to Fig. 35, the sterilization assembly assembled with the electronic device 880 and upper cover 840 may be moved as a whole along an assembly direction D to assemble to the application device 1000, so that the cap 700 is assembled to a proximal end of the application device 1000.

Due to at least one of the following reasons: non-circular shape of the application portion 860 and an accommodating portion 250, off-center positions of holes in the application portion 860 and accommodating portion 250, and engagement between the sharp object 270 and a support base 280, the application portion 860 may be assembled to the application device 1000 in a specific orientation.

In some examples, referring to Fig. 8A or Fig. 8B, the housing 10 may have an assembly slot 194.

In some examples, when the guided portion 730 moves to the ending position 8362 of the guide portion 836, it can align multiple components of a pre-assembly module with multiple components of the application device 1000 during the process of assembling the pre-assembly module with the application device 1000. Thereby, assembly can be effectively performed. In some examples, at the ending position 8362, the application portion 860 is aligned with the accommodating portion 250. In some examples, at the ending position 8362, the engagement feature (second engagement feature 791) of the cap 700 is aligned with the assembly slot 194.

In some examples, the housing 10 may have a linear assembly slot 194. Thereby, the cap 700 can be linearly coupled to the application device 1000.

In some examples, the assembly slot 194 may be disposed at an end of the first engagement feature 191 away from the decoupling groove 193. In some examples, during the process of assembling integrally the sterilized component, pre-assembled with the electronic device 880 and the upper cover 840, onto the application device 1000, the second engagement feature 791 can be aligned with the assembly slot 194.

In some examples, referring to Fig. 8A or Fig. 8B, the assembly slot 194 may have a snap-fit portion 1941. In some examples, the second engagement feature 791 can enter the housing 10 along the extension direction of the assembly slot 194 and abut against the snap-fit portion 1941. In some examples, when the second engagement feature 791 abuts against the snap-fit portion 1941, a force can be applied to the cap 700 and the housing 10 to cause the second engagement feature 791 to press against the snap-fit portion 1941 and enter the first engagement feature 191. Thereby, this can help stably couple the cap 700 to the housing 10.

In some examples, referring to Fig. 35, the housing 10 may have a limit protrusion 195. In some examples, after the second engagement feature 791 presses against the snap-fit portion 1941 and enters the first engagement feature 191, the second engagement feature 791 can abut against the limit protrusion 195. In some examples, the limit protrusion 195 can be configured to restrict the second engagement feature 791 at an end of the first engagement feature 191 close to the assembly slot 194. Thereby, this can help prevent decoupling of the cap 700 from the housing 10 before user use.

In some examples, the extension direction of the first engagement feature 191 may intersect the extension direction of the assembly slot 194. Thereby, the cap 700 and the housing 10 can be decoupled in a manner different from how they are coupled.

In some examples, the extension direction of the first engagement feature 191 may be orthogonal to the extension direction of the assembly slot 194. Thereby, coupling and decoupling the cap 700 and the housing 10 can be facilitated. However, the present disclosure is not limited thereto; the extension direction of the first engagement feature 191 and the extension direction of the assembly slot 194 may have a preset angle, for example, 60 degrees, 70 degrees, or 80 degrees, etc.

In some examples, the assembly slot 194 may extend along the central axis CA of the application device 1000. However, the present disclosure is not limited thereto; the assembly slot 194 may extend in a direction having a preset angle with the central axis CA of the application device 1000, for example, extending at 10 degrees, 20 degrees, or 30 degrees relative to the central axis CA.

As described above, the sharp object 270 may be separably assembled and combined with the support base 280. In some examples, referring to Fig. 5B and Fig. 35, during the process of assembling integrally the sterilized component, pre-assembled with the electronic device 880 and the upper cover 840, onto the application device 1000, the cap portion 276 of the sharp object 270 can extend through the hole 2821 and assemble into the support base 280.

In some examples, after assembling the housing 10 with the sterilized component pre-assembled with the electronic device 880 and the upper cover 840, the application device 1000, the medical device, the sharp object 270, and the cap 700 may have fixed relative positions. A detailed description of the relative positions between various components is provided below.

In some examples, the shape of the medical device 800 may be non-circular.

In some examples, the sharp object 270 may not be assembled at the geometric center of the medical device 800. Thereby, assembling the battery 8821 can be facilitated.

In some examples, the auxiliary mechanism 20 may include the housing 10, a moving body 200, and a accommodating portion 250. In some examples, the housing 10, the moving body 200, and the accommodating portion 250 may have fixed relative positions. In some examples, the accommodating portion 250 may be configured to releasably retain the medical device 800. In some examples, the accommodating portion 250 can accommodate the medical device 800.

In some examples, the medical device 800 and the cap 700 may have a fixed relative position. In some examples, the base 830 and the cap 700 may have a fixed relative position.

In some examples, the cap 700 can be assembled onto the housing 10 at a preset orientation by aligning a first indicating portion with a second indicating portion. Thereby, the medical device 800 can be aligned and accommodated into the accommodating portion 250.

As described above, the base 830 may have a third limiting portion 837. The third limiting portion 837 can be coupled to the guided portion 730. In some examples, the third limiting portion 837 can keep the relative positions of the base 830 and the cap 700 fixed by coupling with the guided portion 730. In some examples, when the third limiting portion 837 is coupled to the guided portion 730, the base 830 and the cap 700 may have a fixed relative position. In some examples, the third limiting portion 837 may be disposed on the guide portion 836 or on an extension line of the guide portion 836.

In some examples, during the process of assembling the base 830 of the medical device 800 with the cap 700, the guided portion 730 can be aligned with the starting position 8361 of the guide portion 836.

In some examples, when the guided portion 730 is aligned with and abuts the starting position 8361, the base 830 and the cap 700 can be relatively screwed to cause relative rotation between the engaging portion 2741 and the mating portion 720. In some examples, during the relative rotation of the base 830 and the cap 700, the guided portion 730 can move from the starting position 8361 along the extension direction of the guide portion 836 to the ending position 8362. In some examples, referring to Fig. 36A, when the guided portion 730 moves to the ending position 8362, the guided portion 730 can be coupled to the third limiting portion 837 located at the ending position 8362. In this case, by guiding the guided portion 730 via the guide portion 836, the base 830 and the cap 700 can be relatively screwed to a preset relative position, and by limiting the guided portion 730 via the third limiting portion 837, the base 830 and the cap 700 can be maintained at the preset relative position, thereby ensuring that the medical device 800 aligns with the accommodating portion 250 when assembling the cap 700 with the housing 10.

In some examples, during the process of assembling the base 830 of the medical device 800 with the cap 700, the first engagement structure 27411 can be aligned with the entry guide groove 723.

In some examples, when the first engagement structure 27411 is aligned with and enters the entry guide groove 723, the engaging portion 2741 and the mating portion 720 can relatively rotate during the relative screwing of the base 830 and the cap 700. In some examples, during relative rotation of the engaging portion 2741 and the mating portion 720, the first engagement structure 27411 can rotate relative to the second engagement structure 721. In some examples, aligning the first engagement structure 27411 with the entry guide groove 723 aligns the engaging portion 2741 with the mating portion 720. In some examples, a first rib 722 is disposed at an end of the second engagement structure 721 away from the entry guide groove 723. In some examples, the first engagement structure 27411 can move from the entry guide groove 723 along the extension direction of the second engagement structure 721 to the first rib 722. In some examples, when the first engagement structure 27411 moves to the first rib 722, it can abut against the first rib 722. In some examples, the angle of rotation of the first engagement structure 27411 relative to the second engagement structure 721 may be equal to a preset angle.

In some examples, when the guided portion 730 is aligned with the starting position 8361, the first engagement structure 27411 can be aligned with the entry guide groove 723. In some examples, when the guided portion 730 is coupled to the third limiting portion 837, the first engagement structure 27411 can be aligned with the first rib 722. In this case, after relatively screwing the cap 700 with the medical device 800 and the sharp object 270 by the preset angle, the cap 700 can be simultaneously coupled with both the medical device 800 and the sharp object 270.

In some examples, a user can decouple the housing 10 and the cap 700 by relatively screwing them.

A detailed description of the process of decoupling the cap 700 from the sharp object 270 follows.

In some examples, during the process of decoupling the cap 700 from the sharp object 270, the mating portion 720 and the engaging portion 2741 can move relative to each other. In some examples, the mating portion 720 can rotate relative to the engaging portion 2741. In some examples, the angle of rotation of the mating portion 720 relative to the engaging portion 2741 may be a preset angle.

In some examples, during the decoupling of the cap 700 and the sharp object 270, the first engagement structure 27411 can move relative to the second engagement structure 721 in a direction away from the first rib 722. In some examples, the first engagement structure 27411 can move relative to the second engagement structure 721 until it moves to the entry guide groove 723. In some examples, when the first engagement structure 27411 moves to the entry guide groove 723, it can move along the entry guide groove 723 in a direction away from the second engagement structure 721 until the engaging portion 2741 is decoupled from the mating portion 720.

In some examples, the motion path of the second engagement structure 721 relative to the first engagement structure 27411 may be an arc, and the angle corresponding to the radian of this arc may be equal to the preset angle. Thereby, after relatively screwing the cap 700 and the sharp object 270 by the preset angle, the cap 700 and the sharp object 270 can be decoupled.

In some examples, the angle of rotation of the mating portion 720 relative to the engaging portion 2741 may also be less than the preset angle. In this case, because after the user relatively screws the cap 700 and the housing 10 by the preset angle, the second engagement feature 791 abuts against one end of the first engagement feature 191, preventing further screwing by the user. By making the angle of rotation of the mating portion 720 relative to the engaging portion 2741 less than the preset angle, the cap 700 and the sharp object 270 can be decoupled earlier. This reduces the possibility that the cap 700 is decoupled from the housing 10 but not yet decoupled from the sharp object 270, facilitating normal use of the analyte monitoring system 1 by the user. Furthermore, compared to the situation where the rotation angle between the mating portion 720 and the engaging portion 2741 is greater than the preset angle, the relative rotation between the cap 700 and the housing 10 can provide guidance for the relative rotation between the mating portion 720 and the engaging portion 2741. This helps prevent a scenario where the cap 700 is decoupled from the housing 10 but the mating portion 720 and the engaging portion 2741 are not decoupled, thereby reducing the risk of the cap 700 damaging the sharp object 270 and the sensor 820.

In some examples, after the cap 700 and the sharp object 270 are decoupled, the cap 700 and the sharp object 270 cease to exert a pulling force on each other. The first sealing element 510 and the second sealing element 520 are no longer compressed, thus releasing the seal of the upper and lower interfaces of the opening portion 831 and exposing the sharp object 270 and the medical device 800.

A detailed description of the process of decoupling the cap 700 from the medical device 800 follows.

In some examples, decoupling the cap 700 from the medical device 800 may refer to decoupling the cap 700 from the base 830 of the medical device 800. In some examples, referring to Fig. 10A, the base 830 may have a guide portion 836 and a third limiting portion 837. In some examples, the cap 700 may include a guided portion 730. In some examples, the base 830 may be coupled to the cap 700 via the third limiting portion 837 coupling with the guided portion 730.

In some examples, during the process of decoupling the cap 700 and the base 830, the guided portion 730 and the guide portion 836 and third limiting portion 837 may move relative to each other. In some examples, the guided portion 730 can decouple from the third limiting portion 837 and then move along the extension path of the guide portion 836. In some examples, the motion path of the guided portion 730 relative to the guide portion 836 and the third limiting portion 837 may be an arc, and the angle corresponding to the radian of this arc may be equal to the preset angle. Thereby, after relatively screwing the cap 700 and the base 830 by the preset angle, the cap 700 and the base 830 can be decoupled.

As described above, the guide portion 836 may be arcuate, and the angle corresponding to the radian of this arc may be equal to the preset angle. Thereby, this helps make the motion path of the guided portion 730 an arc and can restrict the angle corresponding to the radian of the guided portion 730's motion path within the preset angle.

In some examples, the third limiting portion 837 may be located at the ending position 8362. In some examples, during the process of decoupling the cap 700 and the base 830, the guided portion 730 can move from the ending position 8362 to the starting position 8361.

A description of the decoupling of the cap 700 from the housing 10, the decoupling of the cap 700 from the sharp object 270, and the decoupling of the cap 700 from the medical device 800 follows.

In some examples, the decoupling of the cap 700 from the housing 10, the decoupling of the cap 700 from the sharp object 270, and the decoupling of the cap 700 from the medical device 800 may be linked. That is, the cap 700, the housing 10, the sharp object 270, and the medical device 800 may start decoupling simultaneously and complete decoupling simultaneously. In some examples, the process of decoupling the cap 700 from the housing 10, the process of decoupling the cap 700 from the sharp object 270, and the process of decoupling the cap 700 from the medical device 800 can proceed concurrently. In some examples, during the decoupling of the cap 700 from the three components mentioned above, the angle of rotation of the second engagement feature 791 relative to the first engagement feature 191, the angle of rotation of the mating portion 720 relative to the engaging portion 2741, and the angle of rotation of the guided portion 730 relative to the guide portion 836 and the third limiting portion 837 may be equal in real-time. In some examples, during the decoupling process, when the second engagement feature 791 moves from one end of the first engagement feature 191 to the other end, the first engagement structure 27411 can move from the first rib 722 to the entry guide groove 723, and the guided portion 730 can move from the ending position 8362 to the starting position 8361.

Embodiments disclosed herein include:
D. An analyte monitoring system, characterized by comprising: a base including an opening portion; a sensor disposed on the base, the sensor including a tail portion located at the opening portion, a connection portion located outside the opening portion, and a contact portion, the connection portion connecting the tail portion and the contact portion; a sealing assembly configured to seal the opening portion, the sealing assembly comprising a first sealing element.

In some examples, embodiment D can be combined with any one or more of the following additional elements. Element 1: The first sealing element has a first hole aligned with the opening portion. Element 2: It further includes a sharp object extending through the opening portion, the sharp object including a cap portion engaging the first sealing element. Element 3: The first sealing element is sleeved on the cap portion, both ends of the first sealing element embedded in the cap portion. Element 4: The tail portion is aligned with the opening portion. Element 5: The sharp object further includes a needle portion configured to accommodate the tail portion and carry the tail portion to penetrate tissue. Element 6: It further includes a cap having a chamber configured to accommodate the tail portion, the sharp object coupled to the cap to cause the sharp object to compress the first sealing element. Element 7: The end of the cap portion proximate to the base has an engaging portion, and the end of the cap proximate to the base has a mating portion coupled to the engaging portion. Element 8: Coupling is achieved by relative rotation of the engaging portion and the mating portion. Element 9: The engaging portion has a first engagement structure, and the mating portion has a second engagement structure. Element 10: The first engagement structure includes a first engagement end, a second engagement end, and an engagement groove, the size of the engagement groove matching the size of the second engagement structure. Element 11: The first engagement end has an engagement notch. Element 12: The first engagement structure is a first thread, and the second engagement structure is a second thread having a preset angle. Element 13: The mating portion has a first rib arranged at the end point of the second engagement structure. Element 14: The mating portion has an entry guide groove. Element 15: The base includes a support portion having a channel extending towards the opening portion, the connection portion extending through the channel. Element 16: It further includes a sealing cover, the sealing cover at least partially covering the channel. Element 17: The sealing cover has a first notch, at least part of the first notch coinciding with the channel, and the connection portion passes through the first notch. Element 18: The sealing cover engages the first sealing element. Element 19: The base has a guide portion, and the cap has a guided portion that moves along the guide portion during rotation. Element 20: The guide portion is a guide groove formed by indentation on the bottom surface of the base. Element 21: The guide portion presents as an arc having a predetermined curvature. Element 22: The base has a third limiting portion disposed on the guide portion or an extension line of the guide portion and cooperating with the guided portion. Element 23: During the coupling process of the engaging portion and the mating portion, the starting position of the guided portion is the starting position of the guide portion, and the ending position of the guided portion is the ending position of the guide portion. Element 24: The third limiting portion is a protrusion, and the guided portion has a groove cooperating with the third limiting portion. Element 25: The base further includes a fixing portion for fixing the contact portion. Element 26: It further includes an upper cover, the upper cover having a third hole, the third hole aligned with the central hole. Element 27: It further includes a sealing cover, the sealing cover having a central boss embedded in the third hole, the outer edge of the central boss fitting with the inner edge of the third hole. Element 28: The upper cover has an annular ridge surrounding the third hole, the sealing cover has an annular flange surrounding the central boss and forming a second pocket with the central boss, the annular ridge at least partially received within the second pocket. Element 29: The cap portion includes a first part, the outer contour of the first part matching the inner contour of the opening portion and both being racetrack-shaped. Element 30: The sharp object includes a depth-limiting portion protruding outward from the first part.

E. An analyte monitoring system, characterized in that it comprises a base, the base including an opening portion; a sensor disposed on the base, the sensor including a tail portion located in the opening portion, a connection portion located outside the opening portion, and a contact portion, the connection portion connecting the tail portion to the contact portion; a sealing assembly, the sealing assembly sealing the opening portion; a channel extending towards the opening portion, the sealing assembly forming a seal in the channel or in the extending direction of the channel.

In some examples, Embodiment E may be combined with any one or more of the following additional elements. Element 1: The sealing assembly is at least partially disposed within the channel. Element 2: The base includes a support portion, the support portion being a boss. Element 3: The channel is disposed on the support portion. Element 4: It further includes a sealing cover, the sealing cover at least partially covering the channel. Element 5: It further includes a sealing cover, the channel disposed on the sealing cover. Element 6: The sealing cover has a central hole aligned with the opening portion. Element 7: The sealing cover has a first notch, at least part of the first notch coinciding with the channel. Element 8: The base includes a mating portion configured for male-female engagement with the sealing cover. Element 9: The base has a first limiting portion, the sealing cover has a second limiting portion, the first limiting portion aligned with the second limiting portion to guide the sealing cover to be assembled onto the mating portion. Element 10: The first limiting portion abuts the second limiting portion along the circumference of the mating portion. Element 11: The mating portion has a pocket surrounding the opening portion and communicating with the channel, the sealing cover at least partially received within the pocket. Element 12: There is a gap between the portion of the sealing cover received in the pocket and the bottom of the pocket. Element 13: The base has a guide portion, and the cap has a guided portion that moves along the guide portion during rotation. Element 14: The guide portion is a guide groove formed by indentation on the bottom surface of the base. Element 15: The guide portion presents as an arc having a predetermined curvature. Element 16: The base has a third limiting portion disposed on the guide portion or an extension line of the guide portion and cooperating with the guided portion. Element 17: It further includes a sharp object having a cap portion, the end of the cap portion proximate to the base having an engaging portion, and the end of the cap proximate to the base having a mating portion coupled to the engaging portion; during the coupling process of the engaging portion and the mating portion, the starting position of the guided portion is the starting position of the guide portion, and the ending position of the guided portion is the ending position of the guide portion. Element 18: The third limiting portion is a protrusion, and the guided portion has a groove cooperating with the third limiting portion. Element 19: The base further includes a fixing portion for fixing the contact portion. Element 20: It further includes an upper cover, the upper cover having a third hole, the third hole aligned with the central hole. Element 21: It further includes a sealing cover, the sealing cover having a central boss embedded in the third hole, the outer edge of the central boss fitting with the inner edge of the third hole. Element 22: The upper cover has an annular ridge surrounding the third hole, the sealing cover has an annular flange surrounding the central boss and forming a second pocket with the central boss, the annular ridge at least partially received within the second pocket. Element 23: The sharp object includes a needle portion configured to carry the tail portion to penetrate tissue. Element 24: The cap portion includes a first part, the outer contour of the first part matching the inner contour of the opening portion and both being racetrack-shaped. Element 25: The sharp object includes a depth-limiting portion protruding outward from the first part.

F. An analyte monitoring system, characterized in that it comprises a base, the base including an opening portion; a sensor disposed on the base, the sensor including a tail portion located in the opening portion, a connection portion located outside the opening portion, and a contact portion, the connection portion connecting the tail portion to the contact portion; a sealing assembly configured to seal the opening portion, the sealing assembly including a second sealing element.

In some examples, Embodiment F may be combined with any one or more of the following additional elements. Element 1: The second sealing element has a second hole aligned with the opening portion. Element 2: It further includes a cap configured to accommodate the tail portion and engage the second sealing element. Element 3: The cap compresses the second sealing element against the base. Element 4: The side of the cap proximate to the base has a first sealing groove, and the side of the base proximate to the cap has a second sealing groove. Element 5: It further includes a sharp object configured to accommodate the tail portion and carry the tail portion to penetrate tissue, the cap coupled to the sharp object to cause the cap to exert pressure on the second sealing element. Element 6: Coupling is achieved by relative rotation of the cap and the sharp object. Element 7: The base has a guide portion, and the cap has a guided portion that moves along the guide portion during rotation. Element 8: The guide portion is a guide groove formed by indentation on the bottom surface of the base. Element 9: The guide portion presents as an arc having a predetermined curvature. Element 10: The base has a third limiting portion disposed on the guide portion or an extension line of the guide portion and cooperating with the guided portion. Element 11: The sharp object further includes a cap portion, the end of the cap portion proximate to the base having an engaging portion, and the end of the cap proximate to the base having a mating portion coupled to the engaging portion; during the coupling process of the engaging portion and the mating portion, the starting position of the guided portion is the starting position of the guide portion, and the ending position of the guided portion is the ending position of the guide portion. Element 12: The third limiting portion is a protrusion, and the guided portion has a groove cooperating with the third limiting portion. Element 13: The base further includes a fixing portion for fixing the contact portion. Element 14: It further includes an upper cover, the upper cover having a third hole, the third hole aligned with the central hole. Element 15: It further includes a sealing cover, the sealing cover having a central boss embedded in the third hole, the outer edge of the central boss fitting with the inner edge of the third hole. Element 16: The upper cover has an annular ridge surrounding the third hole, the sealing cover has an annular flange surrounding the central boss and forming a second pocket with the central boss, the annular ridge at least partially received within the second pocket. Element 17: The sharp object includes a needle portion configured to carry the tail portion to penetrate tissue. Element 18: The cap portion includes a first part, the outer contour of the first part matching the inner contour of the opening portion and both being racetrack -shaped. Element 19: The sharp object includes a depth-limiting portion protruding outward from the first part.

G. An analyte monitoring system, characterized in that it comprises a base, the base including an opening portion; a sensor disposed on the base, the sensor including a tail portion located in the opening portion, a connection portion located outside the opening portion, and a contact portion, the connection portion connecting the tail portion to the contact portion; a sealing assembly configured to seal the opening portion, the sealing assembly including a first sealing element and a second sealing element; a channel extending towards the opening portion, the sealing assembly forming a seal in the channel or in the extending direction of the channel.

In some examples, Embodiment G may be combined with any one or more of Embodiments D, E, and F.

H. An assembly method for a sterilization component, the sterilization component comprising a base having a guide portion, a sensor, a sealing cover, a sharp object, a sealing assembly, and a cap having a guided portion, the guide portion having a starting position and an ending position, the sealing assembly including a first sealing element and a second sealing element, characterized in that: assembling the sensor onto the base; covering the sealing cover onto the base and the sensor; passing the sharp object through the first sealing element and assembling it onto the base; sleeving the second sealing element on the cap; when the guided portion is aligned with and abuts the starting position, relatively rotating the cap and the base to simultaneously couple the cap to both the base and the sharp object.

In some examples, Embodiment H may be combined with any one or more of the following additional elements. Element 1: The base includes an opening portion, and the sharp object and the sensor pass through the opening portion and extend out of the bottom surface of the base. Element 2: It includes a channel extending towards the opening portion, and the sensor passes through the channel and is located in the opening portion. Element 3: The sealing cover at least partially covers the channel. Element 4: The sealing cover has a first notch aligned with the channel. Element 5: The sharp object includes a cap portion, and the first sealing element is sleeved on the cap portion. Element 6: The sensor includes a tail portion, and the cap has a chamber configured to accommodate the tail portion. Element 7: The sharp object has an engaging portion. Element 8: The cap has a mating portion. Element 9: After relatively rotating the cap and the base, the engaging portion is coupled to the mating portion. Element 10: Relatively rotating the cap and the base by a preset angle to simultaneously couple the cap to both the base and the sharp object. Element 11: The guide portion presents as an arc. Element 12: The guide portion has a predetermined curvature. Element 13: The angle corresponding to the predetermined curvature matches the preset angle. Element 14: The base has a third limiting portion. Element 15: After relatively rotating the cap and the base, the third limiting portion couples with the guided portion.

I. An assembly method for an analyte monitoring system, the assembly method comprising assembling a sensor module with an applicator device, the sensor module comprising a sensor, a sensor control device, a sharp object, and a cap portion, the sensor configured to extend from the bottom of the sensor control device, the sharp object configured to pass through the sensor control device and extend from the bottom of the sensor control device, the cap having a chamber for receiving the tail portion of the sensor and the tail portion of the sharp object, wherein the cap is coupleable to at least one of the sensor control device or the sharp object to form a sealed space for sealing the tail portion of the sensor and the tail portion of the sharp object, and the cap is removably coupled to the applicator device.

In some examples, Embodiment I may be combined with any one or more of the following additional elements. Element 1: The sharp object includes a needle portion and a cap portion configured to carry the needle portion, the cap portion including an abutting portion configured to abut the top of the sensor control device and an engaging portion formed on the cap portion and extending from the bottom of the sensor control device. Element 2: The cap and the engaging portion are coupled by relative rotation. Element 3: The bottom of the sensor control device has a guide portion configured to guide the relative movement of the cap relative to the engaging portion during relative rotation of the cap and the engaging portion. Element 4: The guide portion is a guide groove formed by indentation on the bottom surface of the sensor control device. Element 5: The guide portion presents as an arc having a predetermined curvature. Element 6: The cap has a guided portion configured to move along the guide portion. Element 7: During the coupling process of the engaging portion and the cap, the guide portion defines a starting position and an ending position for the movement of the guided portion.

Element 8: During the coupling process of the engaging portion and the cap, the starting position of the guided portion is the starting position of the guide portion, and the ending position of the guided portion is the ending position of the guide portion. Element 9: In response to the guide portion guiding the guided portion from the starting position to the ending position, the sharp object and/or the application portion have a first preset orientation relative to the cap. Element 10: The cap portion of the sharp object has a plurality of fingers configured to snap onto the support base. Element 11: The cap portion has a top end away from the sensor control device and a bottom end opposite to the top end, and the plurality of fingers are formed at the top end and extend away from the central axis of the sharp portion in a direction from the top end to the bottom end. Element 12: The cap portion has a top end away from the sensor control device and a bottom end opposite to the top end, and in a direction from the bottom end to the top end, the plurality of fingers are generally tapered. Element 13: Assembling the sensor assembly with the application device includes snapping the sensor assembly to the application device. Element 14: The application device includes a housing and an auxiliary mechanism assembled in the housing, the auxiliary mechanism including a moving body, a accommodating portion disposed on the moving body and configured to receive and releasably retain the sensor control device, and a support base disposed on the moving body and configured to receive and fix the sharp object. Element 12: The support base includes a limiting portion configured to inhibit rotation of the sharp object. Element 13: The limiting portion is a plurality of clamping grooves formed on the support base and matching with the plurality of fingers. Element 14: The support base includes a hole configured to receive the cap portion, and the cap portion may extend through the hole and be clamped to the limiting portion.

Element 15: Before snapping the sensor assembly to the application device, further includes aligning the cap portion and the support base along a direction of the central axis of the application device. Element 16: Aligning the cap portion and the support base is to align the plurality of fingers with the plurality of clamping grooves along the direction of the central axis of the application device. Element 17: The support base is assembled on the moving body in a manner having a second preset orientation relative to the housing. Element 18: Aligning the first preset orientation with the second preset orientation along the direction of the central axis of the application device to enable the support base to align with the sharp object. Element 19: The housing includes a first indicating portion having a first preset orientation relative to the support base, the cap includes a second indicating portion having a second preset orientation relative to the sharp object, and when the first indicating portion is aligned with the second indicating portion, the pre-assembled module is aligned with the application device. Element 20: The housing includes a proximal end close to the host during operation, and the proximal end is provided with an assembling groove configured to be coupled with the cap. Element 21: The cap is provided with a second engagement feature cooperating with the assembling groove. Element 22: The engagement feature is a protrusion formed on a peripheral edge of the cap. Element 23: It further includes a first engagement feature formed on the proximal end and located above the groove, and the second engagement feature may abut against the first engagement feature to enable the pre-assembled module to be assembled and coupled to the application device.

Although the present disclosure has been specifically described above with reference to the accompanying drawings and examples, it should be understood that the above description does not limit the present disclosure in any form. Those skilled in the art may make modifications and variations to the present disclosure as needed without departing from the essential spirit and scope of the present disclosure, and these modifications and variations all fall within the scope of the present disclosure.

## Claims

1. An analyte monitoring system, **characterized in that** the analyte monitoring system comprises a medical device, a piercing member, an application device, and a cap; the medical device includes an application portion and a sensor supported by the application portion and at least partially positionable subcutaneously; the piercing member is configured to carry at least a portion of the sensor into the subcutaneous tissue; the application device is configured to move the piercing member; the cap is coupled to the application device and has a sealed space for accommodating at least a portion of the sensor.

2. The analyte monitoring system according to claim 1, **characterized in that** the sensor includes a subcutaneously positionable tail portion, a contact portion for electrical connection, and a connection portion connecting the tail portion and the contact portion; the application portion includes a base having an opening portion; the sensor is supported by the base, and the central axis of the tail portion extends through the opening portion.

3. The analyte monitoring system according to claim 2, **characterized in that** the application portion further includes an upper cover configured to cooperate with the base to form a first space and having a hole aligned with the opening portion.

4. The analyte monitoring system according to claim 3, **characterized in that** a sealed interface is formed between the upper cover and the base around the opening portion, and the sensor extends through the sealed interface.

5. The analyte monitoring system according to claim 2, **characterized in that** the piercing member includes a sharp object having a needle portion and a cap portion; the needle portion is configured to carry the tail portion to pierce into the host's subcutaneous tissue; the cap portion is removably and sealingly engaged with the application portion above the opening portion; the cap is removably and sealingly engaged with the application portion below the opening portion.

6. The analyte monitoring system according to claim 5, **characterized in that** the cap includes a mating portion coupled with at least one of the medical device and the cap portion, and the mating portion has a chamber configured to receive at least a portion of the sensor.

7. The analyte monitoring system according to claim 6, **characterized in that** an end of the cap portion close to the base has an engaging portion coupled with the mating portion; the engaging portion and the mating portion are coupled by relatively screwing to form the sealed space.

8. The analyte monitoring system according to claim 7, **characterized in that** the base has a guide rail, and the cap has a guided portion that moves along the guide rail when rotated.

9. The analyte monitoring system according to claim 8, **characterized in that** the guide rail is a guide groove formed by indenting the bottom surface of the base.

10. The analyte monitoring system according to claim 8, **characterized in that** the guide rail is arc-shaped with a predetermined arc and a predetermined radius.

11. The analyte monitoring system according to claim 8, **characterized in that** the base has a limiting portion disposed on the guide rail or an extension line thereof and cooperating with the guided portion.

12. The analyte monitoring system according to claim 6, **characterized in that** the cap further includes a bottom cover engaged with the mating portion and coupled with a housing of the application device.

13. The analyte monitoring system according to claim 12, **characterized in that** the housing of the application device has a linear assembly slot, and the bottom cover is assembled to the housing of the application device along the assembly slot.

14. The analyte monitoring system according to claim 13, **characterized in that** the housing of the application device further has an engagement feature, and the bottom cover moves along the engagement feature to separate from the application device, wherein an extending direction of the engagement feature intersects with an extending direction of the assembly slot.

15. The analyte monitoring system according to claim 6, **characterized in that** the mating portion and the bottom cover are integrally formed.

16. The analyte monitoring system according to claim 15, **characterized in that** the bottom cover has a chamber configured to accommodate a desiccant, and the chamber of the bottom cover is in communication with the chamber of the mating portion.

17. The analyte monitoring system according to claim 4, **characterized in that** a sealing cover for forming the sealed interface is disposed between the upper cover and the base; the upper cover is sealingly engaged with the sealing cover, and an upper surface of the upper cover is substantially coplanar with an upper surface of the sealing cover.

18. The analyte monitoring system according to claim 11, **characterized in that** during coupling of the engaging portion and the mating portion, a starting position of the guided portion is a starting position of the guide rail, and a ending position of the guided portion is a ending position of the guide rail; the sealed space is formed when the guided portion is located at the ending position.

19. The analyte monitoring system according to claim 13, **characterized in that** at the ending position, the application portion is aligned with the accommodating portion, and the engagement feature of the cap is aligned with the assembly slot.

20. The analyte monitoring system according to claim 1, **characterized in that** the application device includes a housing having a first retaining mechanism, an auxiliary mechanism releasably retained by the first retaining mechanism, and a first triggering mechanism configured to trigger the first retaining mechanism to release the auxiliary mechanism; the piercing member and the medical device are assembled to the auxiliary mechanism; when the auxiliary mechanism is released, the auxiliary mechanism is driven toward the host and at least partially places the medical device subcutaneously in the host through the piercing member, wherein the first triggering mechanism and the first retaining mechanism are integrally formed with the housing of the application device.
